# EUROPEAN PATENT SPECIFICATION

(11) **EP 3 299 451 B1**
(45) Date of publication and mention of the grant of the patent: **07.08.2019**
(21) Application number: 17172872.8
(22) Date of filing: 17.09.2014
(51) Int. Cl.: C12N 5/077, C12N 5/0775, A61K 35/35, A61K 35/28, A61P 17/00, A61P 37/00, A61P 43/00

(54) **METHODS OF USING ADIPOSE TISSUE-DERIVED CELLS IN THE TREATMENT OF RAYNAUD'S PHENOMENON**
VERFAHREN ZUR VERWENDUNG VON ZELLEN AUS FETTGEWEBE ZUR BEHANDLUNG VON RAYNAUD'S SYNDROM
PROCÉDÉS D'UTILISATION DE CELLULES ISSUES DU TISSU ADIPEUX DANS LE TRAITEMENT DU SYNDROME DE RAYNAUD

(30) Priority: 19.09.2013 US 201361880086 P; 09.04.2014 US 201461977466 P
(43) Date of publication of application: 28.03.2018
(62) Divisional of application: 14845486.1
(73) Proprietor: Cytori Therapeutics, Inc., San Diego, California 92121 (US)
(72) Inventor: FRASER, John, San Diego, CA 92121 (US); MAGALON, Guy, San Diego, CA 92121 (US)
(74) Representative: EIP

(56) References cited:
- US-A1- 2012 207 790
- POZZI M R ET AL: "AUTOLOGOUS FAT TRANSFER FOR DIGITAL ULCERS TREATMENT IN SYSTEMIC SCLEROSIS", ANNALS OF THE RHEUMATIC DISEASES, vol. 72, no. Suppl. 3, June 2013 (2013-06) , page 649, XP009190959, & ANNUAL EUROPEAN CONGRESS OF RHEUMATOLOGY (EULAR); MADRID, SPAIN; JUNE 12 -15, 2013
- SCUDERI N ET AL: "Human adipose-derived stromal cells for cell-based therapies in the treatment of systemic sclerosis", CELL TRANSPLANTATION 2013 COGNIZANT COMMUNICATION CORPORATION USA, vol. 22, no. 5, 17 April 2012 (2012-04-17) , pages 779-795, XP002759956, ISSN: 0963-6897
- DAUMAS A ET AL: "Interests and potentials of adipose tissue in scleroderma", REVUE DE MEDECINE INTERNE 2013 ELSEVIER MASSON SAS FRA, vol. 34, no. 12, 17 September 2013 (2013-09-17), pages 763-769, XP002759957, ISSN: 0248-8663

## Description

### FIELD OF THE INVENTION

Aspects of the present invention relate to the field of medicine, specifically, to the study of pain and the study of fibrosis and the effect of adipose tissue and its components on modulating and/or ameliorating pain and/or fibrosis.

### BACKGROUND

An estimated 86 million people, or one third of all Americans, suffer from chronic pain. While acute pain is often a normal sensation that is triggered to send a signal for possible injury, chronic pain is different. Chronic pain persists. Pain signals keep firing in the nervous system for weeks, months, even years.

The origin of the pain may be a specific event such as a physical trauma, (*e.g*., a sprained back), a serious infection, or an ongoing cause of pain, e.g., arthritis, cancer, ear infection. Some people also suffer chronic pain in the absence of any past injury or evidence of body damage. Common chronic pain complaints include headache, low back pain, cancer pain, arthritis pain, neurogenic pain (pain resulting from damage to the peripheral nerves or to the central nervous system itself), and psychogenic pain (pain not due to past disease or injury or any visible sign of damage inside or outside the nervous system).

A person may have two or more co-existing chronic pain conditions. Often, it is not clear whether these disorders share a common cause.

Scerloderma is an autoimmune rheumatic disease associated with fibrosis and chronic pain, and may give rise to various other complications. Systemic Sclerosis, or Scleroderma, is characterized by microvascular abnormalities and progressive fibrosis of skin and internal organs. Individuals afflicted with Scleroderma often complain of disability in daily activities related to hand involvement. Therapeutic interventions on scerloderma hands have mainly focused on treatment of vascular manifestations, but not fibrosis.

No effective treatments for the pain or fibrosis associated with Scleroderma exist today. Accordingly, there is a need in the medical field for improved compositions and methods of suppressing pain and modulating fibrosis, e.g., in scleroderma and other disorders characterized by fibrosis and/or chronic pain.

### SUMMARY OF THE INVENTION

The invention provides a therapeutically effective amount of adipose-derived stem and regenerative cells for use in the treatment of Raynaud's phenomenon in a subject in need thereof. Also provided herein is a method of treating or modulating pain, fibrosis, or both in an individual, comprising administering to the individual a therapeutically effective amount of adipose derived stem and regenerative cells, wherein the therapeutically effective amount is an amount sufficient to cause a detectable improvement in said pain, fibrosis, or pain and fibrosis.

Also provided herein is a method of treating abnormal sensory conditions such as dysaesthesia, allodynia and hyperalgesia, in an individual, comprising administering to the individual a therapeutically effective amount of adipose derived stem and regenerative cells, wherein the therapeutically effective amount is an amount sufficient to cause a detectable improvement in said pain.

In a specific example, said method comprises identifying an individual in need of pain relief, or an individual suffering from pain. In another example, said method additionally comprises determining one or more first levels of pain in said individual prior to administration of said adipose derived stem and regenerative cells, and determining one or more second levels of pain in said individual after administration of said adipose derived stem and regenerative cells, wherein said therapeutically effective amount of adipose derived stem and regenerative cells reduces said one or more second levels of said pain as compared to said one or more first level of pain.

In a more specific example, said therapeutically effective amount of adipose derived stem and regenerative cells results in a detectable improvement in said pain that is greater than, or more long-lasting than, improvement due to administration of a placebo.

In a more specific example, said one or more first levels of pain and said one or more second levels of pain are determined by a pain assessment scale. In a more specific example, said pain assessment scale is the Numeric Pain Intensity Scale; the Pain Quality Assessment Scale; the Simple Descriptive Pain Intensity Scale; the Visual Analog
Scale; the Wong-Baker FACES Pain Rating Scale; the FLACC scale; the CRIES scale; or the COMFORT scale.

In another specific example, said method additionally comprises determining a first level of one or more physiological indicia of pain in said individual prior to administration of said adipose derived stem and regenerative cells, and determining a second level of one or more physiological indicia of pain in said individual after administration of said adipose derived stem and regenerative cells, wherein said therapeutically effective amount of adipose derived stem and regenerative cells reduces said second level as compared to said first level.

In a more specific example, said physiological indicium of pain is heart rate in the individual. In a more specific example, said heart rate in said individual is lower after said administration compared to said heart rate in said individual before said administration. In another more specific embodiment, said physiological indicium of pain is the systolic of said individual. In a more specific embodiment, said systolic of said individual is lower after said administration compared to said systolic in said individual before said administration. In another more specific embodiment, said physiological indicium of pain is the diastolic of said individual. In a more specific embodiment, said diastolic of said individual is lower after said administration compared to said diastolic in said individual before said administration.

In another embodiment of the method of treating pain, said pain is neuropathic pain. In a specific example, said neuropathic pain is caused by diabetic neuropathy. In another specific example, said neuropathic pain is caused by injury to a nerve in said individual. In another specific example, said neuropathic pain is caused by a drug. In certain specific examples, said drug is or comprises a platinum-containing anticancer drug, e.g., oxaliplatin, carboplatin or cisplatin, or another chemotherapeutic drug such as paclitaxel or vincristine. In another example, the neuropathic pain is caused by a virus, e.g., a viral disease such as varicella zoster, herpes (e.g., herpes simplex) or human immunodeficiency virus (HIV). Yet in another example the pain is cause by radiation injury, e.g., radiation injury that is part of cancer treatment. In another specific example, said neuropathic pain is caused by inflammation, e.g., neuroinflammation, neuritis.

In another example of the method of treating pain, said pain is inflammatory pain. In another example, said pain is bone pain. In a specific example, said bone pain is associated with or caused by cancer. In another example, said pain is caused by cancer. In another example, said. pain caused by or associated with vulvodynia, pain caused by or associated with interstitial cystitis, or pain caused by degenerative joint disease such as osteoarthritis. In certain embodiments, said pain is unresponsive to steroid therapy. In certain other examples, said pain is unresponsive to nonsteroidal anti-inflammatory therapy. In certain other examples, said pain is unresponsive to opioid therapy. In certain other examples, said pain is unresponsive to non-specific or mixed mu/delta opioids therapy.

In a specific embodiment of any of the above embodiments, said adipose derived stem and regenerative cells comprises cells that are CD31-. In a more specific embodiment, said adipose derived stem and regenerative cells are additionally CD34+, e.g., the adipose derived stem and regenerative cells are CD31- and CD34+. In a more specific embodiment, said adipose derived stem and regenerative cells are additionally CD45-. In other words, in some embodiments, the cells are CD31-, CD34+, CD45-. In another embodiment, said adipose derived stem and regenerative cells are CD146+. In some embodiments, the cells are CD31-, CD34+, CD45-, CD146+. In another embodiment said adipose derived stem and regenerative cells comprise cells that are CD45+ and in a more specific embodiment are CD45+ and CD206+.

In certain embodiments, said adipose derived stem and regenerative cells are formulated to be administered locally, e.g., topically or by local injection (subcutaneous, intramuscular, and the like). In certain other embodiments, said adipose derived stem and regenerative cells are formulated to be administered systemically, e.g., intravenously or intraarterially or via the lymphatic system.

Preferred examples include methods of modulating pain in a subject in need thereof, comprising administering a therapeutically effective amount of adipose derived regenerative cells to said subject. In some examples, these methods further comprise selecting, identifying, or classifying said subject as an individual experiencing pain. In some examples, said selection, identification, or classification is made by a physician or clinical or diagnostic evaluation. In some examples, said methods further comprise determining or measuring pain in said subject before, after, or during administration of said adipose derived regenerative cells.

Other examples concern methods of modulating fibrosis in a subject in need thereof, comprising administering a therapeutically effective amount of adipose derived cells comprising regenerative cells to said subject. In some examples, said methods further comprise selecting, identifying, or classifying said subject as an individual experiencing fibrosis or having a fibrotic disease. In some examples, said selection, identification, or classification is made by a physician or clinical or diagnostic evaluation. In some examples, said methods further comprise determining or measuring fibrosis or a marker thereof, such as the amount of or level of TNF-alpha, in said subject or in a biological sample obtained from said subject before, after, or during administration of said adipose derived regenerative cells. Additional examples concern methods of improving range of motion in a joint in a subject in need thereof, comprising administering a therapeutically effective amount of adipose derived cells comprising regenerative cells to said subject. In some examples, said methods further comprise selecting, identifying, or classifying said subject as an individual experiencing a loss of joint mobility or a reduction in the range of motion of a joint. In some examples, said selection, identification, or classification is made by a physician or clinical or diagnostic evaluation. In some examples, said methods further comprise determining or measuring the mobility or range of motion of a joint of said subject before, after, or during administration of said adipose derived regenerative cells. In any of the aforementioned embodiments, said adipose-derived regenerative cells can comprise stem cells. In any of the aforementioned embodiments, said adipose-derived regenerative cells can comprise precursor cells. In any of the aforementioned embodiments, said adipose-derived regenerative cells can comprise progenitor cells.

### DEFINITIONS

As used herein, the term "about," when referring to a stated numeric value, indicates a value within plus or minus 10% of the stated numeric value.

As used herein, the term "derived" means isolated from or otherwise purified. For example, adipose derived stem and regenerative cells are isolated from adipose tissue. The term "derived" does not encompass cells that are extensively cultured (e.g., placed in culture conditions in which the majority of dividing cells undergo 3, 4, 5 or less, cell doublings), from cells isolated directly from a tissue, e.g., adipose tissue, or cells cultured or expanded from primary isolates. Accordingly, "adipose derived stem and regenerative cells" refers to cells obtained from adipose tissue, wherein the cells are not extensively cultured.

As used herein, a cell is "positive" for a particular marker when that marker is detectable. For example, an adipose derived regenerative cell is positive for, e.g., CD73 because CD73 is detectable on an adipose derived stem or regenerative cell in an amount detectably greater than background (in comparison to, e.g., an isotype control or an experimental negative control for any given assay). A cell is also positive for a marker when that marker can be used to distinguish the cell from at least one other cell type, or can be used to select or isolate the cell when present or expressed by the cell.

In some contexts, the term "adipose tissue" refers to a tissue containing multiple cell types including adipocytes and vascular cells. Adipose tissue includes multiple regenerative cell types, including adult stem cells (ASCs) and endothelial progenitor and precursor cells. Accordingly, adipose tissue refers to fat, including the connective tissue that stores the fat.

In some contexts, the term "unit of adipose tissue" refers to a discrete or measurable amount of adipose tissue. A unit of adipose tissue may be measured by determining the weight and/or volume of the unit. In reference to the disclosure herein, a unit of adipose tissue may refer to the entire amount of adipose tissue removed from a subject, or an amount that is less than the entire amount of adipose tissue removed from a subject. Thus, a unit of adipose tissue may be combined with another unit of adipose tissue to form a unit of adipose tissue that has a weight or volume that is the sum of the individual units.

In some contexts, the term "portion" refers to an amount of a material that is less than a whole. A minor portion refers to an amount that is less than 50%, and a major portion refers to an amount greater than 50%. Thus, a unit of adipose tissue that is less than the entire amount of adipose tissue removed from a subject is a portion of the removed adipose tissue.

As used herein, "regenerative cells" refers to any heterogeneous or homologous cells obtained using the systems and methods of embodiments disclosed herein which cause or contribute to complete or partial regeneration, restoration, or substitution of structure or function of an organ, tissue, or physiologic unit or system to thereby provide a therapeutic, structural or cosmetic benefit. Examples of regenerative cells include: ASCs, endothelial cells, endothelial precursor cells, endothelial progenitor cells, macrophages, fibroblasts, pericytes, smooth muscle cells, preadipocytes, differentiated or de-differentiated adipocytes, keratinocytes, unipotent and multipotent progenitor and precursor cells (and their progeny), and lymphocytes.

In some contexts, the term "progenitor cell" refers to a cell that is unipotent, bipotent, or multipotent with the ability to differentiate into one or more cell types, which perform one or more specific functions and which have limited or no ability to self-renew. Some of the progenitor cells disclosed herein may be pluripotent.

In some contexts, the term "adipose tissue-derived cells" refers to cells extracted from adipose tissue that has been processed to separate the active cellular component from the mature adipocytes and connective tissue. Separation may be partial or full. That is, the "adipose tissue-derived cells" may or may not contain some adipocytes and connective tissue and may or may not contain some cells that are present in aggregates or partially disaggregated form (for example, a fragment of blood or lymphatic vessel comprising two or more cells that are connected by extracellular matrix). This fraction is referred to herein as "adipose tissue-derived cells," "adipose derived cells," "adipose derived regenerative cells" or "ADC." Typically, ADC refers to the pellet of cells obtained by washing and separating the cells from the adipose tissue. The pellet is typically obtained by centrifuging a suspension of cells so that the cells aggregate at the bottom of a centrifuge container, or alternatively concentrating the cells in a different manner.

### BRIEF DESCRIPTION OF THE DRAWINGS

**FIGURE 1** is a photograph showing a patient's fingers being marked at the site of injection of the adipose-derived cells, as described in Example 1, below.
**FIGURE 2** is a photograph showing an incision being made at an injection site, as described in Example 1, below.
**FIGURE 3** is a photograph showing delivery of the adipose-derived cells to the site of injection, as described in Example 1, below.
**FIGURE 4** is a graph showing patients' Raynaud's severity scores at the time of treatment, and two months following treatment, as described in Example 1 below.
**FIGURE 5** is a bar graph showing the number of study patients showing a >30% worsening in Raynaud's severity scores, 10-33% worsening in Raynaud's severity scores, unchanged Raynaud's severity scores (-10% to +10%), improved Raynaud's severity scores (10%-33%), and major improvement in Raynaud's severity scores (>33%).
**FIGURE 6** is a graph showing the Scleroderma Health Assessment Questionnaire (SHAQ) scores of the patients at baseline (time of treatment) and two months following treatment, as described in Example 1, below.
**FIGURE 7** is a bar graph showing the number of study patients showing a >30% worsening in SHAQ scores, 10-33% worsening in SHAQ scores, unchanged SHAQ scores (-10% to +10%), improved SHAQ scores (10%-33%), and major improvement in SHAQ scores (>33%).
**FIGURE 8** is a graph showing patients' Cochin scores at the time of treatment, and two months following treatment, as described in Example 1 below.

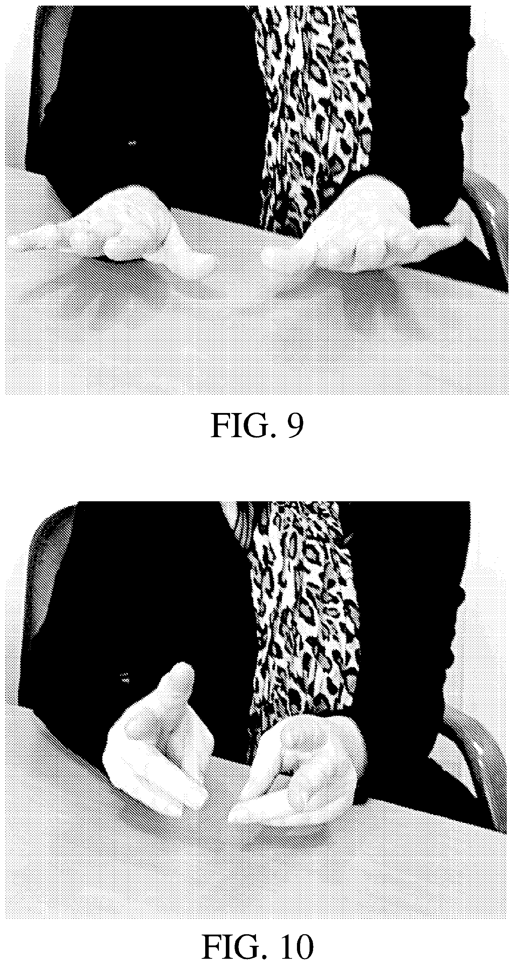

**FIGURE 9** is a photograph of a study patient's hands depicting the movement associated with typing two months following treatment, as described in Example 1 below.
**FIGURE 10** is a photograph of a study patient's hands depicting hand movements used in manipulation of scissors two months following treatment, as described in Example 1 below.
**FIGURE 11** is a graph showing baseline, 2 month follow up, and 6 month follow up scores for hand pain (visual analogue scale "VAS"); Cochin Hand Function Scale ("CHFS"), Raynaud's Condition Score ("RCS"); and Scleroderma Health Assessment Questionnaire ("SHAQ"), as described in Example 1, below.

### DETAILED DESCRIPTION OF THE PREFERRED EMBODIMENT

### Methods of Treatment of Pain

Described herein are methods of treating pain comprising the administration of adipose derived stem and regenerative cells, e.g., adipose derived stem and regenerative cells as described and prepared herein and in United States Patent Nos. 7,390,484.

Pain is generally defined as an unpleasant sensory and emotional experience associated with actual or potential tissue damage or described in terms of such damage. Merskey H, Bogduk N, eds., Classification of Chronic Pain, International Association for the Study of Pain (IASP) Task Force on Taxonomy, IASP Press: Seattle, 209-214, 1994. Because the perception of pain is highly subjective, it is one of the most difficult pathologies to diagnose and treat effectively. Pain may be acute, meaning that it is induced immediately following a stimulus (e.g., exposure to flame, sharp or blunt impact, or sudden ischemic event), or chronic, meaning that it is persistent and/or recurrent at relatively frequent intervals.

In one example, provided herein is a method of treating an individual having pain, comprising administering to the individual a therapeutically effective amount of adipose derived cells, *e.g*., adipose-derived cells comprising stem cells, adipose-derived cells comprising regenerative cells, adipose-derived cells comprising stem and regenerative cells wherein the therapeutically effective amount is an amount sufficient to cause a detectable improvement in said pain or a symptom associated with said pain. In one example, a patient is identified as being in need of suppression or treatment of pain. In one example, said method additionally comprises determining a first level of pain in said individual prior to administration of said adipose derived stem and regenerative cells, and determining a second level of pain in said individual after administration of said adipose derived stem and regenerative cells, wherein said therapeutically effective amount of adipose derived stem and regenerative cells reduces said second level of said pain as compared to said first level of pain.

In certain embodiments, the therapeutically effective amount of adipose derived stem and regenerative cells, when administered, results in greater, or more long-lasting, improvement of pain in the individual as compared to administration of a placebo.

In certain examples, the pain is nociceptive pain. Nociceptive pain is typically elicited when noxious stimuli such as inflammatory chemical mediators are released following tissue injury, disease, or inflammation and are detected by normally functioning sensory receptors (nociceptors) at the site of injury. See, e.g., Koltzenburg, M. Clin. J. of Pain 16:S131-S138 (2000). Examples of causes of nociceptive pain include, but are not limited to, chemical or thermal burns, cuts and contusions of the skin, osteoarthritis, rheumatoid arthritis, tendonitis, and myofascial pain. In certain examples, nociceptive pain is stimulated by inflammation.

In certain other examples, the pain is neuropathic pain. Neuropathic pain reflects injury or impairment of the nervous system, and has been defined as "pain initiated or caused by a primary lesion or dysfunction in the nervous system." Merskey H, Bogduk N, eds., Classification of Chronic Pain, International Association for the Study of Pain (IASP) Task Force on Taxonomy, IASP Press: Seattle, 209-214, 1994. In a specific example, the neuropathic pain is characterized by altered excitability of peripheral neurons. In other specific examples, the neuropathic pain includes, but is not limited to, pain associated with diabetic neuropathy, postherpetic neuralgia, trigeminal neuralgia, inflammation (e.g., neuroinflammation, neuritis), and post-stroke pain. In certain examples, the neuropathic pain is continuous, episodic, and is described as, e.g., burning, tingling, prickling, shooting, electric-shock-like, jabbing, squeezing, deep aching, or spasmodic. In certain other examples, the individual having neuropathic pain additionally experiences partial or complete sensory deficit, abnormal or unfamiliar unpleasant sensations (dysaesthesia), pain resulting from non-noxious stimuli, or disproportionate perception of pain in response to supra-threshold stimuli (hyperalgesia).

In another specific example, the neuropathic pain is complex regional pain syndrome (CRPS). In a specific example, CRPS affects the extremities in the absence of a nerve injury (CRPS type I). In a more specific example, said CRPS type I includes reflex sympathetic dystrophy (RSD). In a more specific example, said RSD is stage I RSD, or "early RSD". In early RSD, pain is more severe than would be expected from the injury, and it has a burning or aching quality. It may be increased by dependency of the limb, physical contact, or emotional upset. The affected area typically becomes edematous, may be hyperthermic or hypothermic, and may show increased nail and hair growth. Radiographs may show early bony changes. In another more specific example, said RSD is stage II RSD, or "established RSD". In a more specific example, said established RSD comprises, in addition to pain, induration of edematous tissue; hyperhidrosis of skin with livedo reticularis or cyanosis; hair loss; ridging, cracking or brittling of nails; development of dry hands; and/or noticeable atrophy of skin and subcutaneous tissues. Pain remains the dominant feature. In another more specific example, said RSD is stage III RSD, or "late RSD". In a more specific example, said late RSD comprises pain that spreads proximally; irreversible tissue damage; thin, shiny skin; and bone demineralization visible on radiographs.

In another specific example, the neuropathic pain is pain caused by a drug, e.g., a chemotherapeutic drug or anti-cancer drug. In specific examples, the drug is or comprises a platinum-containing drug, a taxane, an epothilone, a plant alkaloid, or a thalidomide. In more specific examples, the drug is or comprises bortezomib, carboplatin (e.g., PARAPLATIN®), cisplatinum (e.g., PLATINOL®), cytarabine (e.g., CYTOSAR®, Ara-C), docetaxel (e.g., TAXOTERE®), etoposide/VP-16 (VEPESID®), gemcitibine (e.g., GEMZAR®), HALAVEN® (eribulin mesylate), hexamethylmelamine (e.g., HEXALIN®), paclitaxel (e.g., TAXOL®; ABRAXANE™), oxaliplatin (e.g., ELOXATIN®), suramin, thalidomide (e.g., THALOMID®), vinblastine (e.g., VELBAN®; ALKABAN-AQ®), vincristine (e.g., ONCOVIN®, VINCASAR PFS®, Vincrex), or vinorelbine (NAVELBINE®).

In certain other specific examples, the drug is an antibiotic. In certain other examples, the drug is a statin.

In certain other specific examples, the drug is or comprises amlodipine (e.g., NORVASC®, Lotril or Lotrel), atorvastatin (e.g., LIPITOR®), duloxetine (e.g., CYMBALTA®), pregabalin (LYRICA®), allopurinol (e.g., LOPURIM®, ZYLOPRIM®), aminodipinberglate, amiodarone (e.g., CORDERONE®, PACERONE®), amiodipine, amitriptyline (e.g., ELAVIL™, ENDEP™, VANATRIP™), metronidazole (e.g., FLAGYL®, METROGEL™), nitrofurantoin (e.g., FURADANTIN®, MACROBID®, MACRODANTIN®, NITRO MACRO), perhexyline, VYTORIN®, ciprofloxacin (e.g., CIPRO®, PROQUIN®), disulfuram (e.g., ANTABUSE), zolpidem (e.g., AMBIEN®), buspirone (e.g., BUSPAR), clonazepam (e.g., KLONOPIM, CEBERKLON, VALPAX), alaprazolam (e.g., XANAX®), phenyloin (DILANTIN®), citalopram (e.g., CELEXA), duloxetine (e.g., CYMBALTA®), venlaxafine (e.g., EFFEXOR, EFFEXOR XR®), nortriptyline (e.g., AVENTYL HCL, PAMELOR), sertraline (e.g., ZOLOFT®), paroxetine (e.g., PAXIL, PAXIL CR®), atenolol (e.g., TENORMIN, SENORMIN), perindopril (e.g., ACEON), altace (e.g., RAMIPRIL®), losartan (e.g., COZAAR®, HYZAAR®), hydralazine (e.g., APRESOLINE®), hydrochlorothiazide (e.g., HYDRODIURIL™, EZIDE™, HYDRO-PAR™, MICROZIDE™), lisinopril (e.g., PRINOVIL®, ZESTRIL®), telmisartan (e.g., MICARDIS™), perhexyline, prazosin (e.g., MINIPRESS®), lisinopril (e.g., PRINIVIL®, ZESTRIL®), lovastatin (e.g., ALTOCOR®, MEVACOR®), CADUET®, rosuvatatin (e.g., CRESTOR®), fluvastatin (e.g., LESCOL®, LESCOL® XL), simvastatin (e.g., ZOCOR®), cerivastatin (e.g., LIPOBAY™), gemfibrozil (e.g., LOPID®), pravastatin (e.g., PRAVACHOL®, PRAVIGARD PAC™), d4T (stavudine, e.g., ZERIT®), ddC (zalcitibine; e.g., HIVID®), ddI (didanosine, e.g., VIDEX® EC), isoniazid (e.g., TUBIZID®), diaminodiphenylsulfone (DDS, dapsone)

In certain examples, the neuropathic pain is not pain caused by a drug, e.g., a chemotherapeutic drug or anti-cancer drug. In specific examples, the neuropathic pain is not pain caused by a platinum-containing drug, a taxane, an epothilone, a plant alkaloid, or a thalidomide. In more specific examples, the neuropathic pain is not pain caused by bortezomib, carboplatin (e.g., PARAPLATIN®), cisplatinum (e.g., PLATINOL®), cytarabine (e.g., CYTOSAR®, Ara-C), docetaxel (e.g., TAXOTERE®), etoposide/VP-16 (VEPESID®), gemcitibine (e.g., GEMZAR®), HALAVEN® (eribulin mesylate), hexamethylmelamine (e.g., HEXALIN®), paclitaxel (e.g., TAXOL®; ABRAXANE™), oxaliplatin (e.g., ELOXATIN®), suramin, thalidomide (e.g., THALOMID®), vinblastine (e.g., VELBAN®; ALKABAN-AQ®), vincristine (e.g., ONCOVIN®, VINCASAR PFS®, Vincrex), or vinorelbine (NAVELBINE®).

In another specific example, said CRPS affects the extremities in the presence of a nerve injury (CRPS type II). In a more specific example, said CRPS II includes causalgia. In another specific example, said CRPS includes sympathetic maintained pain syndrome. In certain examples, symptoms of CRPS include but are not limited to pain, autonomic dysfunction, edema, movement disorder, dystrophy, atrophy, burning pain, allodynia (pain with light touch). In certain examples, CRPS-related pain is accompanied by swelling and joint tenderness, increased sweating, sensitivity to temperature, and/or color change of the skin.

In certain other specific examples, the neuropathic pain is neuropathic pain caused by or related to a dietary deficiency. In a more specific example, the dietary deficiency is vitamin B12 (cobalamin, cyanocobalamin) deficiency. In another more specific example, the dietary deficiency is vitamin B6 (pyridoxine, pyridoxal phosphate) deficiency. In another more specific example, the dietary deficiency is vitamin B1 (thiamine) deficiency. In another specific example, the individual having neuropathic pain, caused by nutritional deficiency, has had bariatric surgery. In another specific example, the neuropathic pain is caused by or is related to alcoholism or consumption of alcohol by the individual having pain.

In certain examples, the pain is caused by or associated with vulvodynia. Vulvodynia is pain of the vulva, e.g., pain unexplained by vulvar or vaginal infection or skin disease. In one example, the pain of vulvodynia is localized to the vulvar region, e.g., in the vestibular region such as vulvar vestibulitis or vestibulodynia. In another example, the pain of vulvodynia may extend into the clitoris, e.g., clitorodynia. Example of causes of vulvodynia include, but are not limited to, dyspareunia, injury to or irritation of the nerves that innervate the vulva, genetic predisposition to inflammation, allergy, autoimmune disorders (e.g., lupus erythematosus or Sjogren's Syndrome), infection (e.g., yeast infections, HPV or bacterial vaginosis), and neuropathy. Exemplary symptoms of vulvodynia include without limitation, diffuse pain or burning sensation on or around the vulva, the labia majora, labia minor, or the vestibule.

In certain examples, the pain is caused by or associated with interstitial cystitis. Interstitial cystitis, also known as bladder pain syndrome, is a chronic condition, often characterized by, e.g., pain or pressure associated with the bladder, pain associated with urination, irritative voiding, urinary frequency, urgency, or pain or pressure in pelvis. The pathology and pathogenesis of interstitial cystitis is not clearly understood. However, several possible causes have been proposed, e.g., vascular obstruction, autoimmunity, inflammation, leaky bladder lining, mast cells, stress, and genetic, neurogenic and endocrine causes. In one example, diagnosis of interstitial cystitis can be done by, e.g., the Pelvic Pain Urgency/Frequency (PUF) Patient Survey or the KCl test, also known as the potassium sensitivity test.

In certain other examples, the pain is visceral pain.

In certain other examples, the pain is post-operative pain, such as that resulting from trauma to tissue caused during surgery.

In certain other examples, the pain is mixed pain, e.g., is chronic pain that has nociceptive and neuropathic components. In specific examples, said mixed pain is cancer pain or low back pain.

In certain other examples, the pain is migraine pain or pain from headache, e.g., vascular headache, cluster headache or toxic headache.

In some examples, the pain is from systemic sclerosis or scleroderma, e.g., diffuse or systemic scleroderma. In yet other embodiments, the pain is from Raynaud's disease.

In specific examples, said symptoms associated with pain include, but are not limited to, one or more of autonomic dysfunction, inability to initiate movement, weakness, tremor, muscle spasm, dystonia, dystrophy, atrophy, edema, stiffness, joint tenderness, increased sweating, sensitivity to temperature, light touch (allodynia), color change to the skin, hyperthermic or hypothermic, increased nail and hair growth, early bony changes, hyperhidrotic with livedo reticularis or cyanosis, lost hair, ridged, cracked or brittle nails, dry hand, diffuse osteoporosis, irreversible tissue damage, thin and shiny skin, joint contractures, and marked bone demineralization.

In certain embodiments, the administration of adipose derived cells, *e.g.,* adipose-derived cells comprising stem cells, adipose-derived cells comprising regenerative cells, adipose-derived cells comprising stem and regenerative cells, and the like, to an individual in accordance with the methods described herein results in a reduction in pain in the individual without an accompanying side effect that is associated with one or more drugs indicated/used for treatment of pain, e.g., gabapentin. In a specific embodiment, the use of adipose derived cells, e.g., adipose-derived cells comprising stem cells, adipose-derived cells comprising regenerative cells, adipose-derived cells comprising stem and regenerative cells, and the like, in accordance with the methods described herein results in reduction of pain in an individual to whom the adipose derived stem and regenerative cells are administered, but does not result in sensory and/or motor coordination deficiency in said individual.

### Pain Assessment Scales

In one embodiment, the therapeutically effective amount of adipose derived cells, *e.g*., adipose-derived cells comprising stem cells, adipose-derived cells comprising regenerative cells, adipose-derived cells comprising stem and regenerative cells, and the like, is administered to the individual having pain is an amount that results in a detectable reduction in the pain in the individual. The reduction can be detectable to the individual, detectable to an observer, or both. In certain embodiments of the methods of treatment provided herein, the level of pain in the individual is assessed by the individual, e.g., as guided by a medical doctor, or as part of a pre-treatment workup, according to one or more individual pain scales. In certain other embodiments, the level of pain in the individual is assessed by an observer using one or more observer pain scales. Where levels of pain are assessed according to the method before and after administration of adipose derived stem and regenerative cells, the same scale is preferably used for each assessment. Pain in the individual can be assessed once or more than once, e.g., 2, 3, 4, or 5 times, before administration of adipose derived stem and regenerative cells, and once or more than once, e.g., 2, 3, 4, or 5 times, after administration of adipose derived stem and regenerative cells.

In one embodiment, pain in the individual is assessed by the 0-10 Numeric Pain Intensity Scale. In this scale, zero equals no pain, and 10 equals the worst pain. In certain embodiments, e.g., the Pain Quality Assessment Scale, the pain is broken down into more than one numeric descriptor, e.g., 0-10 for how "hot" the pain feels, 0-10 for how "intense" the pain feels, 0-10 for how "sharp" the pain feels, 0-10 for how "dull" the pain feels, 0-10 for how "cold" the pain feels, 0-10 for how "sensitive" the pain feels, 0-10 for how "tender" the pain feels, 0-10 for how "itchy" the pain feels, 0-10 for how "shooting" the pain feels, 0-10 for how "numb" the pain feels, 0-10 for how "tingling" the pain feels, 0-10 for how "electrical" the pain feels, 0-10 for how "cramping" the pain feels, 0-10 for how "throbbing" the pain feels, 0-10 for how "radiating" the pain feels, 0-10 for how "aching" the pain feels, 0-10 for how "heavy" the pain feels, and/or 0-10 for how "unpleasant" the pain feels.

In another embodiment, pain in the individual is assessed by the Simple Descriptive Pain Intensity Scale. In this scale, pain is described as, e.g., "no pain", "mild pain", "moderate pain", "severe pain", "very severe pain" or "worst possible pain".

In another embodiment, pain in the individual is assessed by the Visual Analog Scale. In the Visual Analog Scale, the individual is presented with a graph consisting of a vertical line; one end of the line is labeled "no pain" and the other end is labeled "worst possible pain". The individual is asked to mark the line at a point between the two ends indicating the level of pain perceived by the individual.

In another embodiment, pain in the individual is assessed by the Wong-Baker FACES Pain Rating Scale. In the FACES Pain Rating Scale, the level of pain is indicated by a series of cartoon faces, typically six faces, appearing happy to progressively more unhappy. In a specific embodiment, the faces are subtexted with phrases such as "no hurt", "hurts little bit" "hurts little more", "hurts even more", "hurts whole lot" and "hurts worst". In another specific embodiment, the faces are subtexted with phrases such as "no pain", "mild, annoying pain", "nagging, uncomfortable, troublesome pain", "distressing, miserable pain", "intense, dreadful, horrible pain" and "worst possible, unbearable, excruciating pain", either alone or accompanied by a numeric 0 to 10 scale.

In certain embodiments, pain in the individual is assessed by the FLACC (Face, Legs, Activity, Cry and Consolability) scale. In specific embodiments, each of the five characteristics is rated from, e.g., 0 to 2, with 2 indicating pain and 0 indicating no pain. The scores may be used separately or totaled.

In certain other embodiment, pain in the individual is assessed by the CRIES (Crying, Requires O2 for SaO2 (hemoglobin saturation), Increased vital signs (blood pressure and heart rate, Expression and Sleepless) scale. In specific embodiments, each of the five characteristics is rated from, e.g., 0 to 2, with 2 indicating pain and 0 indicating no pain. The scores may be used separately or totaled.

In certain embodiment, pain in the individual is assessed by the COMFORT scale, which assesses nine different characteristics (alertness, calmness, respiratory distress, crying, physical movement, muscle tone, facial tension, blood pressure and heart rate), each rated on a scale of 1-5, with 1 indicating no or least pain, and 5 most pain. The scores may be used individually or totaled.

### Physiological Indicia of Pain

As used herein, "treatment of pain" and the like can comprise completely eliminating pain; noticeable reduction of pain by the individual suffering the pain; detectable reduction of pain or indicia of pain by objective criteria (e.g., heart rate, blood pressure, muscle tone, or the like); or a combination of any two or all three. In certain other embodiments, pain in the individual can be assessed, either before or after administration of adipose derived stem and regenerative cells, or both, by physiological criteria, e.g., physiological criteria of stress. Such physiological criteria can include objectively measurable criteria such as heart rate or blood pressure, e.g., elevated heart rate or blood pressure as compared to a non-pain state in the individual, or as compared to an expected norm (*e.g*., 120 systolic and 80 diastolic; 60 beats per minute), or elevated levels of stress hormones, *e.g*., cortisol, norepinephrine, or the like. Such physiological criteria can also, or instead, include subjectively measurable criteria such as facial expressions, muscle tensioning (muscle tone), sweating, trembling, and the like.

Thus, in certain examples, the therapeutically effective amount of adipose derived stem and regenerative cells, administered to the individual having pain, results in a detectable reduction in heart rate in the individual, e.g., a 5%, 10%, 15%, 20%, 25%, 30%, 35%, 40%, 45% or 50% reduction; a reduction of heart rate from 120 beats per minute (bpm) or above to below 110 bpm; a reduction from 110 bpm or above to below 100 bpm; a reduction from 100 bpm or above to below 90 bpm; a reduction from 90 bpm or above to below 80 bpm; a reduction from 120 bpm or above to below 100 bpm; a reduction from above to below 90 bpm; a reduction from 100 bpm above to below 80 bpm; a reduction from 130 bpm above to below 100 bpm; a reduction from 120 bpm above to below 90 bpm; a reduction from 110 bpm to below 80 bpm; or a reduction from 120 bpm or above to below 80 bpm.

In certain other examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable reduction in blood pressure in the individual, e.g., a reduction of 5%, 10%, 15%, 20%, 25%, 30%, 35%, 40%, 45% or 50% reduction in the individual's systolic, diastolic, or both; a reduction in the individual's systolic from 200 or above to under 190; a reduction in the systolic from 190 or above to under 180; a reduction in the systolic from 180 or above to under 170; a reduction in the systolic from 170 or above to under 160; a reduction in the systolic from 160 or above to under 150; a reduction in the systolic from 150 or above to under 140; a reduction in the systolic from 140 or above to under 130; a reduction in the systolic from 200 or above to under 180; a reduction in the systolic from 190 or above to under 170; a reduction in the systolic from 180 or above to under 160; a reduction in the systolic from 170 or above to under 150; a reduction in the systolic from 160 or above to under 140; a reduction in the systolic from 150 or above to under 130; a reduction in the systolic from 200 or above to under 170; a reduction in the systolic from 190 or above to under 160; a reduction in the systolic from 180 or above to under 150; a reduction in the systolic from 170 or above to under 140; a reduction in the systolic from 160 or above to under 130; a reduction in the systolic from 200 or above to under 160; a reduction in the systolic from 190 or above to under 150; a reduction in the systolic from 180 or above to under 140; a reduction in the systolic from 200 or above to under 130; a reduction in the systolic from 200 or above to under 150; a reduction in the systolic from 190 or above to under 140; a reduction in the systolic from 180 or above to under 130; a reduction in the systolic from 200 or above to under 140; a reduction in the systolic from 190 or above to under 130; or a reduction in the systolic from 200 or above to under 130; a reduction in the individual's diastolic from 140 or above to under 130; a reduction in the diastolic from 130 or above to under 120; a reduction in the diastolic from 120 or above to under 110; a reduction in the diastolic from 110 or above to under 100; a reduction in the diastolic from 100 or above to under 90; a reduction in the diastolic from 140 or above to under 120; a reduction in the diastolic from 110 or above to below 90; a reduction in the diastolic from 140 or above to below 110; a reduction in the diastolic from 130 or above to under 100; a reduction in the diastolic from 120 or above to under 90; a reduction in the diastolic from 140 or above to below 100; a reduction in the diastolic from 130 or above to below 90; or a reduction in the diastolic from 140 or above to below 90.

In certain examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable reduction in the amount of one or more cytokines (e.g., pro-inflammatory cytokines) in the individual. In a specific example, administration of adipose derived cells, *e.g*., adipose-derived cells comprising stem cells, adipose-derived cells comprising regenerative cells, adipose-derived cells comprising stem and regenerative cells, and the like, to individuals having pain in accordance with the methods described herein results in a decrease in the amount of TNF-α, IL-2, IL-3, IL-6, IL-12, IL-17, IL-18, and/or interferon-γ, or any combination thereof, in the individual. Assessment of decreases in cytokines in the individual can be accomplished using any method known in the art, e.g., the cytokine levels in the blood plasma of the individual can be measured using, e.g., ELISA.

In certain examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable increase in one or more cytokines in the individual. In a specific example, administration of adipose derived stem and regenerative cells to individual having pain in accordance with the methods described herein results in an increase in the amount of IL-10 in the individual. Assessment of increases in cytokine levels in the individual can be accomplished using any method known in the art, e.g., the cytokine levels in the blood plasma of the individual can be measured using, e.g., ELISA.

In certain examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable increase in one or more stress hormones in the individual. In a specific example, administration of adipose derived cells, *e.g*., adipose-derived cells comprising stem cells, adipose-derived cells comprising regenerative cells, adipose-derived cells comprising stem and regenerative cells, and the like, to individual having pain in accordance with the methods described herein results in a decrease in the amount of cortisol in the individual. Other exemplary stress hormones include, but are not limited to, corticotrophin-releasing factor, adrenocorticotropin, 8-lipotropin, 3-endorphin, vasopressin, prolactin, and the like. Assessment of decrease in cortisol levels in the individual can be accomplished using any method known in the art, e.g., the cortisol levels in the saliva or blood plasma or serum of the individual can be measured using, e.g., ELISA or any other method known to those in the art.

In certain examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable reduction in the amount of one or more chemokines in the individual. In a specific example, administration of adipose derived cells, *e.g*., adipose-derived cells comprising stem cells, adipose-derived cells comprising regenerative cells, adipose-derived cells comprising stem and regenerative cells, and the like, to individual having pain in accordance with the methods described herein results in a decrease in the amount of CCL2, CCL12, and/or CXCL1, or any combination thereof, in the individual. Assessment of chemokines in the individual can be accomplished using any method known in the art, e.g., the chemokine levels in the blood plasma of the individual can be measured using, e.g., ELISA or any other method known to those int heart.

In certain examples, the therapeutically effective amount of adipose derived cells, *e.g*., adipose-derived cells comprising stem cells, adipose-derived cells comprising regenerative cells, adipose-derived cells comprising stem and regenerative cells, and the like, when administered to the individual having pain, results in a detectable reduction in the activation and/or differentiation in one or more cell types in the individual. In a specific example, administration of adipose derived cells, *e.g*., adipose-derived cells comprising stem cells, adipose-derived cells comprising regenerative cells, adipose-derived cells comprising stem and regenerative cells, and the like, to individual having pain in accordance with the methods described herein results in a decrease in the activation and/or differentiation of dendritic cells, T cells, and/or macrophages, or any combination thereof, in the individual. Assessment of the activation and/or differentiation of specific cell types in the individual can be accomplished using any method known in the art, e.g., measurement of specific cell markers present in specific areas of the individual, e.g., measurement/assessment of specific cell markers associated with cells of the blood, or associated with cells found in specific tissues/organs.

### Suppression of an Inflammatory Response Associated with, or Causative of, Pain

Inflammation is not a sole source of pain. However, in certain examples, adipose derived stem and regenerative cells can be used to ameliorate pain related to, or caused by, inflammation. In one example, provided herein is a method for the amelioration of pain in an individual comprising contacting immune cell(s) in the individual with an effective amount of adipose derived cells, *e.g*., adipose-derived cells comprising stem cells, adipose-derived cells comprising regenerative cells, adipose-derived cells comprising stem and regenerative cells, and the like, wherein said effective amount is an amount that (1) detectably suppresses an immune response in said individual, and (2) detectably reduces pain in said individual. In specific examples, said adipose derived stem and regenerative cells detectably suppress T cell proliferation in a mixed lymphocyte reaction (MLR) assay or a regression assay. The contacting can be accomplished by, e.g., administering the adipose derived cells, *e.g.,* adipose-derived cells comprising stem cells, adipose-derived cells comprising regenerative cells, adipose-derived cells comprising stem and regenerative cells, and the like, to the individual, e.g., locally, systemically, or regionally (or by a combination of such). Thus, provided herein is a method for the amelioration of pain in an individual comprising contacting immune cell(s) in the individual with an effective amount of adipose derived cells, *e.g*., adipose-derived cells comprising stem cells, adipose-derived cells comprising regenerative cells, adipose-derived cells comprising stem and regenerative cells, and the like, wherein said effective amount is an amount that (1) detectably modulates, e.g., suppresses, an immune and/or inflammatory response in said individual, and (2) detectably reduces pain in said individual. In a specific example, contacting immune cell(s) in the individual with an effective amount of adipose derived stem and regenerative cells results in a decrease in the amount of TNF-α, IL-2, IL-3, IL-6, IL-12, IL-17, IL-18, and/or interferon-γ, or any combination thereof, in the individual. In another specific example, contacting immune cell(s) in the individual with an effective amount of adipose derived cells, *e.g*., adipose-derived cells comprising stem cells, adipose-derived cells comprising regenerative cells, adipose-derived cells comprising stem and regenerative cells, and the like, results in a decrease in the amount of CCL2, CCL12, and/or CXCL1, or any combination thereof, in the individual. In another specific example, contacting immune cell(s) in the individual with an effective amount of adipose derived cells, *e.g*., adipose-derived cells comprising stem cells, adipose-derived cells comprising regenerative cells, adipose-derived cells comprising stem and regenerative cells, and the like, results in an increase in the amount of IL-10 in the individual.

In another example, provided herein is a method for the amelioration of pain in an individual comprising administering an effective amount of adipose derived cells, *e.g.,* adipose-derived cells comprising stem cells, adipose-derived cells comprising regenerative cells, adipose-derived cells comprising stem and regenerative cells, and the like, to the individual, wherein said effective amount is an amount that (1) detectably modulates, e.g., suppresses, an immune and/or inflammatory response in said individual, and (2) detectably reduces pain in said individual. In specific examples, said adipose derived cells, *e.g.,* adipose-derived cells comprising stem cells, adipose-derived cells comprising regenerative cells, adipose-derived cells comprising stem and regenerative cells, and the like, detectably suppress T cell proliferation in a mixed lymphocyte reaction (MLR) assay or a regression assay. The administering can be performed locally, systemically, or regionally (or by a combination of such). In a specific example, administering an effective amount of adipose derived cells, *e.g.,* adipose-derived cells comprising stem cells, adipose-derived cells comprising regenerative cells, adipose-derived cells comprising stem and regenerative cells, and the like, to the individual results in a decrease in the amount of TNF-α, IL-2, IL-3, IL-6, IL-12, IL-17, IL-18, and/or interferon-γ, or any combination thereof, in the individual. In another specific example, administering an effective amount of adipose derived cells, *e.g*., adipose-derived cells comprising stem cells, adipose-derived cells comprising regenerative cells, adipose-derived cells comprising stem and regenerative cells, and the like, to the individual results in a decrease in the amount of CCL2, CCL12, and/or CXCL1, or any combination thereof, in the individual. In another specific example, administering an effective amount of adipose derived cells, *e.g.,* adipose-derived cells comprising stem cells, adipose-derived cells comprising regenerative cells, adipose-derived cells comprising stem and regenerative cells, and the like, to the individual results in an increase in the amount of IL-10 in the individual.

An "immune cell" in the context of this method means any cell of the immune system (adaptive or innate), particularly T cells and NK (natural killer) cells, dendritic cells, and macrophages. Thus, in various examples of the method, adipose derived stem and regenerative cells are contacted with a plurality of immune cells, wherein the plurality of immune cells are, or comprises, a plurality of T cells (e.g., a plurality of CD3+ T cells, CD4+ T cells and/or CD8+ T cells) and/or natural killer cells. An "immune response" in the context of the method can be any response by an immune cell to a stimulus normally perceived by an immune cell, e.g., a response to the presence of an antigen. In various examples, an immune response can be the proliferation of T cells (e.g., CD3+ T cells, CD4+ T cells and/or CD8+ T cells) in response to a foreign antigen, such as an antigen present in a transfusion or graft, or to a self-antigen, as in an autoimmune disease. The immune response can also be a proliferation of T cells contained within a graft. The immune response can also be any activity of a natural killer (NK) cell, the maturation of a dendritic cell, the differentiation of T cells, skewing macrophages into the M1 or M2 lineage, or the like.

Adipose derived cells, *e.g*., adipose-derived cells comprising stem cells, adipose-derived cells comprising regenerative cells, adipose-derived cells comprising stem and regenerative cells, and the like, used for reduction or amelioration of pain, e.g., by reduction of inflammation, can also be derived from a single species, e.g., the species of the intended recipient or the species of the immune cells the function of which is to be reduced or suppressed, or can be derived from multiple species.

In various examples, said contacting or administering the adipose-derived cells is sufficient to suppress an immune function (e.g., T cell proliferation in response to an antigen) or inflammation in an individual afflicted with pain by at least 50%, 60%, 70%, 80%, 90% or 95%, compared to the immune function in the absence of the adipose derived cells, *e.g*., adipose-derived cells comprising stem cells, adipose-derived cells comprising regenerative cells, adipose-derived cells comprising stem and regenerative cells, and the like. Such suppression in an in vivo context can be determined in an in vitro assay (see below) using, e.g., a sample of T cells from the individual; that is, the degree of suppression in the in vitro assay can be extrapolated, for a particular number of adipose derived cells, *e.g.,* adipose-derived cells comprising stem cells, adipose-derived cells comprising regenerative cells, adipose-derived cells comprising stem and regenerative cells, and the like, and a number of immune cells in a recipient individual, to a degree of suppression in the individual.

Adipose derived stem and regenerative cells can be tested, e.g., in an MLR comprising combining CD4+ or CD8+ T cells, dendritic cells (DC) and adipose derived stem and regenerative cells in a ratio of about 10:1:2, wherein the T cells are stained with a dye such as, e.g., CFSE that partitions into daughter cells, and wherein the T cells are allowed to proliferate for about 6 days. The T cells and/or DC cells can be obtained from the individual to be treated, e.g., can be autologous to the individual, or can be allogeneic to the individual. The adipose derived cells, *e.g*., adipose-derived cells comprising stem cells, adipose-derived cells comprising regenerative cells, adipose-derived cells comprising stem and regenerative cells, and the like, are immunosuppressive if the T cell proliferation at 6 days in the presence of adipose derived stem and regenerative cells is detectably reduced compared to T cell proliferation in the presence of DC and absence of adipose derived cells, *e.g*., adipose-derived cells comprising stem cells, adipose-derived cells comprising regenerative cells, adipose-derived cells comprising stem and regenerative cells, and the like. In one example of an MLR, for example, adipose cells, *e.g*., adipose-derived cells comprising stem cells, adipose-derived cells comprising regenerative cells, adipose-derived cells comprising stem and regenerative cells, and the like, can be either thawed or harvested from culture. About 20,000 adipose derived stem and regenerative cells are resuspended in 100 µl of medium (RPMI 1640, 1 mM HEPES buffer, antibiotics, and 5% pooled human serum), and allowed to attach to the bottom of a well for 2 hours. CD4+ and/or CD8+ T cells are isolated from whole peripheral blood mononuclear cells Miltenyi magnetic beads. The cells are CFSE stained, and a total of 100,000 T cells (CD4+ T cells alone, CD8+ T cells alone, or equal amounts of CD4+ and CD8+ T cells) are added per well. The volume in the well is brought to 200 µl, and the MLR is allowed to proceed.

In certain examples, the anti-inflammatory activity (i.e., immunosuppressive activity) of the adipose derived cells, *e.g*., adipose-derived cells comprising stem cells, adipose-derived cells comprising regenerative cells, adipose-derived cells comprising stem and regenerative cells, and the like, is determined prior to administration to the individual suffering pain. This can be accomplished, for example, by determining the immunosuppressive activity of a sample of the adipose derived cells, *e.g*., adipose-derived cells comprising stem cells, adipose-derived cells comprising regenerative cells, adipose-derived cells comprising stem and regenerative cells, and the like, to be administered for the amelioration of pain. Such an activity can be determined, for example, by testing a sample of the adipose derived cells, *e.g*., adipose-derived cells comprising stem cells, adipose-derived cells comprising regenerative cells, adipose-derived cells comprising stem and regenerative cells, and the like, in, e.g., an MLR or regression assay. In one example, an MLR is performed with the sample, and a degree of immunosuppression demonstrated by the sample adipose derived cells, *e.g*., adipose-derived cells comprising stem cells, adipose-derived cells comprising regenerative cells, adipose-derived cells comprising stem and regenerative cells, and the like, in the assay is determined. The degree of pain amelioration is expected to correlate with the immunosuppressive activity of the sampled adipose derived cells, *e.g.,* adipose-derived cells comprising stem cells, adipose-derived cells comprising regenerative cells, adipose-derived cells comprising stem and regenerative cells, and the like. Thus, the MLR is one exemplary method that can be used in the examples disclosed herein, as a method of determining the absolute and relative ability of a particular population of adipose derived stem and regenerative cells or adipose derived cells, *e.g*., adipose-derived cells comprising stem cells, adipose-derived cells comprising regenerative cells, adipose-derived cells comprising stem and regenerative cells, and the like, to ameliorate pain attributable to inflammation.

The parameters of the MLR can be varied to provide more data or to best determine the capacity of a sample of adipose derived stem and regenerative cells or adipose derived stem and regenerative cells to immunosuppress, and therefore ameliorate pain. For example, because immunosuppression by adipose derived stem and regenerative cells appears to increase roughly in proportion to the number of adipose cells, *e.g*., adipose-derived cells comprising stem cells, adipose-derived cells comprising regenerative cells, adipose-derived cells comprising stem and regenerative cells, and the like, present in the assay, the MLR can be performed with, in one example, two or more numbers of adipose derived stem and regenerative cells, e.g., 1×10³, 3×10³, 1×10⁴ and/or 3×10⁴ adipose derived cells, *e.g.,* adipose-derived cells comprising stem cells, adipose-derived cells comprising regenerative cells, adipose-derived cells comprising stem and regenerative cells, and the like, per reaction. The number of adipose derived stem and regenerative cells relative to the number of T cells in the assay can also be varied. For example, adipose derived stem and regenerative cells and T cells in the assay can be present in any ratio of, e.g. about 10:1 to about 1:10, preferably about 1:5, though a relatively greater number of adipose derived cells, *e.g*., adipose-derived cells comprising stem cells, adipose-derived cells comprising regenerative cells, adipose-derived cells comprising stem and regenerative cells, and the like, or T cells can be used.

The regression assay or BTR assay can be used in similar fashion.

In some examples, immunosuppression and/or immunomodulation in a subject can be determined by testing for the level of various cytokines, immune cells, and the like, as described herein, before and after administration of the adipose-derived cells to the subject.

Adipose derived cells, *e.g*., adipose-derived cells comprising stem cells, adipose-derived cells comprising regenerative cells, adipose-derived cells comprising stem and regenerative cells, and the like, can be administered to an individual in a ratio, with respect to a known or expected number of immune cells, e.g., T cells, in the individual, of from about 10:1 to about 1:10, preferably about 1:5. However, adipose derived cells, *e.g*., adipose-derived cells comprising stem cells, adipose-derived cells comprising regenerative cells, adipose-derived cells comprising stem and regenerative cells, and the like, can be administered to an individual in a ratio of, in non-limiting examples, about 10,000:1, about 1,000:1, about 100:1, about 10:1, about 1:1, about 1:10, about 1:100, about 1:1,000 or about 1:10,000. Generally, about 1×10⁵ to about 1×10⁸ adipose derived cells, e.g., adipose-derived cells comprising stem cells, adipose-derived cells comprising regenerative cells, adipose-derived cells comprising stem and regenerative cells, and the like, per recipient kilogram, preferably about 1×10⁶ to about 1×10⁷ cells per recipient kilogram can be administered to effect immunosuppression. For example, in various examples, about 1×10⁵ to about 1×10⁸ adipose derived stem cells per recipient kilogram are administered for immunosuppression. In various examples, about 1×10⁵ to about 1×10⁸ adipose derived regenerative cells per recipient kilogram are administered for immunosuppression. For example, in various examples, about 1×10⁵ to about 1×10⁸ adipose derived progenitor cells per recipient kilogram are administered for immunosuppression. In various examples, adipose derived cells, e.g., adipose-derived cells comprising stem cells, adipose-derived cells comprising regenerative cells, adipose-derived cells comprising stem and regenerative cells, and the like, administered to an individual or subject comprise at least, about, or no more than, 1×10⁵, 3×10⁵, 1×10⁶, 3×10⁶, 1×10⁷ 3×10⁷, 1×10⁸, 3×10⁸, 1×10⁹, 3×10⁹ adipose-derived cells, e.g., adipose-derived cells comprising stem cells, adipose-derived cells comprising regenerative cells, adipose-derived cells comprising stem and regenerative cells, and the like, or more.

The adipose derived cells, *e.g*., adipose-derived cells comprising stem cells, adipose-derived cells comprising regenerative cells, adipose-derived cells comprising stem and regenerative cells, and the like, can also be administered with one or more second types of stem or stromal cells, e.g., mesenchymal stem cells or mesenchymal stromal cells from bone marrow, cord blood, muscle, dental pulp, amnion, and the like. Such second stem cells can be administered to an individual with said adipose derived cells, *e.g*., adipose-derived cells comprising stem cells, adipose-derived cells comprising regenerative cells, adipose-derived cells comprising stem and regenerative cells, and the like, in a ratio of, e.g., between about 1:10 to about 10:1.

To facilitate contacting, or proximity of, adipose derived cells, *e.g.,* adipose-derived cells comprising stem cells, adipose-derived cells comprising regenerative cells, adipose-derived cells comprising stem and regenerative cells, and the like, and immune cells *in vivo,* the adipose derived cells, *e.g.,* adipose-derived cells comprising stem cells, adipose-derived cells comprising regenerative cells, adipose-derived cells comprising stem and regenerative cells, and the like, can be administered to an individual by any route sufficient to bring the adipose derived cells, *e.g*., adipose-derived cells comprising stem cells, adipose-derived cells comprising regenerative cells, adipose-derived cells comprising stem and regenerative cells, and the like, and immune cells into contact with each other. For example, the adipose derived stem and regenerative cells can be administered to the individual, e.g., intravenously, intramuscularly, intraperitoneally, intraocularly, parenterally, intrathecally, subcutaneously, into the lymphatic system (e.g., into a lymph vessel or lymph node) or directly into an organ, e.g., fingers, pancreas, and the like. The adipose derived cells, *e.g.,* adipose-derived cells comprising stem cells, adipose-derived cells comprising regenerative cells, adipose-derived cells comprising stem and regenerative cells, and the like, can be administered to an area of an individual suffering pain at the site of pain, or at a site of nerve damage causing the pain. For in vivo administration, the adipose derived cells, *e.g*., adipose-derived cells comprising stem cells, adipose-derived cells comprising regenerative cells, adipose-derived cells comprising stem and regenerative cells, and the like, can be formulated as a pharmaceutical composition, as described below.

In another example, the adipose derived cells, *e.g*., adipose-derived cells comprising stem cells, adipose-derived cells comprising regenerative cells, adipose-derived cells comprising stem and regenerative cells, and the like, administered to the individual suffering from pain have been genetically engineered to express one or more anti-inflammatory cytokines In a specific example, said anti-inflammatory cytokines comprise IL-10, IL)-1 receptor antagonist, IL-4, IL-6, IL-10, IL-11, IL-13, or any combination thereof.

### Second Therapeutic Compositions and Second Therapies

In any of the above methods of treatment of pain in an individual, the method can comprise the administration of a second therapeutic composition or second therapy, e.g., an anti-pain medication or therapy. In a preferred example, the second active agents are capable of relieving pain, inhibiting or modulating inflammatory reactions, providing a sedative effect or an antineuralgic effect, or ensuring patient comfort.

In certain examples, the second therapeutic compositions comprise, but are not limited to, opioid analgesics, non-narcotic analgesics, anti-inflammatories, cox-2 inhibitors, alpha-adrenergic receptor agonists or antagonists, ketamine, anesthetic agents, NMDA antagonists, immunomodulatory agents, immunosuppressive agents, antidepressants, anticonvulsants, anti-hypertensives, anxiolytics, calcium channel blockers, muscle relaxants, corticosteroids, hyperbaric oxygen, JNK inhibitors, other therapeutics known to relieve pain, and pharmaceutically acceptable salts, solvates, hydrates, stereoisomers, clathrates, prodrugs and pharmacologically active metabolites thereof.

In certain examples, the second therapeutic composition is an opioid. Opioids can be used, e.g., to treat severe pain. Examples of opioid analgesics include, but are not limited to, oxycodone (e.g., OXYCONTIN®), morphine sulfate (e.g., MS CONTIN®, DURAMORPH®, and/or ASTRAMORPH®), meperidine (e.g., DEMEROL®), and fentanyl transdermal patch (e.g., DURAGESIC®) and other known conventional medications. Oxycodone (e.g., OXYCONTIN®) is a long-acting form of an opioid and may be used, e.g., in initial and later stages of CRPS.

Non-narcotic analgesics and anti-inflammatories may be used, e.g., for treatment of pain during pregnancy and breastfeeding. Non-steroidal anti-inflammatory drugs (NSAIDs) may be used, e.g., in the early stage of pain syndrome. Examples of anti-inflammatories include, but are not limited to, salicylic acid acetate (e.g., aspirin), ibuprofen (e.g., MOTRIN®, ADVIL®, or the like), ketoprofen (e.g., ORUVAIL®), rofecoxib (e.g., VIOXX®), naproxen sodium (e.g., ANAPROX®, NAPRELAN®, NAPROSYN®, or the like), ketorolac (e.g., ACULAR®), or other known conventional medications. A specific cox-2 inhibitor is celecoxib (e.g., CELEBREX).

Examples of second therapeutic compounds that are antidepressants include, but are not limited to, nortriptyline (PAMELOR®), amitriptyline (ELAVIL®), imipramine (TOFRANIL®), doxepin (SINEQUANO), clomipramine (ANAFRANIL®), fluoxetine (PROZAC®), sertraline (ZOLOFT®), nefazodone (SERZONE®), venlafaxine (EFFEXOR®), trazodone (DESYREL®), bupropion (WELLBUTRIN®) and other known conventional medications. See, e.g., Physicians' Desk Reference, 329, 1417, 1831 and 3270 (57th ed., 2003).

Examples of second therapeutic compounds that are anticonvulsant drugs include, but are not limited to, carbamazepine, oxcarbazepine, gabapentin (NEURONTIN®), phenyloin, sodium valproate, clonazepam, topiramate, lamotrigine, zonisamide, and tiagabine. See, e.g., Physicians' Desk Reference, 2563 (57th ed., 2003).

Other second therapeutic compounds useful in the examples described herein include, but are not limited to, corticosteroids (e.g., prednisone, dexamethasone or hydrocortisone), orally active class Ib anti-arrhythmic agents (e.g., mexiletine), calcium channel blockers (e.g., nifedipine), beta-blockers (e.g., propranolol), alpha-blocker (e.g., phenoxybenzamine), and alpha2-adrenergic agonists (e.g., clonidine) can also be used in combination with an immunomodulatory compound. See, e.g., Physicians' Desk Reference, 1979, 2006 and 2190 (57th ed., 2003).

Any combination of the above therapeutic agents can be administered. Such therapeutic agents can be administered in any combination with the adipose derived stem and regenerative cells, at the same time or as a separate course of treatment.

In some examples, the second therapeutic is administered concomitantly with the adipose-derived cells (e.g., in a single composition, or in different compositions administered at the same time. In some examples, the second therapeutic is administered prior to, or subjsequent to, the administration of the adipose-derived cells. Preferably, the second therapeutic is administered within 24 hours of the adipose-derived cells.

It should be noted that some of the second therapeutic compounds listed above (e.g., fluoxetine), though having a beneficial effect, may themselves as a side effect cause neuropathic pain in a small number of recipients. Generally, such compounds are considered safe to administer; however, one of ordinary skill in the art (e.g., a physician) will be able to determine the relative benefit of administering such a second therapeutic compound compared to the risk of further neuropathic pain.

The adipose derived cells described herein, *e.g*., adipose-derived cells comprising stem cells, adipose-derived cells comprising regenerative cells, adipose-derived cells comprising stem and regenerative cells, and the like, can be administered to the individual suffering from pain in the form of a pharmaceutical composition, e.g., a pharmaceutical composition suitable for intravenous, intramuscular or intraperitoneal injection. The adipose-derived cells, *e.g*., adipose-derived cells comprising stem cells, adipose-derived cells comprising regenerative cells, adipose-derived cells comprising stem and regenerative cells, and the like, can be administered to the individual in a single dose, or in multiple doses. Where cells, *e.g*., adipose-derived cells comprising stem cells, adipose-derived cells comprising regenerative cells, adipose-derived cells comprising stem and regenerative cells, and the like, are administered in multiple doses, the doses can be part of a therapeutic regimen designed to relieve the pain, or can be part of a long-term therapeutic regimen designed to treat the underlying cause of the pain. In examples in which adipose derived cells, e.g., adipose-derived cells comprising stem cells, adipose-derived cells comprising regenerative cells, adipose-derived cells comprising stem and regenerative cells, and the like, are administered with a second therapeutic agent, or with a second type of stem cell, the adipose derived cells and second therapeutic agent and/or second type of stem cell can be administered at the same time (*e.g.,* as a single composition, or in separate compositions) or different times, e.g., the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 days or more of each other.

### Scleroderma

Scleroderma is an autoimmune rheumatic disease associated with fibrosis and chronic pain. It is classified as either localized scleroderma or systemic scleroderma. The localized scleroderma is sub-classified as either morphea (waxy patches on the skin of varying sizes) or linear scleroderma (a streak or line of hardened, waxy skin on an arm or leg or on the forehead, which can lead to joint abnormalities); whereas systemic scleroderma is classified as either limited (CREST syndrome - Calcinosis, Raynaud Phenomenon, Esophageal dysfunction, Sclerodactyly and Telangiectasia) or diffuse (skin thickening occurs more rapidly and involves more skin areas than in limited disease and a higher risk of developing "sclerosis" or fibrous hardening of the internal organs). These are broad categories that include many different manifestations.

Most significant is that both limited and diffuse scleroderma can be accompanied with pulmonary hypertension, a condition in which the lung's blood vessels become narrow, leading to impaired blood flow through the lungs resulting in shortness of breath, as well as, pulmonary fibrosis. Inflammation in the body's blood vessels leads to their narrowing. Further damage from the inflammation and impact of the increased blood pressure can lead to destruction of smaller arteries. As a result, fibrosis takes place. This process can have devastating implications and the heart and lungs can suffer a significant loss in efficiency. Pulmonary arterial hypertension (PAH) is the leading cause of death in scleroderma patients.

Systemic sine scleroderma is a form of scleroderma, which lacks skin changes but has systemic manifestations. The systemic forms of scleroderma can cause cardiac (heart) problems. Systemic scleroderma can cause arrhythmias, pericardial effusion, scarring, left and right ventricle dysfunction, myocardial ischemia, cardiac remodeling, and heart failure.

Severe complications from scleroderma include:
Heart: Untreated high blood pressure strains the heart; irregular heart rhythm and enlargement of the heart lead to heart failure.
Kidney: Scleroderma renal crisis in which malignant hypertension develops and causes acute renal failure.
Lung: Two-thirds of all patients suffer from respiratory problems, such as shortness of breath, coughing, difficulty breathing, alveolitis (inflammation of lung air sacs), pneumonia, and cancer.
Digestive: Esophagus damage, acid reflux, gastric antral vascular ectasia (GAVE), which occasionally may bleed profusely.
Skin and joints: Carpal tunnel syndrome, joint pain, and stiffness.
Mouth: Flat white patches, loss of attached gingival mucosa, gingival recession, and diffuse widening of the periodontal ligament (PDL) space. Dysphagia may result from collagen deposition in the lingual and esophageal submucosa. Resorption of the posterior ramus of the mandible, the coronoid process, and the condyle. Inelasticity of the mouth.

Several other conditions are associated with development of fibrosis. Examples of such conditions are: local and/or systemic fibrosis secondary to treatment with chemotherapeutic agents (e.g., bleomycin or the like) or radiotherapy, fibrosis secondary to trauma (e.g., burn, accidental trauma, surgical incision or intervention, or the like). The fibrosis is associated with such conditions frequently leads to reduced function of the affected area: for example, reduced range of motion of a joint or skin surface, impaired healing, reduced compliance. In some cases this can be treated by invasively resecting the scar tissue and grafting in normal tissue (such as a split thickness skin graft) or by other surgical procedures that release the scar and associated adhesions from adjacent tissue. Secondary fibrosis and recurrence of the functional deficit and pain is common following these approaches. Hence, there is substantial need for an improved approach both in terms of an approach that is less invasive and in terms of efficacy.

In some examples, the adipose-derived cells disclosed herein improve fibrosis in the lungs, heart, kidney, and joints in subjects with diffuse scleroderma. In some embodiments, the adipose-derived cells disclosed herein improve or treat symptoms associated with limited scleroderma.

In some examples, the methods of treating or preventing scleroderma or systemic sclerosis disclosed herein comprise administering adipose-derived cells in multiple doses. In some examples, the methods of treating or preventing scleroderma or systemic sclerosis disclosed herein comprise administering multiple doeses of adipose-derived cells over a period of time, *e.g*., every 1, 2,3, 4, 5, 6, 7 or more days, every 1, 2, 3, 4, 5, 6, 7, 8, 9, 10, 11, or 12 months, every 1.5 years, every 2 years, or any amount of time in between.

In some examples, treatment with adipose-derived cells as disclosed herein results in a significant improvement in Cochin score, *e.g.,* more than 5%, more than 10%, more than 20%, more than 30%, more than 40%, or more that 50%, from baseline (prior to administration of the adipose-derived cells), to at least 1 month, at least 2 months, at least 3 months, at least 4 months, at least 5 months, at least 6 months, at least 7 months, at least 8 months, at least 9 months, at least 10 months, at least 11 months, at least a year, or longer, following administration of adipose-derived cells as described herein to the subject.

In some embodiments, treatment with adipose-derived cells as disclosed herein results in a significant improvement in VAS score of pain, *e.g.,* more than 5%, more than 10%, more than 20%, more than 30%, more than 40%, or more that 50%, from baseline (prior to administration of the adipose-derived cells), to at least 1 month, at least 2 months, at least 3 months, at least 4 months, at least 5 months, at least 6 months, at least 7 months, at least 8 months, at least 9 months, at least 10 months, at least 11 months, at least a year, or longer, following administration of adipose-derived cells as described herein to the subject.

In some embodiments, treatment with adipose-derived cells as disclosed herein results in a significant improvement in SHAQ score (scleroderma health assessment questionnaire), *e.g.,* more than 5%, more than 10%, more than 20%, more than 30%, more than 40%, or more that 50%, from baseline (prior to administration of the adipose-derived cells), to at least 1 month, at least 2 months, at least 3 months, at least 4 months, at least 5 months, at least 6 months, at least 7 months, at least 8 months, at least 9 months, at least 10 months, at least 11 months, at least a year, or longer, following administration of adipose-derived cells as described herein to the subject.

In some embodiments, treatment with adipose-derived cells as disclosed herein results in a significant improvement in mRodnan skin score, *e.g.,* more than 5%, more than 10%, more than 20%, more than 30%, more than 40%, or more that 50%, from baseline (prior to administration of the adipose-derived cells), to at least 1 month, at least 2 months, at least 3 months, at least 4 months, at least 5 months, at least 6 months, at least 7 months, at least 8 months, at least 9 months, at least 10 months, at least 11 months, at least a year, or longer, following administration of adipose-derived cells as described herein to the subject.

In some examples, treatment with adipose-derived cells as disclosed herein results in a significant a decrease in the number of mega-capillaries in the nail folds of subects, e.g., more than 5%, more than 10%, more than 20%, more than 30%, more than 40%, or more that 50%, from baseline (prior to administration of the adipose-derived cells), to at least 1 month, at least 2 months, at least 3 months, at least 4 months, at least 5 months, at least 6 months, at least 7 months, at least 8 months, at least 9 months, at least 10 months, at least 11 months, at least a year, or longer, following administration of adipose-derived cells as described herein to the subject.

### Raynaud's Phenomenon

Raynaud's phenomenon is characterized by excessively reduced blood flow in response to cold or emotional stress, causing discoloration of the fingers, toes, and occasionally other areas. This condition may also cause nails to become brittle with longitudinal ridges. Raynaud's syndrome arises as the result of vasospasms that decrease blood supply to the respective regions. Raynaud's phenomenon by itself is just a sign (hypoperfusion) accompanied by a symptom (discomfort). When linked to pathogenesis, it can be part of Raynaud's disease (also known as primary Raynaud's phenomenon), where the cause is unknown, or part of Raynaud's syndrome (secondary Raynaud's phenomenon), which is a syndrome caused by a known primary disease, most commonly connective tissue disorders such as systemic lupus erythematosus.

Current therapies for Raynaud's phenomenon require either (i) daily prophylactic medication delivered orally or intravenously, e.g. with a calcium channel blocker or beta blocker, which is undesirable due to long term and frequent side effects (e.g. postural hypertension), or (ii) are slow in onset and unreliable for treating symptoms as they occur (acutely). Accordingly, there is a need for treatments in Raynaud's phenomenon.

Various causes of secondary Raynaud's phenomenon are known, and include, for example connective tissue disorders such scleroderma, systemic lupus erythematosus, rheumatoid arthritis, Sjögren's syndrome, dermatomyositis, polymyositis, mixed connective tissue disease, cold agglutinin disease, Ehlers-Danlos syndrome, and the like. Eating disorders such as anorexia nervosa can also give rise to Raynaud's phenomenon. Obstructive disorders, such atherosclerosis, Buerger's disease, Takayasu's arteritis, subclavian aneurysms, thoracic outlet syndrome, and the like can also cause Raynaud's phenomenon. Drugs such as beta blockers, chemotherapeutics (*e.g*., bleomycin and the like), ciclosporine, bromocriptine, ergotamine, sulfasalazine, stimulant medications (*e.g*., medications used in the treatment of ADHD). anthrax vaccines, and the like, can also give rise to Raynaud's phenomenon. Physical trauma, such as that sustained in auto accidents or other traumatic events, Lyme disease, hypothyroidism, cryoglobulinemia, malignancy, chronic fatigue syndrome, reflex sympathetic dystrophy, carpal tunnel syndrome, magnesium deficiency, multiple sclerosis, and erythromelalgia (the opposite of Raynaud's, with hot and warm extremities) often co-exists in patients with Raynaud's are also known to give rise to Raynaud's phenomenon. The adipose-derived cells disclosed herein, (*e.g.,* the adipose-derived cells comprising stem cells, adipose-derived cells comprising stem cells and precursor cells, adipose-derived cells comprising stem cells and other regenerative cells, and the like), are useful in the treatment of Raynaud's phenomenon arising from any one or more of the foregoing. As such, the cells described herein, *e.g*., adipose-derived cells comprising stem cells, adipose-derived cells comprising regenerative cells, adipose-derived cells comprising stem and regenerative cells, and the like, may treat or prevent primary or secondary Raynaud's syndrome.

Various tests known in the art can be used to diagnose Raynaud's phenomenon. While the skilled person will readily appreciate that tests developed in the future for the diagnosis of Raynaud's disease are also useful in the embodiments described herein, non limiting examples of tests useful in the diagnosis of Raynaud's disease in the embodiments disclosed herein include:
(1) Digital artery pressure: pressures are measured in the arteries of the fingers before and after the hands have been cooled. A decrease of at least 15 mmHg is diagnostic (positive).
(2) Doppler ultrasound: to assess blood flow.
(3) Full blood count: this may reveal a normocytic anaemia suggesting the anaemia of chronic disease or renal failure.
(3) Blood test for urea and electrolytes: this may reveal renal impairment.
(4) Thyroid function tests: this may reveal hypothyroidism.
(5) An autoantibody screen, tests for rheumatoid factor, Erythrocyte sedimentation rate, and C-reactive protein, which may reveal specific causative illnesses or a generalized inflammatory process.
(6) Nail fold vasculature: this can be examined under the microscope.

The adipose-derived cell can treat or prevent one or more symptom's of Raynaud's phenomenon (whether primary or secondary), *e.g*., including but not limited to those described herein and using the diagnostic methods disclosed herein, and including but not limited to preventing or reducing the extent of the vasoconstriction that occurs when a subject with Raynaud's phenomenon is exposed to stress or cold; and reversing or lessening the cold induced or stress-induced vasoconstriction of the cutaneous arterial circulation.

### Methods of Treating Fibrosis

Fibrosis is a condition characterized by a deposition of extracellular matrix components in the internal organs, including the kidneys, heart, lungs, liver, skin and joints.

Lung fibrosis is one of the predominant fibrotic diseases. Idiopathic Pulmonary Fibrosis (IPF) is characterized by chronic inflammation of the alveolar walls with progressive fibrosis, of unknown etiology. IPF, or cryptogenic fibrosing alveolitis, causes 50 to 60% of cases of idiopathic interstitial lung disease (for reviews on IPF, see Khalil N and O'Connor 2004 and Selman et al. 2004).

Usual interstitial pneumonia (UIP), a specific histopathologic pattern of interstitial pneumonia, is the classic pattern found on lung biopsy in IPF. At low magnification, the tissue appears heterogeneous, with alternating areas of normal lung, interstitial inflammation, fibrosis, and honeycombing. Interstitial inflammation consists of an alveolar septal infiltrate of lymphocytes, plasma cells, and histiocytes associated with hyperplasia of type II pneumocytes. The fibrotic zones are composed mainly of dense acellular collagen, although scattered foci of proliferating fibroblasts (fibroblastic foci), which are the sites of early and active disease, may also be seen, usually in an intra-alveolar location. Areas of honeycombing are composed of cystic fibrotic airspaces, frequently lined with bronchiolar epithelium and filled with mucus. Neutrophils may pool in the mucus. Smooth muscle hyperplasia often occurs in areas of fibrosis and honeycombing. The subpleural and paraseptal distribution, patchy character, and temporal heterogeneity are the most helpful features in identifying UIP.

An identical pattern of interstitial inflammation and fibrosis occurs in collagen vascular disorders (e.g., RA, SLE, progressive systemic sclerosis, mixed connective tissue disease, diabetes mellitus), pneumoconioses (e.g., asbestosis), radiation injury, and certain drug-induced lung diseases (e.g., by nitrofurantoin).

The clinical course of IPF is progressive; median survival is 4 to 6 yr after diagnosis. Prednisone is the usual treatment in case of IPF. Response to treatment is variable, but patients with earlier disease, at a more cellular stage before scarring predominates, appear more likely to improve with corticosteroid or cytotoxic therapy. Supportive and palliative treatment includes O₂ in high concentrations to relieve hypoxemia and, if bacterial infection occurs, antibiotics. Lung transplantation has been successful in patients with end-stage lung disease. Fibrosis of the liver relates to an accumulation in the liver of connective tissue resulting from an imbalance between production and degradation of the extracellular matrix and accentuated by the collapse and condensation of preexisting fibers (for reviews see Afdhal NH and Nunes D. 2004. Kershenobich and Weissbrod. 2003. Pinzani and Rombouts 2004).

Liver fibrosis is a common response to hepatocellular necrosis or injury, which may be induced by a wide variety of agents, e.g., any process disturbing hepatic homeostasis (especially inflammation, toxic injury, diabetes, steatohepatitis, or altered hepatic blood flow) and infections of the liver (viral, bacterial, fungal, and parasitic). Numerous storage disorders resulting from inborn errors of metabolism are often associated with fibrosis, including lipid abnormalities (Gaucher's disease); glycogen storage diseases (especially types III,. IV, VI, IX, andX); .alpha..sub.1-antitrypsin deficiency; storage of exogenous substances, as seen in iron-overload syndromes (hemochromatosis) and copper storage diseases (Wilson's disease); accumulation of toxic metabolites (as in tyrosinemia, fructosemia, and galactosemia); and peroxisomal disorders (Zellweger syndrome). Numerous chemicals and drugs cause fibrosis, especially alcohol, methotrexate, isoniazid, oxyphenisatin, methyidopa, chlorpromazine, tolbutamide, and amiodarone. Disturbances of hepatic circulation (e.g., chronic heart failure, Budd-Chiari syndrome, veno-occlusive disease, portal vein thrombosis) and chronic obstruction to bile flow can lead to fibrosis. Lastly, congenital hepatic fibrosis is an autosomal recessive malformation.

The normal liver is made up of hepatocytes and sinusoids distributed within an extracellular matrix composed of collagen (predominantly types I, III, and IV) and noncollagen proteins, including glycoproteins (e.g., fibronectin, laminin) and several proteoglycans (e.g., heparan sulfate, chondroitin sulfate, dermatan sulfate, hyaluronate). Fibroblasts, normally found only in the portal tracts, can produce collagen, large glycoproteins, and proteoglycans.

Other liver cells (particularly stellate cells, hepatocytes and fat-storing Kupffer, and endothelial cells) also can produce extracellular matrix components. Fat-storing cells, located beneath the sinusoidal endothelium in the space of Disse, are precursors of fibroblasts, capable of proliferating and producing an excess of extracellular matrix. The development of fibrosis from active deposition of collagen is a consequence of liver cell injury, particularly necrosis, and inflammatory cells. The precise factors released from these cells is not known, but one or more cytokines or products of lipid peroxidation are likely. Kupffer cells and activated macrophages produce inflammatory cytokines. New fibroblasts form around necrotic liver cells; increased collagen synthesis leads to scarring. Quiescent stellate cells can become activated leading to upregulation of fibrosis. Fibrosis may derive from active fibrogenesis and from impaired degradation of normal or altered collagen. Fat-storing cells, Kupffer cells, and endothelial cells are important in the clearance of type I collagen, several proteoglycans, and denatured collagens. Changes in these cells' activities may modify the extent of fibrosis. For the histopathologist, fibrous tissue may become more apparent from passive collapse and condensation of preexisting fibers.

Thus, increased synthesis or reduced degradation of collagen results in active deposition of excessive connective tissue, which affects hepatic function: (1) Pericellular fibrosis impairs cellular nutrition and results in hepatocellular atrophy. (2) Within the space of Disse, fibrous tissue accumulates around the sinusoids and obstructs the free. passage of substances from the blood to the hepatocytes. (3) Fibrosis around hepatic venules and the portal tracts disturbs hepatic blood flow. Venous resistance across the liver Increases from portal vein branches to sinusoids and finally to hepatic veins. All three routes can be involved.

The fibrous bands that link portal tracts with central veins also promote anastomotic channels: Arterial blood, bypassing the normal hepatocytes, is shunted to efferent hepatic veins, which further impairs hepatic function and can accentuate hepatocellular necrosis. The extent to which these processes are present determines the magnitude of hepatic dysfunction: e.g., in congenital hepatic fibrosis, large fibrous bands involve predominantly the portal regions but usually spare the hepatic parenchyma. Congenital hepatic fibrosis thus presents as portal hypertension with preserved hepatocellular function.

Scleroderma is a disease of the connective tissue characterized by fibrosis of the skin and internal organs, leading to organ failure and death (Black et al., 1998; Clements and Furst, 1996; for reviews see Varga J. 2004. Chung and Utz, 2004. Rhew and Barr, 2004). Scleroderma has a spectrum of manifestations and a variety of therapeutic Implications. It comprises localized scleroderma, systemic sclerosis, scleroderma-like disorders, and Sine scleroderma (Smith, 2000). Whilst localized scleroderma is a rare dermatologic disease associated with fibrosis and manifestations limited to skin, systemic sclerosis is a multisystem disease with variable risk for internal organ involvement and variation in the extent of skin disease. Systemic sclerosis can be diffuse or limited. Limited systemic sclerosis is also called CREST (calcinosis, Raynaud's esophageal dysfunction, sclerodactyly, telangiectasiae). Scleroderma-like disorders are believed to be related to industrial environment exposure. In Sine disease, there is internal organ involvement without skin changes.

The major manifestations of scleroderma and in particular of systemic sclerosis are inappropriate excessive collagen synthesis and deposition, endothelial dysfunction, spasm, collapse and obliteration by fibrosis.

Scleroderma is a rare disease with a stable incidence of approximately 19 cases per 1 million persons. The cause of scleroderma is unknown. However, the genetic predisposition is important. Abnormalities involve autoimmunity and alteration of endothelial cell and fibroblast function. Indeed, systemic sclerosis is probably the most severe of the autoimmune diseases with a reported 50% mortality within 5 years of diagnosis (Silman, 1991).

In terms of diagnosis, an important clinical parameter is skin thickening proximal to the metacarpophalangeal joints. Raynaud's phenomenon is a frequent, almost universal component of scleroderma. It is diagnosed by color changes of the skin upon cold exposure. Ischemia and skin thickening are symptoms of Raynaud's disease.

Several underlying biological processes are implicated in the initiation, severity and progression of the disease and include vascular dysfunction, endothelial cell activation and damage, leukocyte accumulation, auto-antibody production and crucially, an uncontrolled fibrotic response which may lead to death (Clements and Furst, 1996). Fibroblasts have a pivotal role in the pathogenesis of this disease. Primary fibroblasts obtained from patients with scleroderma exhibit many of the characteristic properties of the disease seen in vivo, notably increased extracellular matrix synthesis and deposition, notably of collagen and fibronectin, and altered growth factor and cytokine production such as of TGF-β. and CTGF (Strehlow and Korn, 1998 and LeRoy, 1974).

The examples disclosed herein relate to a treatment of fibrosis, including but not limited to fibrosis associated with scleroderma, using adipose derived cells, *e.g.,* adipose-derived cells comprising stem cells, adipose-derived cells comprising regenerative cells, adipose-derived cells comprising stem and regenerative cells, and the like. There are different types of scleroderma. The multi-systemic fibrotic form, also called systemic sclerosis, comprises two sub-types depending on the degree of skin involvement: The limited cutaneous form affects the extremities whereas the diffuse subtype also involves the trunk. Besides the systemic disease, there are localized forms of the disease including e.g. morphea, that involve certain areas of the skin, sometimes joints and muscles, but not the internal organs. The major hallmarks of systemic sclerosis are widespread microvascular damage and progressive fibrosis of the skin and internal organs. The most evident clinical symptom is usually the hardening of the skin and associated scarring. The skin may appear tight, reddish or scaly. Despite the progress in managing complications which occur mostly due to organ failure, to date, there is still neither cure nor disease-specific treatment. Furthermore, the liver, kidney, lung, heart and pericardium, eye, skin, mouth, pancreas, gastrointestinal tract, brain, breast, bone marrow, bone, genitourinary, a tumor, or a wound, may be affected.

The cells described herein, *e.g*., adipose-derived cells comprising stem cells, adipose-derived cells comprising regenerative cells, adipose-derived cells comprising stem and regenerative cells, and the like, may treat or prevent fibrosis resulting from conditions including but not limited to rheumatoid arthritis, lupus, pathogenic fibrosis, fibrosing disease, fibrotic lesions such as those formed after Schistosoma japonicum infection, radiation damage, autoimmune diseases, Lyme disease, chemotherapy induced fibrosis, radiation-induced fibrosis, HIV or infection-induced focal sclerosis, failed back syndrome due to spinal surgery scarring, abdominal adhesion post surgery scarring, fibrocystic formations, fibrosis after spinal injury, surgery-induced fibrosis, mucosal fibrosis, peritoneal fibrosis caused by dialysis, and Adalimumab-associated pulmonary fibrosis.

Specifically, in the liver, the cells described herein, *e.g*., adipose-derived cells comprising stem cells, adipose-derived cells comprising regenerative cells, adipose-derived cells comprising stem and regenerative cells, and the like, may treat or prevent fibrosis resulting from conditions including but not limited to alcohol, drug, and/or chemically induced cirrhosis, ischemia-reperfusion injury after hepatic transplant, necrotizing hepatitis, hepatitis B, hepatitis C, primary biliary cirrhosis, and primary sclerosing cholangitis.

Relating to the kidney, the cells described herein, *e*.*g*., *e*.*g*., adipose-derived cells comprising stem cells, adipose-derived cells comprising regenerative cells, adipose-derived cells comprising stem and regenerative cells, and the like, may treat or prevent fibrosis resulting from conditions including but not limited to proliferative and sclerosing glomerulonephritis, nephrogenic fibrosing dermopathy, diabetic nephropathy, renal tubulointerstitial fibrosis, and focal segmental glomerulosclerosis.

Relating to the lung, the cells described herein, *e.g.,* ADCs, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to pulmonary interstitial fibrosis, sarcoidosis, pulmonary fibrosis, idiopathic pulmonary fibrosis, asthma, chronic obstructive pulmonary disease, diffuse alveolar damage disease, pulmonary hypertension, neonatal bronchopulmonary dysplasia, chronic asthma, and emphysema. There are several sub-names or synonyms for pulmonary fibrosis including, but not limited to, cryptogenic fibrosing alveolitis, diffuse interstitial fibrosis, idiopathic interstitial pneumonitis, Hamman-Rich syndrome, silicosis, asbestosis, berylliosis, coal worker's pneumoconiosis, black lung disease, coal miner's disease, miner's asthma, anthracosis, and anthracosilicosis.

Relating to the heart and/or pericardium, the adipose-derived cells described herein, e.g., adipose-derived cells comprising stem cells, adipose-derived cells comprising regenerative cells, adipose-derived cells comprising stem and regenerative cells, and the like,, may treat or prevent fibrosis resulting from conditions including but not limited to myocardial fibrosis, atherosclerosis, coronary artery restenosis, congestive cardiomyopathy, heart failure, and other post-ischemic conditions.

Relating to the eye, the adipose-derived cells described herein, cells, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to exophthalmos of Grave's disease, proliferative vitreoretinopathy, anterior capsule cataract, corneal fibrosis, corneal scarring due to surgery, trabeculectomy-induced fibrosis, progressive subretinal fibrosis, multifocal granulomatous chorioretinitis, and other eye fibrosis.

Relating to the skin, the adipose-derived cells described herein, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to Depuytren's contracture, scleroderma, keloid scarring, psoriasis, hypertrophic scarring due to burns, atherosclerosis, restenosis, cutaneous fibrosis arising from chemotherapy exposure, and psuedoscleroderma caused by spinal cord injury.

Relating to the mouth and/or esophagus, the cells described herein, cells, e.g., adipose-derived cells comprising stem cells, adipose-derived cells comprising regenerative cells, adipose-derived cells comprising stem and regenerative cells, and the like, may treat or prevent fibrosis resulting from conditions including but not limited to periodontal disease scarring, gingival hypertrophy secondary to drugs, and congenital esophageal stenosis.

Relating to the pancreas, the cells described herein, cells, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to pancreatic fibrosis, stromal remodeling pancreatitis, and stromal fibrosis.

Relating to the gastrointestinal tract, the cells described herein, cells, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to collagenous colitis, villous atrophy, crypt hyperplasia, polyp formation, fibrosis of Crohn's disease, and healing gastric ulcer.

Relating to the brain, the cells described herein, cells, *e.g*., adipose-derived cells comprising stem cells, adipose-derived cells comprising regenerative cells, adipose-derived cells comprising stem and regenerative cells, and the like, may treat or prevent fibrosis resulting from conditions including but not limited to glial scar tissue.

Relating to the breast, the cells described herein, cells, *e.g*., adipose-derived cells comprising stem cells, adipose-derived cells comprising regenerative cells, adipose-derived cells comprising stem and regenerative cells, and the like, may treat or prevent fibrosis resulting from conditions including but not limited to fibrocystic disease and desmoplastic reaction to breast cancer, cancer and fibrosis secondary to treatment for breast malignancy.

Relating to the bone marrow, the cells described herein, cells, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to fibrosis in myelodysplasia and neoplastic diseases.

Relating to the bone, the cells described herein, cells, *e.g*., adipose-derived cells comprising stem cells, adipose-derived cells comprising regenerative cells, adipose-derived cells comprising stem and regenerative cells, and the like, may treat or prevent fibrosis resulting from conditions including but not limited to rheumatoid pannus formation.

Relating to the genitourinary system, the cells described herein, cells, *e.g.,* adipose-derived cells comprising stem cells, adipose-derived cells comprising regenerative cells, adipose-derived cells comprising stem and regenerative cells, and the like, may treat or prevent fibrosis resulting from conditions including but not limited to endometriosis, uterine fibroids, ovarian fibroids, and Peyronie's disease.

Relating to radiation induced damage, the cells described herein, cells, *e.g.,* adipose-derived cells comprising stem cells, adipose-derived cells comprising regenerative cells, adipose-derived cells comprising stem and regenerative cells, and the like, may treat or prevent fibrosis related to, but not limited to, treatment of head and neck cancer, ovarian cancer, prostate cancer, lung cancer, gastrointestinal cancer, colon cancer, and breast cancer.

### Methods of making an adipose-derived cell population comprising stem and regenerative cells

In some embodiments, adipose tissue is processed to obtain a refined, enriched, concentrated, isolated, or purified population of adipose-derived cells, *e.g.,* a population of adipose-derived cells comprising stem cells, a population of adipose-derived cells comprising regenerative cells, a population of adipose-derived cells comprising stem and regenerative cells, and the like useful in the embodiments disclosed herein, using a cell processing unit, gradient sedimentation, filtration, or a combination of any one or more of these approaches. In general, adipose tissue is first removed from a subject (e.g., a mammal, a domestic animal, a rodent, a horse, a dog, cat, or human) then it is processed to obtain a cell population, *e.g*., a population of adipose-derived cells comprising stem cells, a population of adipose-derived cells comprising regenerative cells, a population of adipose-derived cells comprising stem and regenerative cells, and the like. For allogeneic transplantation, an appropriate donor can be selected using methods known in the art, for example, methods used for selection of bone marrow donors. The volume of adipose tissue collected from the patient can vary from about 1 cc to about 2000 cc and in some embodiments up to about 3000 cc. The volume of tissue removed will vary from patient to patient and will depend on a number of factors including but not limited to: age, body habitus, coagulation profile, hemodynamic stability, severity of insufficiency or injury, co-morbidities, and physician preference.

The adipose tissue can be obtained by any method known to a person of ordinary skill in the art. For example, the adipose tissue may be removed from a subject by suction-assisted lipoplasty, ultrasound-assisted lipoplasty, or excisional lipectomy. In addition, the procedures may include a combination of such procedures, such as a combination of excisional lipectomy and suction-assisted lipoplasty. If the tissue or some fraction thereof is intended for re-implantation into a subject, the adipose tissue should be collected in a manner that preserves the viability of the cellular component and that minimizes the likelihood of contamination of the tissue with potentially infectious organisms, such as bacteria and/or viruses. Thus, the tissue extraction should be performed in a sterile or aseptic manner to minimize contamination. Suction-assisted lipoplasty may be desired to remove the adipose tissue from a patient as it provides a minimally invasive method of collecting tissue with minimal potential for stem cell damage that may be associated with other techniques, such as ultrasound-assisted lipoplasty.

Accordingly, adipose tissue provides a rich source of a population of cells that is easily enriched for adipose-derived stem cells, adipose-derived regenerative cells, adipose-derived stem and regenerative cells, or the like. Collection of adipose tissue is also more patient-friendly and is associated with lower morbidity than collection of a similar volume of, for example, skin or a much larger volume of tonsil.

For suction-assisted lipoplastic procedures, adipose tissue is collected by insertion of a cannula into or near an adipose tissue depot present in the patient followed by aspiration of the adipose into a suction device. In some embodiments, a small cannula may be coupled to a syringe, and the adipose tissue may be aspirated using manual force. Using a syringe or other similar device may be desirable to harvest relatively moderate amounts of adipose tissue (e.g., from 0.1 ml to several hundred milliliters of adipose tissue). Procedures employing these relatively small devices require only local anesthesia. Larger volumes of adipose tissue (e.g., greater than several hundred milliliters) may require general anesthesia at the discretion of the donor and the person performing the collection procedure. When larger volumes of adipose tissue are to be removed, relatively larger cannulas and automated suction devices may be employed.

Excisional lipectomy procedures include, and are not limited to, procedures in which adipose tissue-containing tissues (e.g., skin) is removed as an incidental part of the procedure; that is, where the primary purpose of the surgery is the removal of tissue (e.g., skin in bariatric or cosmetic surgery) and in which adipose tissue is removed along with the tissue of primary interest. Subcutaneous adipose tissue may also be extracted by excisional lipectomy in which the adipose tissue is excised from the subcutaneous space without concomitant removal of skin.

The amount of tissue collected can depend on a number of variables including, but not limited to, the body mass index of the donor, the availability of accessible adipose tissue harvest sites, concomitant and pre-existing medications and conditions (such as anticoagulant therapy), and the clinical purpose for which the tissue is being collected. Experience with transplant of hematopoietic stem cells (bone marrow or umbilical cord blood-derived stem cells used to regenerate the recipient's blood cell-forming capacity) shows that engraftment is cell dose-dependent with threshold effects (Smith, et al., 1995; Barker, et al., 2001). Thus, it is possible that the general principle that "more is better" will be applied within the limits set by other variables and that where feasible the harvest will collect as much tissue as possible.

The adipose tissue that is removed from a patient is then collected into a device (e.g., cell processing unit, centrifuge, or filtration unit) for further processing so as to remove collagen, adipocytes, blood, and saline, thereby obtaining a cell population comprising adipose derived cells, *e.g*., adipose-derived cells comprising stem cells, adipose-derived cells comprising regenerative cells, adipose-derived cells comprising stem and regenerative cells, and the like. Preferably the population of adipose derived cells containing ADRCs is free from contaminating collagen, adipocytes, blood, and saline. The major contaminating cells in adipose tissue (adipocytes) have low density and are easily removed by flotation.

Adipose tissue processing to obtain a refined, concentrated, and isolated population of adipose-derived cells, *e.g.,* a population of adipose-derived cells comprising stem cells, a population of adipose-derived cells comprising regenerative cells, a population of adipose-derived cells comprising stem cells and regenerative cells and the like, and modifications thereto are preferably performed using methods described, for example, in U.S. App. Ser. No. 10/316,127 (U.S. Pat. App. Pub. No. 2003/0161816), entitled SYSTEMS AND METHODS FOR TREATING PATIENTS WITH PROCESSED LIPOASPIRATE CELLS, filed December 9, 2002, and U.S. App. Ser. No. 10/877,822 (U.S. Pat. App. Pub. No. 2005/0084961), entitled SYSTEMS AND METHODS FOR SEPARATING AND CONCENTRATING REGENERATIVE CELLS FROM TISSUE, filed June 25, 2004; U.S. App. Ser. No.. 10/242,094, entitled PRESERVATION OF NON EMBRYONIC CELLS FROM NON HEMATOPOIETIC TISSUES, filed September 12, 2002, which claims the benefit of U.S. App. Ser. No. 60/322,070 filed September 14, 2001; U.S. Application Ser. No. 10/884,638, entitled SYSTEMS AND METHODS FOR ISOLATING AND USING CLINICALLY SAFE ADIPOSE DERIVED REGENERATIVE CELLS, filed on July 2,2004. The applications above disclose the processing of adipose-derived cells in a system that is configured to maintain a closed, sterile fluid/tissue pathway. This can be achieved by use of a pre-assembled, linked set of closed, sterile containers and tubing allowing for transfer of tissue and fluid elements within a closed pathway. This processing set can be linked to a series of processing reagents (e.g., saline, enzymes, etc.) inserted into a device, which can control the addition of reagents, temperature, and timing of processing thus relieving operators of the need to manually manage the process. In a preferred embodiment, the entire procedure from tissue extraction through processing and placement into the recipient is performed in the same facility, indeed, even within the same room, of the patient undergoing the procedure.

For many applications, preparation of the active cell population requires depletion of the mature fat-laden adipocyte component of adipose tissue. This can be achieved by a series of washing and disaggregation steps in which the tissue is first rinsed to reduce the presence of free lipids (released from ruptured adipocytes) and peripheral blood elements (released from blood vessels severed during tissue harvest), and then disaggregated to free intact adipocytes and other cell populations from the connective tissue matrix. In some embodiments, the adipose-derived cells, *e.g*., adipose-derived cells comprising stem cells, adipose-derived cells comprising regenerative cells, adipose-derived cells comprising stem and regenerative cells, and the like, are provided with blood vessel endothelial cells (BECs), BEC progenitors (EPCs), and adipose tissue-derived stem cells, adipose tissue-derived stromal cells, and other cellular elements. In some embodiments the adipose-derived cells, *e.g.,* cells, *e.g.,* adipose-derived cells comprising stem cells, adipose-derived cells comprising regenerative cells, adipose-derived cells comprising stem and regenerative cells, and the like, comprise cells that are in the form of aggregates or partially disaggregated fragments, for example, two or more vascular cells linked by extracellular matrix. In some embodiments such aggregates comprise large aggregates or fragments comprising more than 10 cells or more than 100 cells linked by extracellular matrix. Such aggregates may include, but are not limited to blood or lymph vessel fragments in which several cells remain linked in an approximation of their original orientation to one another (including, by way of non-limiting example, vascular endothelial cells and pericytes or smooth muscle cells linked by some or all of the extracellular matrix that bound them together in the tissue prior to processing). In a particular embodiment such aggregates may comprise several hundred cells in contact or associated with fewer adipocytes than they were in the tissue prior to processing.

Rinsing is an optional but preferred step, wherein the tissue is mixed with a solution to wash away free lipid and single cell components, such as those components in blood, leaving behind intact adipose tissue fragments. In one embodiment, the adipose tissue that is removed from the patient is mixed with isotonic saline or other physiologic solution(s), e.g., Plasmalyte® of Baxter Inc. or Normosol® of Abbott Labs. Intact adipose tissue fragments can be separated from the free lipid and cells by any means known to persons of ordinary skill in the art including, but not limited to, filtration, decantation, sedimentation, or centrifugation. In some embodiments, the adipose tissue is separated from non-adipose tissue by employing a filter disposed within a tissue collection container, as discussed herein. In other embodiments, the adipose tissue is separated from non-adipose tissue using a tissue collection container that utilizes decantation, sedimentation, and/or centrifugation techniques to separate the materials.

The intact tissue fragments are then disaggregated using any conventional techniques or methods, including mechanical force (mincing or shear forces), ultrasonic or other physical energy, lasers, microwaves, enzymatic digestion with single or combinatorial proteolytic enzymes, such as collagenase, trypsin, lipase, liberase HI, nucleases, or members of the Blendzyme family as disclosed in U.S. Pat. No. 5,952,215, "Enzyme composition for tissue dissociation," and pepsin, or a combination of mechanical and enzymatic methods. For example, the cellular component of the intact tissue fragments may be disaggregated by methods using collagenase-mediated dissociation of adipose tissue, similar to the methods for collecting microvascular endothelial cells in adipose tissue, as disclosed in U. S. Pat. No. 5,372,945. Additional methods using collagenase that may be used are disclosed in, e.g., U.S. Patent Nos. 5,830,741, "Composition for tissue dissociation containing collagenase I and II from clostridium histolyticum and a neutral protease" and by Williams, et al., 1995, "Collagenase lot selection and purification for adipose tissue digestion," Cell Transplant 4(3):281-9. Similarly, a neutral protease may be used instead of collagenase, as disclosed in Twentyman, et al. (Twentyman, et al., 1980, "Use of bacterial neutral protease for disaggregation of mouse tumours and multicellular tumor spheroids," Cancer Lett. 9(3):225-8). Furthermore, the methods described herein may employ a combination of enzymes, such as a combination of collagenase and trypsin or a combination of an enzyme, such as trypsin, and mechanical dissociation.

Adipose tissue-derived cells, adipose-derived cells comprising stem cells, adipose-derived cells comprising regenerative cells, adipose-derived cells comprising stem and regenerative cells, and the like, may then be obtained from the disaggregated tissue fragments by reducing the number of mature adipocytes. A suspension of the disaggregated adipose tissue and the liquid in which the adipose tissue was disaggregated is then passed to another container, such as a cell collection container. The suspension may flow through one or more conduits to the cell collection container by using a pump, such as a peristaltic pump, that withdraws the suspension from the tissue collection container and urges it to the cell collection container. Other embodiments may employ the use of gravity or a vacuum while maintaining a closed system. Separation of the cells in the suspension may be achieved by buoyant density sedimentation, centrifugation, elutriation, filtration, differential adherence to and elution from solid phase moieties, antibody-mediated selection, differences in electrical charge, immunomagnetic beads, fluorescence activated cell sorting (FACS), or other means. Examples of these various techniques and devices for performing the techniques may be found in U. S. Pat. Nos. 6,277,060; 6,221,315; 6,043,066; 6,451,207; 5,641,622; and 6,251,295. Many of these devices can be incorporated within the cell processing unit, while maintaining a closed system.

In some embodiments, the cells in the suspension are separated from the acellular component of the suspension using a spinning membrane filter. In other embodiments, the cells in the suspension are separated from the acellular component using a centrifuge. In one such exemplary embodiment, the cell collection container may be a flexible bag that is structured to be placed in a centrifuge (e.g., manually or by robotics). In other embodiments, a flexible bag is not used. After centrifugation, the cellular component containing ADRCs forms a pellet, which may then be resuspended with a buffered solution so that the cells can be passed through one or more conduits to a mixing container, as discussed herein. The resuspension fluids may be provided by any suitable means. For example, a buffer may be injected into a port on the cell collection container, or the cell collection container may include a reserve of buffer that can be mixed with the pellet of cells by rupturing the reserve. When a spinning membrane filter is used, resuspension is optional since the cells remain in a volume of liquid after the separation procedure.

In one embodiment a subpopulation of the adipose-derived cells, *e.g.,* adipose-derived cells comprising stem cells, adipose-derived cells comprising regenerative cells, adipose-derived cells comprising stem and regenerative cells, and the like, is selected from other cells by short term adherence to a surface, for example, plastic. In one embodiment the duration of adherence for the purpose of selection is approximately one hour. In a second embodiment the duration of adherence to the surface is 24 hours.

Although some embodiments described herein are directed to methods of fully disaggregating the adipose tissue to separate the active cells from the mature adipocytes and connective tissue, additional embodiments are directed to methods in which the adipose tissue is only partially disaggregated. For example, partial disaggregation may be performed with one or more enzymes, which are removed from at least a part of the adipose tissue early relative to an amount of time that the enzyme would otherwise be left thereon to fully disaggregate the tissue. Such a process may require less processing time and would generate fragments of tissue components within which multiple adipose-derived cells, *e.g.,* adipose-derived cells comprising stem cells, adipose-derived cells comprising regenerative cells, adipose-derived cells comprising stem and regenerative cells, and the like, remain in partial or full contact. In another embodiment mechanical force (for example ultrasound energy or shear force) is applied to prepare the cells, *e.g*., adipose-derived cells comprising stem cells, adipose-derived cells comprising regenerative cells, adipose-derived cells comprising stem and regenerative cells, and the like, and/or fragments comprising adipose-derived cells isolated from all or some of the mature adipocytes with which they were associated in the tissue prior to processing.

In some embodiments, the tissue is washed with sterile buffered isotonic saline and incubated with collagenase at a collagenase concentration, a temperature, and for a period of time sufficient to provide adequate disaggregation. In a preferred embodiment, the collagenase enzyme used will be approved for human use by the relevant authority (e.g., the U. S. Food and Drug Administration). Suitable collagenase preparations include recombinant and non-recombinant collagenase. Non-recombinant collagenase may be obtained from F. Hoffmann-La Roche Ltd., Indianapolis, IN and/or Advance Biofactures Corp., Lynbrook, NY. Recombinant collagenase may also be obtained as disclosed in U.S. Pat. No. 6,475,764.

In one embodiment, solutions contain collagenase at concentrations of about 10 µg/ml to about 50 µg/ml (e.g., 10µg/ml, 20µg/ml, 30µg/ml, 40µg/ml, or 50µg/ml) and are incubated at from about 30°C to about 38°C for from about 20 minutes to about 60 minutes. These parameters will vary according to the source of the collagenase enzyme, optimized by empirical studies, in order to confirm that the system is effective at extracting the desired cell populations in an appropriate time frame. A particular preferred concentration, time and temperature is 20 µg/ml collagenase (mixed with the neutral protease dispase; Blendzyme 1, Roche) and incubated for 45 minutes at about 37° C. An alternative preferred embodiment applies 0.5 units/mL collagenase (mixed with the neutral protease thermolysin; Blendzyme 3). In a particularly preferred embodiment the collagenase enzyme used is material approved for human use by the relevant authority (e.g., the U.S. Food and Drug Administration). The collagenase used should be free of micro-organisms and contaminants, such as endotoxin.

Following disaggregation the active cell population can be washed/rinsed to remove additives and/or by-products of the disaggregation process (e.g., collagenase and newly-released free lipid). The active cell population can then be concentrated by centrifugation or other methods known to persons of ordinary skill in the art, as discussed above. These post-processing wash/concentration steps may be applied separately or simultaneously. In one embodiment, the adipose-derived cells, *e.g*., adipose-derived cells comprising stem cells, adipose-derived cells comprising regenerative cells, adipose-derived cells comprising stem and regenerative cells, and the like, are concentrated and the collagenase removed by passing the cell population through a continuous flow spinning membrane system or the like, such as, for example, the system disclosed in U.S. Pat. Nos. 5,034,135 and 5,234,608.

In addition to the foregoing, there are many known post-wash methods that may be applied for further purifying the adipose-derived cell population that comprises stem cells, regenerative cells, stem cells and regenerative cells, and the like. These include both positive selection (selecting the target cells), negative selection (selective removal of unwanted cells), or combinations thereof. In addition to separation by flow cytometry as described herein and in the literature, cells can be separated based on a number of different parameters, including, but not limited to, charge or size (e.g., by dielectrophoresis or various centrifugation methods, etc.).

Many other conformations of the staged mechanisms used for cell processing will be apparent to one skilled in the art. For example, mixing of tissue and saline during washing and disaggregation can occur by agitation or by fluid recirculation. Cell washing may be mediated by a continuous flow mechanism such as the spinning membrane approach, differential adherence, differential centrifugation (including, but not limited to differential sedimentation, velocity, or gradient separation), or by a combination of means. Similarly, additional components allow further manipulation of cells, including addition of growth factors or other biological response modifiers, and mixing of cells with natural or synthetic components intended for implant with the cells into the recipient.

Post-processing manipulation may also include cell culture or further cell purification (Kriehuber, et al., 2001; Garrafa, et al., 2006). In some embodiments, once the adipose-derived cell population, cells, *e.g*., adipose-derived cells comprising stem cells, adipose-derived cells comprising regenerative cells, adipose-derived cells comprising stem and regenerative cells, and the like, is obtained, it is further refined, concentrated, enriched, isolated, or purified using a cell sorting device and/or gradient sedimentation. Mechanisms for performing these functions may be integrated within the described devices or may be incorporated in separate devices. In many embodiments, however, a therapeutically effective amount of a concentrated population of adipose derived cells, *e.g*., adipose-derived cells comprising stem cells, adipose-derived cells comprising regenerative cells, adipose-derived cells comprising stem and regenerative cells, and the like, is used to prepare a medicament for the reduction of pain and/or fibrosis (e.g., in sclerodema, Raynaud's syndrome, or other fibrosis-linked diseases and disorders), wherein said concentrated population of cells is to be administered to a patient in need thereof without culturing the cells before administering them to the patient. That is, some examples concern methods to reduce pain, fibrosis, or both, wherein a therapeutically effective amount of a concentrated population of adipose derived cells, *e.g*., adipose-derived cells comprising stem cells, adipose-derived cells comprising regenerative cells, adipose-derived cells comprising stem and regenerative cells, and the like, is administered to a patient in need thereof without culturing the cells before administering them to the patient.

In a preferred embodiment, the tissue removal system and processing set would be present in the vicinity of the patient receiving the treatment, such as the operating room or out-patient procedure room (effectively at the patient's bedside). This allows rapid, efficient tissue harvest and processing, and decreases the opportunity for specimen handling/labeling error, thereby allowing for performance of the entire process in the course of a single surgical procedure.

As described in U.S. Application No. 10/884,638, entitled SYSTEMS AND METHODS FOR ISOLATING AND USING CLINICALLY SAFE ADIPOSE DERIVED REGENERATIVE CELLS, filed on July 2, 2004, one or more additives may be added to the cells during and/or after processing. Some examples of additives include agents that optimize washing and disaggregation, additives that enhance the viability of the active cell population (*e.g*., adipose-derived cells comprising stem cells, adipose-derived cells comprising regenerative cells, adipose-derived cells comprising stem and regenerative cells, and the like), during processing, anti-microbial agents (e.g., antibiotics), additives that lyse adipocytes and/or red blood cells, or additives that enrich for cell populations of interest (by differential adherence to solid phase moieties or to otherwise promote the substantial reduction or enrichment of cell populations).

The adipose-derived cells, *e.g*., adipose-derived cells comprising stem cells, adipose-derived cells comprising regenerative cells, adipose-derived cells comprising stem and regenerative cells, and the like, obtained as described herein can be cultured according to approaches known in the art, and the cultured cells can be used in several of the embodied methods. For example, adipose-derived cells, *e.g*., adipose-derived cells comprising stem cells, adipose-derived cells comprising regenerative cells, adipose-derived cells comprising stem and regenerative cells, and the like, can be cultured on collagen-coated dishes or 3D collagen gel cultures in endothelial cell basal medium in the presence of low or high fetal bovine serum or similar product, as described in Ng, et al., Nov 2004, "Interstitial flow differentially stimulates blood and lymphatic endothelial cell morphogenesis in vitro," Microvasc Res. 68(3):258-64. Alternatively, adipose-derived cells, *e.g*., adipose-derived cells comprising stem cells, adipose-derived cells comprising regenerative cells, adipose-derived cells comprising stem and regenerative cells, and the like, can be cultured on other extracellular matrix protein-coated dishes. Examples of extracellular matrix proteins that may be used include, but are not limited to, fibronectin, laminin, vitronectin, and collagen IV. Gelatin or any other compound or support, which similarly promotes adhesion of endothelial cells into culture vessels may be used to culture ADRCs, as well.

Examples of basal culture medium that can be used to culture adipose-derived cells, *e.g*., adipose-derived cells comprising stem cells, adipose-derived cells comprising regenerative cells, adipose-derived cells comprising stem and regenerative cells, and the like, *in vitro* include, but are not limited to, EGM, RPMI, M199, MCDB131, DMEM, EMEM, McCoy's 5A, Iscove's medium, modified Iscove's medium or any other medium known in the art to support the growth of blood endothelial cells. Examples of supplemental factors or compounds that can be added to the basal culture medium that could be used to culture ADRCs include, but are not limited to, ascorbic acid, heparin, endothelial cell growth factor, endothelial growth supplement, glutamine, HEPES, Nu serum, fetal bovine serum, human serum, equine serum, plasma-derived horse serum, iron-supplemented calf serum, penicillin, streptomycin, amphotericin B, basic and acidic fibroblast growth factors, insulin-growth factor, astrocyte conditioned medium, fibroblast or fibroblast-like cell conditioned medium, sodium hydrogencarbonate, epidermal growth factor, bovine pituitary extract, magnesium sulphate, isobutylmethylxanthine, hydrocortisone, dexamethasone, dibutyril cyclic AMP, insulin, transferrin, sodium selenite, oestradiol, progesterone, growth hormone, angiogenin, angiopoietin-1, Del-1, follistatin, granulocyte colony-stimulating factor (G-CSF), erythropoietin, hepatocyte growth factor (HGF) /scatter factor (SF), leptin, midkine , placental growth factor, platelet-derived endothelial cell growth factor (PD-ECGF), platelet-derived growth factor-BB (PDGF-BB), pleiotrophin (PTN), progranulin, proliferin, transforming growth factor-alpha (TGF-alpha), transforming growth factor-beta (TGF-beta), tumor necrosis factor-alpha (TNF-alpha), vascular endothelial growth factor (VEGF)/vascular permeability factor (VPF), interleukin-3 (IL-3), interleukin 7 (IL-7), interleukin-8 (IL-8), ephrins, matrix metalloproteinases (such as MMP2 and MMP9), or any other compound known in the art to promote survival, proliferation or differentiation of endothelial cells.

Further processing of the cells may also include: cell expansion (of one or more regenerative cell types) and cell maintenance (including cell sheet rinsing and media changing); sub-culturing; cell seeding; transient transfection (including seeding of transfected cells from bulk supply); harvesting (including enzymatic, non-enzymatic harvesting and harvesting by mechanical scraping); measuring cell viability; cell plating (e.g., on microtiter plates, including picking cells from individual wells for expansion, expansion of cells into fresh wells); high throughput screening; cell therapy applications; gene therapy applications; tissue engineering applications; therapeutic protein applications; viral vaccine applications; harvest of regenerative cells or supernatant for banking or screening, measurement of cell growth, lysis, inoculation, infection or induction; generation of cell lines (including hybridoma cells); culture of cells for permeability studies; cells for RNAi and viral resistance studies; cells for knock-out and transgenic animal studies; affinity purification studies; structural biology applications; assay development and protein engineering applications.

In general, a system useful for isolating a population of adipose-derived cells, *e.g.,* a population of adipose-derived cells comprising stem cells, a population of adipose-derived cells comprising regenerative cells, a population of adipose-derived cells comprising stem cells and regenerative cells, comprises a) a tissue collection container including i) a tissue collecting inlet port structured to receive adipose tissue removed from a subject, and ii) a filter disposed within the tissue collection container, which is configured to retain the adipose-derived cell population from said subject and to pass adipocytes, blood, and saline; b) a mixing container or cell processing chamber coupled to the tissue collection container by a conduit such that a closed pathway is maintained, wherein said mixing container receives said cell population and said mixing container comprises an additive port for introducing at least one additive to said population of adipose-derived cells; and an outlet port configured to allow removal of said population of adipose-derived cells from the mixing container or cell processing chamber for administration to a patient. In some embodiments, said mixing container or cell processing container further comprises a cell concentration device such as a spinning membrane filter and/or a centrifuge. Examples of the embodiments disclosed herein also include a cell sorter, which is attached to said mixing chamber or cell processing chamber by a conduit and is configured to receive cells from said mixing chamber or cell processing chamber, while maintaining a closed pathway. Examples of the embodiments above may also include a centrifuge attached to said mixing chamber or cell processing chamber by a conduit and configured to receive said population of adipose-derived cells, while maintaining a closed pathway, wherein said centrifuge comprises a gradient suitable for further separation and purification of said population of adipose-derived cells (e.g., ficoll-hypaque). Said centrifuge containing said gradient, which is configured to receive said population of adipose-derived cells may also be contained within said mixing container or cell processing chamber.

### Measuring ADRCs and ADRC subsets in an isolated cell population

A measurement, analysis, or characterization of the population of adipose-derived cells described herein to determine the presence of certain cells in the population can be undertaken within the closed system of a cell processing unit or outside of the closed system of a cell processing unit using any number of protein and/or RNA detection assays available in the art. Additionally, the measurement, analysis, or characterization of the adipose-derived cells, or certain cells (*e*.*g*., stem cells, progenitor cells, precursor cells, and the like), can be part of or can accompany the isolation procedure (e.g., cell sorting using an antibody specific for certain cell types (*e.g*., regenerative cells) or gradient separation using a media selective for certain cell types).

In some embodiments the measurement or characterization of the isolated cell population is conducted by detecting the presence or absence of a protein marker that is unique to certain cell types (*e.g*., adipose-derived regenerative cells, adipose-derived stem cells, adipose-derived precursor cells, adipose-derived progenitor cells, endothelial cells, endothelial precursor cells, or the like) is otherwise considered to confirm the presence of the specific cell type of interest by those of skill in the art. In addition to conventional Western blots using antibody probes specific for said proteins or markers, immunoselection techniques that exploit on cell surface marker expression can be performed using a number of methods known in the art and described in the literature. Such approaches can be performed using an antibody that is linked directly or indirectly to a solid substrate (e.g., magnetic beads) in conjunction with a manual, automated, or semi-automated device as described by Watts, et al., for separation of CD34-positive cells (Watts, et al., 2002, Variable product purity and functional capacity after CD34 selection: a direct comparison of the CliniMACS (v2.1) and Isolex 300i (v2.5) clinical scale devices," Br J Haematol. 2002 Jul;118(1):117-23), by panning, use of a Fluorescence Activated Cell Sorter (FACS), or other means.

Separation, measurement, and characterization can also be achieved by positive selection using antibodies that recognize cell surface markers or marker combinations that are expressed by certain cell types, but not by one or more of the other cell types or subpopulations present within the cell population. Separation, measurement, and characterization can also be achieved by negative selection, in which non-desired cell types are removed from the isolated population of adipose-derived cells using antibodies or antibody combinations that do not exhibit appreciable binding to ADRCs. Markers that are specifically expressed by ADRCs have been described. Examples of antibodies that could be used in negative selection include, but are not limited to, markers expressed by endothelial cells. There are many other antibodies well known in the art that can be applied to negative selection. The relative specificity of markers for ADRCs can also be exploited in a purification and/or characterization or measurement strategy. For example, a fluorescently-labeled ligand can be used in FACS-based sorting of cells, or an ligand conjugated directly or indirectly to a solid substrate can be used to separate in a manner analogous to the immunoselection approaches described above.

Measurement and characterization of the adipose-derived cell population to determine the presence or absence of specific cell types (*e*.*g*., specific types of regenerative cells) can also involve analysis of one or more RNAs that encode a protein that is unique to or otherwise considered by those of skill in the art to be a marker that indicates the presence or absence of a ADRCs. In some embodiments, for example, the isolated cell population or a portion thereof is analyzed for the presence or absence of an RNA that encodes one or more of, e.g., CD45, CD11b, CD14, CD68, CD90, CD73, CD31 and/or CD34. The detection of said RNAs can be accomplished by any techniques available to one of skill in the art, including but not limited to, Northern hybridization, PCR-based methodologies, transcription run-off assays, gene arrays, and gene chips.

### Compositions comprising ADRCs and ADRC subsets

In accordance with the aforementioned approaches, raw adipose tissue is processed to substantially remove mature adipocytes and connective tissue thereby obtaining a heterogeneous plurality of adipose tissue-derived cells comprising adipose-derived cells, *e*.*g*., adipose-derived cells comprising stem cells, adipose-derived cells comprising regenerative cells, adipose-derived cells comprising stem and regenerative cells, and the like, suitable for placement within the body of a subject. The extracted adipose-derived cells, *e*.*g*., adipose-derived cells comprising stem cells, adipose-derived cells comprising regenerative cells, adipose-derived cells comprising stem and regenerative cells, and the like, may be provided in a neat composition comprising these cells substantially free from mature adipocytes and connective tissue or in combination with an inactive ingredient (e.g., a carrier) or a second active ingredient (e.g., adipose-derived stem cell and/or adipose-derived endothelial cell). The cells may be placed into the recipient alone or in combination (e.g., in a single composition or co-administered) with biological materials, such as cells, tissue, tissue fragments, or stimulators of cell growth and/or differentiation, supports, prosthetics, or medical devices. The composition may include additional components, such as cell differentiation factors, growth promoters, immunosuppressive agents, or medical devices, as discussed herein, for example. In some embodiments, the cells, with any of the above mentioned additives, are placed into the person from whom they were obtained (e.g., autologous transfer) in the context of a single operative procedure with the intention of providing a therapeutic benefit to the recipient.

Accordingly, aspects of the invention include compositions that comprise, consist, or consist essentially of a refined, enriched, concentrated, isolated, or purified adipose-derived cell population, *e.g*., adipose-derived cells comprising stem cells, adipose-derived cells comprising regenerative cells, adipose-derived cells comprising stem and regenerative cells, and the like, and mixtures of these cells with a biological material, additive, support, prosthetic, or medical device, including but not limited to, unprocessed adipose tissue, adipocytes, collagen matrix or support, cell differentiation factors, growth promoters, immunosuppressive agents, processed adipose tissue containing adipose-derived stem cells and/or progenitor cells, and cell populations already containing an enriched amount of ADRCs. In some embodiments, the aforementioned compositions comprise an amount or concentration of refined, isolated, or purified ADRCs that is greater than or equal to 0.5%-1%, 1-2%, 2%-4%, 4%-6%, 6%-8%, 8%-10%, 10%-20%, 20%-30%, 30%-40%, 40%-50%, 50%-60%, 60%-70%, 70%-80%, 80%-90%, or 90%-100% ADRCs, as compared to the total adipose-tissue cell population. In some embodiments, the ADRCs express an amount of, e.g., CD45, CD11b, CD14, CD68, CD90, CD73, CD31 and/or CD34..

In some embodiments, the adipose-derived cell *e.g*., adipose-derived cells comprising stem cells, adipose-derived cells comprising regenerative cells, adipose-derived cells comprising stem and regenerative cells, and the like, described herein is formulated in compositions that include at least one pharmaceutically acceptable diluent, adjuvant, or carrier substance, using any available pharmaceutical chemistry techniques. Generally, this entails preparing compositions that are essentially free of impurities that could be harmful to humans or animals.

Appropriate salts and buffers can be employed to stabilize and to facilitate uptake of the adipose-derived cell population that comprises ADRCs. Compositions contemplated herein can comprise an effective amount of the adipose-derived cells *e*.*g*., adipose-derived cells comprising stem cells, adipose-derived cells comprising regenerative cells, adipose-derived cells comprising stem and regenerative cells, and the like, in a pharmaceutically acceptable carrier or aqueous medium.

Administration of the compositions described herein can be via any common route so long as the target tissue is available via that route. Compositions administered according to the methods described herein may be introduced into the subject by, e.g., by intravenous, intraarterial, intralymphatic, subcutaneous, intradermal, intramuscular, intramammary, intraperitoneal, intrathecal, retrobulbar, intrapulmonary (e.g., term release); by oral, sublingual, nasal, anal, vaginal, or transdermal delivery, by spray or other direct application, or by surgical implantation at a particular site. In each of these methods of administration the compositions may or may not comprise a carrier or other material that has the property of increasing retention of the composition at the site of action or of facilitating the traffic of the composition to the site of action. The introduction may consist of a single dose or a plurality of doses over a period of time. Vehicles for cell therapy agents are known in the art and have been described in the literature. See, for example Remington's Pharmaceutical Sciences, 18th Ed. (1990, Mack Publ. Co, Easton Pa. 18042) pp 1435-1712. Sterile solutions are prepared by incorporating the adipose-derived cell population *e.g*., adipose-derived cells comprising stem cells, adipose-derived cells comprising regenerative cells, adipose-derived cells comprising stem and regenerative cells, and the like, in the required amount in the appropriate buffer with or without one or more of the other components described herein.

Combination therapy with any two or more agents described herein also is contemplated as an aspect of the invention. Similarly, every combination of agents described herein, packaged together as a new kit, or formulated together as a single composition, is considered an aspect of the invention. Compositions for use according to aspects of the invention preferably include the adipose-derived cell population *e.g*., adipose-derived cells comprising stem cells, adipose-derived cells comprising regenerative cells, adipose-derived cells comprising stem and regenerative cells, and the like, formulated with a pharmaceutically acceptable carrier. The cells can also be applied with additives to enhance, control, or otherwise direct the intended therapeutic effect. For example, in some embodiments, the adipose-derived cell population *e.g*., adipose-derived cells comprising stem cells, adipose-derived cells comprising regenerative cells, adipose-derived cells comprising stem and regenerative cells, and the like, can be further purified by use of antibody-mediated positive and/or negative cell selection to enrich the cell population to increase efficacy, reduce morbidity, or to facilitate ease of the procedure. Similarly, cells can be applied with a biocompatible matrix, which facilitates *in vivo* tissue engineering by supporting and/or directing the fate of the implanted cells. In the same way, cells can be administered following genetic manipulation such that they express gene products that are believed to or are intended to promote the therapeutic response provided by the cells.

The adipose-derived cell population, *e.g*., adipose-derived cells comprising stem cells, adipose-derived cells comprising regenerative cells, adipose-derived cells comprising stem and regenerative cells, and the like, can be applied alone or in combination with other cells, tissue, tissue fragments, growth factors, biologically active or inert compounds, resorbable plastic scaffolds, or other additive intended to enhance the delivery, efficacy, tolerability, or function of the population. The adipose-derived cell population that comprises ADRCs can also be modified by insertion of DNA or by placement in cell culture in such a way as to change, enhance, or supplement the function of the cells for derivation of a structural or therapeutic purpose.

In more embodiments, the adipose-derived cell population *e.g*., adipose-derived cells comprising stem cells, adipose-derived cells comprising regenerative cells, adipose-derived cells comprising stem and regenerative cells, and the like, are combined with a gene encoding a pro-drug converting enzyme which allows cells to activate pro-drugs within the site of engraftment, that is, within a tumor. Addition of the gene (or combination of genes) can be by any technology known in the art including but not limited to adenoviral transduction, "gene guns," liposome-mediated transduction, and retrovirus or lentivirus-mediated transduction, plasmid, or adeno-associated virus. Cells can be implanted along with a carrier material bearing gene delivery vehicle capable of releasing and/or presenting genes to the cells over time such that transduction can continue or be initiated in situ. Particularly when the cells and/or tissue containing the cells are administered to a patient other than the patient from whom the cells and/or tissue were obtained, one or more immunosuppressive agents can be administered to the patient receiving the cells and/or tissue to reduce, and preferably prevent, rejection of the transplant.

Still more embodiments concern the *ex vivo* transfection of an adipose-derived cell population, *e.g*., adipose-derived cells comprising stem cells, adipose-derived cells comprising regenerative cells, adipose-derived cells comprising stem and regenerative cells, and the like, and subsequent transfer of these transfected cells to subjects. It is contemplated that such embodiments can be an effective approach to upregulate *in vivo* levels of the transferred gene and for providing relief from a disease or disorder resulting from under-expression of the gene(s) or otherwise responsive to upregulation of the gene (*see e.g.,* Gelse, et al., 2003, "Articular cartilage repair by gene therapy using growth factor-producing mesenchymal cells," Arthritis Rheum. 48:430-41; Huard, et al, 2002, "Muscle-derived cell-mediated ex vivo gene therapy for urological dysfunction," Gene Ther. 9:1617-26; Kim, et al., 2002, "Ex vivo gene delivery of IL-IRa and soluble TNF receptor confers a distal synergistic therapeutic effect in antigen-induced arthritis," Mol. Ther. 6:591-600). Delivery of an adipose-derived cell population, *e.g*., adipose-derived cells comprising stem cells, adipose-derived cells comprising regenerative cells, adipose-derived cells comprising stem and regenerative cells, and the like,to appropriate cells is effected *ex vivo*, *in situ*, or *in vivo* by use of vectors, and more particularly viral vectors (e.g., adenovirus, adeno-associated virus, or a retrovirus), or *ex vivo* by use of physical DNA transfer methods (e.g., liposomes or chemical treatments). See, for example, Anderson, 1998, "Human Gene Therapy," Nature Suppl. to vol. 392 (6679):25-20. Gene therapy technologies are also reviewed by Friedmann, 1989, "Progress toward human gene therapy," Science 244(4910):1275-1281, Verma (1990), "Gene therapy." Scientific American 263(5): 68-84, and Miller (1992), "Human gene therapy comes of age," Nature, 357:455-460. An adipose-derived cell population, *e.g*., adipose-derived cells comprising stem cells, adipose-derived cells comprising regenerative cells, adipose-derived cells comprising stem and regenerative cells, and the like, can be cultured *ex vivo* in the presence of an additive (e.g., a compound that induces differentiation or pancreatic cell formation) in order to proliferate or to produce a desired effect on or activity in such cells. Treated cells can then be introduced to a subject.

In some embodiments, the *ex vivo* gene therapy is conducted locally, e.g., to the site of a fibrotic scar. For example, by using catheter-mediated transfer an adipose-derived cell population, *e.g*., adipose-derived cells comprising stem cells, adipose-derived cells comprising regenerative cells, adipose-derived cells comprising stem and regenerative cells, and the like, can be transferred into a mammalian subject. Materials and methods for local delivery are reviewed, e.g., in Lincoff, et al. (1994), "Local drug delivery for the prevention of restenosis. Fact, fancy, and future," Circulation, 90: 2070-2084. For example, adipose-derived cells .*g*., adipose-derived cells comprising stem cells, adipose-derived cells comprising regenerative cells, adipose-derived cells comprising stem and regenerative cells, and the like, can be provided to a subject by an infusion-perfusion balloon catheter (preferably a microporous balloon catheter), such as those that have been described in the literature for intracoronary drug infusions. See, e.g., U.S. Pat. No. 5,713,860 (Intravascular Catheter with Infusion Array); U.S. Pat. No. 5,087,244; U.S. Pat. No. 5,653,689; Wolinsky, et al. (1990) (Wolinsky Infusion Catheter), "Use of a perforated balloon catheter to deliver concentrated heparin into the wall of the normal canine artery," J. Am. Coll. Cardiol. 15: 475-481; and Lambert et al., 1993, "Local drug delivery catheters: functional comparison of porous and microporous designs," Coron. Artery Dis. 4: 469-475. Use of such catheters for site-directed somatic cell gene therapy is described, e.g., in Mazur, et al., 1994, "Coronary restenosis and gene therapy," Texas Heart Institute Journal 21: 104-111.

Aspects of the invention also concern the *ex vivo* transfection of adipose-derived cells, *e.g.,* ADRCs (stem cells, progenitor cells, precursor cells, or combinations of stem cells and progenitor cells and/or precursor cells) with a gene encoding a therapeutic polypeptide, and administration of the transfected cells to the mammalian subject. Procedures for seeding a vascular graft with genetically modified endothelial cells are described in, e.g., U. S. Pat. No. 5,785,965, "VEGF gene transfer into endothelial cells for vascular prosthesis,".

In some embodiments, the administering step comprises implanting a prosthetic or medical device (e.g., intravascular stent) in the mammalian subject, where the stent is coated or impregnated with an adipose-derived cell population that comprises ADRCs. Exemplary materials for constructing valves, stents or grafts coated or seeded with transfected endothelial cells are described in Pavcnik, et al., 2004, "Second-generation percutaneous bioprosthetic valve: a short-term study in sheep," Eur. J. Endovasc. Surg. 40:1223-1227, and Arts, et al., 2002, "Contaminants from the Transplant Contribute to Intimal Hyperplasia Associated with Microvascular Endothelial Cell Seeding," Eur. J. Endovasc. Surg. 23:29-38. See also U. S. Pat. App. Ser. No. 11/317,422, entitled CELL-LOADED PROSTHESIS FOR REGENERATIVE INTRALUMINAL APPLICATIONS, filed December 22, 2005. For example, in one variation, a synthetic valve that comprises an adipose-derived cell population that comprises ADRCs is sutured to a square stainless steel stent. The square stent has a short barb at each end to provide anchors for the valve during placement, and the submucosa membrane is slit at the diagonal axis of the stent to create the valve opening.

Surfaces of the synthetic valve can be coated with a transfected or non-transfected adipose-derived cell population that comprises that comprises regenerative cells *(e.g.,* that comprises stem cells, that comprises progenitor cells, that comprises precursor cells, or other regenerative cells - including any combination thereof) e.g., by placing the synthetic valve in an appropriate cell culture medium for 1-3 days prior to implantation to allow for complete coverage of valve surface with the cells.

In another embodiment, the administering step comprises implanting an intravascular stent in the mammalian subject, where the stent is coated or impregnated, as described in literature cited above and reviewed in Lincoff, et al., 1994. A metal or polymeric wire for forming a stent is coated with a composition such as a porous biocompatible polymer or gel that is impregnated with (or can be dipped in or otherwise easily coated immediately prior to use with) a transfected or non-transfected adipose-derived cell population that comprises regenerative cells (*e*.*g*., that comprises stem cells, that comprises progenitor cells, that comprises precursor cells, or other regenerative cells-including any combination thereof). The wire is coiled, woven, or otherwise formed into a stent suitable for implantation into the lumen of a vessel using conventional materials and techniques, such as intravascular angioplasty catheterization. Exemplary stents that may be improved in this manner are described and depicted in U. S. Pat. Nos. 5,800,507 and 5,697,967 (Medtronic, Inc., describing an intraluminal stent comprising fibrin and an elutable drug capable of providing a treatment of restenosis); U. S. Pat. No. 5,776,184 (Medtronic, Inc., describing a stent with a porous coating comprising a polymer and a therapeutic substance in a solid or solid/solution with the polymer); U. S. Pat. No. 5,799,384 (Medtronic, Inc., describing a flexible, cylindrical, metal stent having a biocompatible polymeric surface to contact a body lumen); and U. S. Pat. Nos. 5,824,048, 5,679,400 and 5,779,729.

As disclosed herein, the adipose-derived cell population that comprises regenerative cells (*e*.*g*., that comprises stem cells, that comprises progenitor cells, that comprises precursor cells, or other regenerative cells-including any combination thereof) may be provided to the subject, or applied directly to the damaged tissue, or in proximity to the damaged tissue, without further processing or following additional procedures to further purify, modify, stimulate, or otherwise change the cells. For example, the cells obtained from a patient may be provided back to said patient without culturing the cells before administration. In several embodiments, the collection and processing of adipose tissue, as well as, administration of the adipose-derived cell population that comprises ADRCs is performed at a patient's bedside. In a preferred embodiment the cells are extracted from the adipose tissue of the person into whom they are to be implanted, thereby reducing potential complications associated with antigenic and/or immunogenic responses to the transplant. However, the use of cells extracted from another individual is also contemplated.

In accordance with the invention herein disclosed, the adipose tissue-derived cells can be delivered to the patient soon after harvesting the adipose tissue from the patient. For example, the cells may be administered immediately after the processing of the adipose tissue to obtain a composition of adipose tissue-derived regenerative cells (*e*.*g*., adipose-derived stem cells, progenitor cells, precursor cells, or any combination of regenerative cells). In one embodiment, the preferred timing of delivery should take place on the order of hours to days after diagnosis of edema or of a procedure likely to place the patient at risk for developing edema. In another embodiment, the harvest and, in certain cases the treatment, can take place in advance of a procedure likely to induce a pancreatic disorder. Ultimately, the timing of delivery will depend upon patient availability and the time required to process the adipose tissue. In another embodiment, the timing for delivery may be relatively longer if the cells to be delivered to the patient are subject to additional modification, purification, stimulation, or other manipulation, as discussed herein. Furthermore, the adipose-derived cell population that comprises ADRCs may be administered multiple times. For example, the cells may be administered continuously over an extended period of time (e.g., hours), or may be administered in multiple injections extended over a period of time. For example, in some embodiments, the cells can be administered in one or more injections (*e.g*., 2, 3, 4, 5, 6, 7, 8, 9, 10, or more), into affected tissue of the subject. By way of example, in some embodiments, a subject having scleroderma can be administered adipose-derived cells as disclosed herein in one or a plurality of different sites in each finger. Accordingly, some embodiments provide for the administration of cells on each side of fingers, *e*.*g*., each side of the finger next to the first and second phalanx..

The number of the adipose-derived cells, *e.g*., adipose-derived cells comprising stem cells, adipose-derived cells comprising regenerative cells, adipose-derived cells comprising stem cells and other regenerative cells, and the like, administered to a patient may be related to the cell yield after adipose tissue processing. In addition, the dose delivered will depend on the route of delivery of the cells to the patient. Fewer cells may be needed when intra-pancreatic delivery systems are employed, as these systems and methods can provide the most direct pathway for treating pancreatic conditions. The cell dose administered to the patient will also be dependent on the amount of adipose tissue harvested and the body mass index of the donor (as a measure of the amount of available adipose tissue). The amount of tissue harvested will also be determined by the extent of the injury or insufficiency. Multiple treatments using multiple tissue harvests or using a single harvest with appropriate storage of cells between applications are within the scope of this invention.

A portion of the total number of adipose-derived cells - *e.g.,* adipose-derived cells comprising stem cells, adipose-derived cells comprising regenerative cells, adipose-derived cells comprising stem cells and other regenerative cells, and the like, may be retained for later use or cryopreserved. Portions of the processed adipose tissue may be stored before being administered to a patient. For short term storage (e.g., less than 6 hours) cells may be stored at or below room temperature in a sealed container with or without supplementation with a nutrient solution. Medium term storage (e.g., less than 48 hours) is preferably performed at 2-8°C in an isosmotic, buffered solution (for example Plasmalyte®) in a container composed of or coated with a material that prevents cell adhesion. Longer term storage is preferably performed by appropriate cryopreservation and storage of cells under conditions that promote retention of cellular function, such as disclosed in PCT App. No. PCT/US02/29207, filed September 13, 2002 and U.S. Pat. App. Ser. No. 60/322,070, filed September 14, 2001

In some embodiments, the amount of adipose derived cells (e.g., an enriched, concentrated, isolated, or purified population of the adipose-derived cells comprising ADRCs), which is provided to a subject in need thereof is greater than or equal to about 10,000, 20,000, 30,000, 40,000, 50,000, 60,000, 70,000, 80,000, 90,000, 100,000, 110,000, 120,000, 130,000,140,000, 150,000, 160,000, 170,000, 180,000, 190,000, or 200,000 cells and the amount of ADRCs (*e*.*g*., the amount of stem cells, progenitor cells, precursor cells, or a combination of different types of regenerative cells - such as a combination of stem cells and progenitor cells) in said population of adipose derived cells can be greater than or equal to 0.5%-1%, 1-2%, 2%-4%, 4%-6%, 6%-8%, 8%-10%, 10%-20%, 20%-30%, 30%-40%, 40%-50%, 50%-60%, 60%-70%, 70%-80%, 80%-90%, or 90%-100% of the total population of adipose derived cells. The dose can be divided into several smaller doses, e.g., for administering over a period of time or for injection into different parts of the affected tissue, e.g., by local injection. However, this dosage can be adjusted by orders of magnitude to achieve the desired therapeutic effect.

The adipose-derived cells (*e.g*., adipose-derived cells comprising stem cells, adipose-derived cells comprising regenerative cells, adipose-derived cells comprising stem cells and other regenerative cells, and the like) can also be subjected to cell culture on a scaffold material prior to being implanted. Thus, tissue engineered valves, pancreatic vessels, and other structures could be synthesized on natural or synthetic matrices or scaffolds using ADRCs prior to insertion or implantation into the recipient.

Many routes of administration can be suitable for the therapeutics described herein. In some variations, oral, intravenous, intraarterial, and other systemic administrations are used. In some variations, local delivery to an edematous limb or other portion of the body, such as administered subcutaneously at a site of edema, is contemplated.

In some embodiments, direct administration of the adipose-derived cells (*e.g*., adipose-derived cells comprising stem cells, adipose-derived cells comprising regenerative cells, adipose-derived cells comprising stem cells and other regenerative cells, and the like) to the site of intended benefit is preferred. This can be achieved by local injection into the tissue, direct injection into a pancreatic structure or pancreatic vessel, through insertion of a suitable cannula, by arterial or venous infusion (including retrograde flow mechanisms) or by other means disclosed herein or known in the art.

The adipose-derived cell population, *e.g*., the cell population that includes stem cells, regenerative cells, stem cells and other regenerative cells, or the like, can be applied by several routes including systemic administration by venous or arterial infusion (including retrograde flow infusion and including infusion into blood and/or lymphatic vessels) or by direct injection. Systemic administration, particularly by peripheral venous access, has the advantage of being minimally invasive relying on the natural transport of cells from the blood to the pancreas. The adipose-derived cell population that comprises ADRCs can be injected in a single bolus, through a slow infusion, or through a staggered series of applications separated by several hours or, provided cells are appropriately stored, several days or weeks. The adipose-derived cell population that comprises ADRCs can also be applied by use of catheterization such that the first pass of cells through the area of interest is enhanced by using balloons. As with peripheral venous access, the adipose-derived cell population that comprises ADRCs may be injected through the catheters in a single bolus or in multiple smaller aliquots. Cells can also be injected into interstitial space or applied in the form of a spray or sheet.

As previously set forth above, in a preferred embodiment, the adipose-derived cell population, e.g., the cell population that includes stem cells, regenerative cells, stem cells and other regenerative cells, or the like, is administered directly into the patient. In other words, the active cell population (e.g., the ADRCs, progenitor cells, stem cells and/or combinations thereof) are administered to the patient without being removed from the system or exposed to the external environment of the system before being administered to the patient. Providing a closed system reduces the possibility of contamination of the material being administered to the patient. Thus, processing the adipose tissue in a closed system provides advantages over existing methods because the active cell population is more likely to be sterile. In some embodiments, the only time the adipose-derived cell population that comprises ADRCs are exposed to the external environment, or removed from the system, is when the cells are being withdrawn into an application device and administered to the patient. In other embodiments, the application device can also be part of the closed system. Accordingly, a complete closed system is maintained from removal of the adipose tissue from the subject (e.g., cannula) to introduction to the subject (e.g., application device). Thus, the cells used in these embodiments are may be processed for culturing or cryopreservation and may be administered to a patient without further processing, or may be administered to a patient after being mixed with other tissues, cells, or additives.

In other embodiments, at least a portion of the adipose-derived cell population that comprises ADRCs can be stored for later implantation/infusion. The population may be divided into more than one aliquot or unit such that part of the population of cells is retained for later application while part is applied immediately to the patient. Moderate to long-term storage of all or part of the cells in a cell bank is also within the scope of this invention, as disclosed in U.S. Pat. App. Ser. No. 10/242,094, entitled PRESERVATION OF NON EMBRYONIC CELLS FROM NON HEMATOPOIETIC TISSUES, filed September 12, 2002, which claims the benefit of U.S. App. Ser. No. 60/322,070, filed September 14, 2001. At the end of processing, the concentrated cells may be loaded into a delivery device, such as a syringe, for placement into the recipient by any means known to one of ordinary skill in the art. The adipose-derived cell population that comprises ADRCs with or without an additive can be used in several therapeutic methods as described in the following section.

### Pain Models

In certain embodiments, the adipose derived cells, e.g., adipose-derived cells comprising stem cells, adipose-derived cells comprising regenerative cells, adipose-derived cells comprising stem and regenerative cells and the like described herein are characterized by their ability to reduce or ameliorate pain, e.g., in an animal pain model. For example, where a batch or lot of adipose derived stem and regenerative cells is produced, a sample of the batch or lot can be tested using one or more animal models of pain. Adipose derived stem and regenerative cells, from which samples that produce acceptable reductions in pain in a pain assay have been obtained, can then be selected for further use, e.g., for amelioration of any type of pain, or for amelioration of a specific type of pain. It should be understood that the adipose derived stem and regenerative cells tested need only be tested in, and/or show efficacy in one assay to be considered therapeutically effective; testing in multiple, or all, animal models of pain is not necessary. In certain embodiments, the sample adipose derived stem and regenerative cells can be tested in a pain assay that is relevant to one or more related types of pain, or pain relevant to treating a particular patient population.

In certain embodiments, the adipose derived cells, *e.g*., adipose-derived cells comprising stem cells, adipose-derived cells comprising regenerative cells, adipose-derived cells comprising stem and regenerative cells and the like can be tested in, for example, an acetic acid-induced model of visceral pain. Such a study can be conducted, e.g., by administering acetic acid intraperitoneally in a dose volume of about 10 mL/kg to mice, with vehicle or adipose derived stem and regenerative cells (e.g., 1×10⁶ to 1×10⁸) administered prior to acetic acid administration. The number of writhings are recorded for the subsequent 20 minutes after discarding the first the 5 minutes. Each experiment contains the following groups: vehicle+2-5 doses of adipose derived stem and regenerative cells+positive control; n=10/group.

In certain other embodiments, a sample of adipose derived stem and regenerative cells can be tested using the Chung spinal nerve ligation model which L5 and L6 spinal nerves are tightly ligated, resulting in a stable and long-lasting neuropathic pain.

In certain other embodiments, a sample of adipose derived cells, *e.g.,* adipose-derived cells comprising stem cells, adipose-derived cells comprising regenerative cells, adipose-derived cells comprising stem and regenerative cells and the like can be tested using a taxol assay of peripheral neuropathy in which pain develops over time in the rat after the administration of a series of Taxol injections.

In certain other embodiments, a sample of adipose-derived cells *e.g.,* adipose-derived cells comprising stem cells, adipose-derived cells comprising regenerative cells, adipose-derived cells comprising stem and regenerative cells and the like, can be tested using the Bennet Model of neuropathic pain (allodynia), in which pain, induced by application of loose ligatures around one of the sciatic nerves, is shown by the sharp withdrawal of affected hind paw to light mechanical stimuli.

In certain other embodiments, a sample of adipose derived cells, *e.g.,* adipose-derived cells comprising stem cells, adipose-derived cells comprising regenerative cells, adipose-derived cells comprising stem and regenerative cells and the like, can be tested using a carrageenan administration-induced model of pain.

In certain other embodiments, a sample of adipose derived cells, *e.g.,* adipose-derived cells comprising stem cells, adipose-derived cells comprising regenerative cells, adipose-derived cells comprising stem and regenerative cells and the like, can be tested using a Complete Freund's Adjuvant model of neuropathic pain (e.g., allodynia). For example, five rats can be used per group, with an initial dose of, e.g., 1×10⁶ to 1×10⁷ i.v., with efficacy determined by reduction of CFA-induced hind paw hyperalgesia. Unpaired Student's t test is applied for comparison between vehicle control and treated groups. Other pain medications can be used as positive controls (mg/kg per os), e.g., aspirin>100; cyclosporine A>100; dexamethasone>30; gabapentin^{∼}200; indomethacin>10; or morphine 30.

In certain other embodiments, a sample of adipose derived *e.g*., adipose-derived cells comprising stem cells, adipose-derived cells comprising regenerative cells, adipose-derived cells comprising stem and regenerative cells and the like, can be tested using phenylquinone (PQ) as a pain-engendering compound, e.g., using five mice per condition, an initial dose of, e.g., 1×10⁶ to 1×10⁷ i.v., 1 hr pretreatment, followed by determination of reduction of PQ (2 mg/kg i.p.)-induced writhing during a 5 min observation period.

In certain other embodiments, a sample of adipose derived *e.g*., adipose-derived cells comprising stem cells, adipose-derived cells comprising regenerative cells, adipose-derived cells comprising stem and regenerative cells and the like, can be tested using a hind paw incision model of, e.g., postoperative pain.

In certain other embodiments, a sample of adipose derived cells, *e.g.,* adipose-derived cells comprising stem cells, adipose-derived cells comprising regenerative cells, adipose-derived cells comprising stem and regenerative cells and the like, can be tested using a tail flick model, in which response to tail flick pain stimulus is assessed before and after administration of the adipose derived stem and regenerative cells.

The pain assays provided herein are non-limiting examples only; other assays known in the art may be used as well.

### EXAMPLES

### EXAMPLE 1

Scleroderma is a chronic disease characterized by excessive deposits of collagen in the skin or other organs. Scleroderma can be localized or generalized. The localized form of the disease, while disabling, tends not to be fatal. The generalized form of the disease, manifesting as diffuse scleroderma or systemic sclerosis, can be fatal as a result of heart, kidney, lung or intestinal damage. The three types of scleroderma are diffuse scleroderma and limited (CREST syndrome) scleroderma, which are systemic, and morphea/linear scleroderma, which is limited to the skin. Diffuse scleroderma is the most severe form, with victims experiencing rapid onset, widespread skin hardening, and significant internal organ damage, particularly to the lungs and gastrointestinal tract.

The limited form of scleroderma is much milder, exhibiting a slower onset and progression. Skin hardening is usually confined to the hands and face, internal organ involvement is less severe than in the diffuse form. Typically, Raynaud's phenomenon may precede scleroderma by several years. Raynaud's phenomenon is due to vasoconstriction of the small arteries of exposed peripheries--particularly the hands and feet--in the cold, and is classically characterized by a triphasic color change--first white, then blue and finally red on rewarming. The limited form is often referred to as CREST syndrome, where "CREST" is an acronym for the five main features, calcinosis (calcium deposits in soft tissue, e.g., the skin), Raynaud's syndrome, esophageal dysmotility, sclerodactyly (scleroderma of the fingers), and telangiectasia (spider veins).

A human clinical trial was performed in which 12 scleroderma patients (average age 54.5±10.3 years; range 34-68 years) were treated with Adipose-Derived Regenerative Cells. All patients had a diagnosis of scleroderma with associated Raynaud's syndrome with 14.3±7.7 (mean ± standard deviation) duration of disease (range 5 years to 34 years).

Following informed consent and the completion of baseline, pre-treatment evaluations, the patient was anesthetized and placed in a sterile field. A small number of 0.5cm stab incisions were created using an 11 blade scalpel. Tumescent solution comprising lactated Ringers solution supplemented with xylocaine and adrenaline was infiltrated into the subcutaneous tissue at a ratio of approximately 1 volume of solution to 1 volume of adipose to be aspirated.

After allowing sufficient time for the epinephrine to induce vasoconstriction, the adipose tissue was aspirated through the same incision site using a 2mm diameter Mercedes-style liposuction cannula (Mentor, Santa Barbara, CA). Approximately 120g of adipose tissue was aspirated. The incision site was then closed with a suture and a pressure bandage applied to the area.

Adipose-derived cells, *e.g*., adipose-derived cells comprising regenerative cells, were processed using the methods described herein. The 5mL output was then diluted with lactated Ringers solution to a final volume of 10ml (1mL per digit).

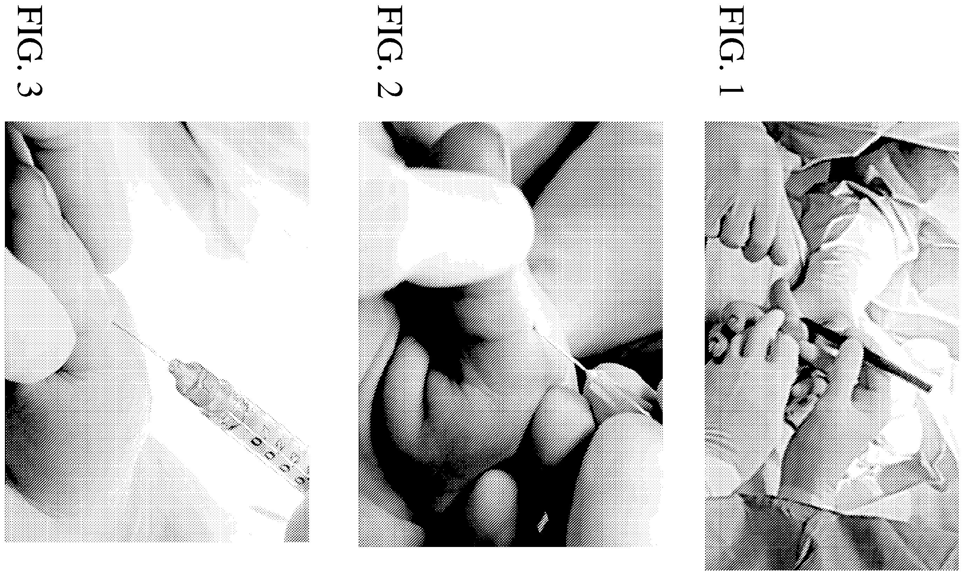

ADRCs were injected into the subcutaneous and subdermal space by injection through a blunt cannula. Two injections were performed for each finger (one on each side of the digit laterally along the length of the digit; ∼0.5mL injection per side). First, the injection sites on both dorso-lateral aspects of each finger and thumb were marked as shown in **Figure 1****.**

An incision was made at the first injection site using sharp needle (25G) as shown in **Figure 2****.**

A blunt cannula was then inserted into the incision site. A 1mL syringe attached to the cannula was used to deliver 1mL of ADRCs per finger (0.5mL into each side of the finger) as shown in **Figure 3**. In order to perform accurate injection magnification was used as needed by the treating surgeon.

The injection site was then closed with a dry dressing and the subsequent injection was performed as above until all 20 injections were completed.

### Evaluations

The primary endpoints for this study were: pain (as assessed by a Visual Analog Scale; VAS), Raynaud's Severity Score, and two widely assessments of hand function; the Cochin Score and the Scleroderma Health Assessment Questionnaire. These were preformed prior to treatment (baseline) and at intervals following treatment. Data are provided herein for the two and six month follow-up time points.

### 1. Raynaud's Severity Score

Raynaud's Syndrome is a painful condition in which stimuli such as cold induce vasoconstriction and loss of blood flow to the extremities, in particular, the end of the fingers. A period of severe ischemia is then followed by reperfusion and significant pain. Scleroderma patients can have multiple daily Raynaud's episodes frequently lasting an hour or more each. The Raynaud's Score (RS) is a validated patient-reported measure of the severity of the disease.

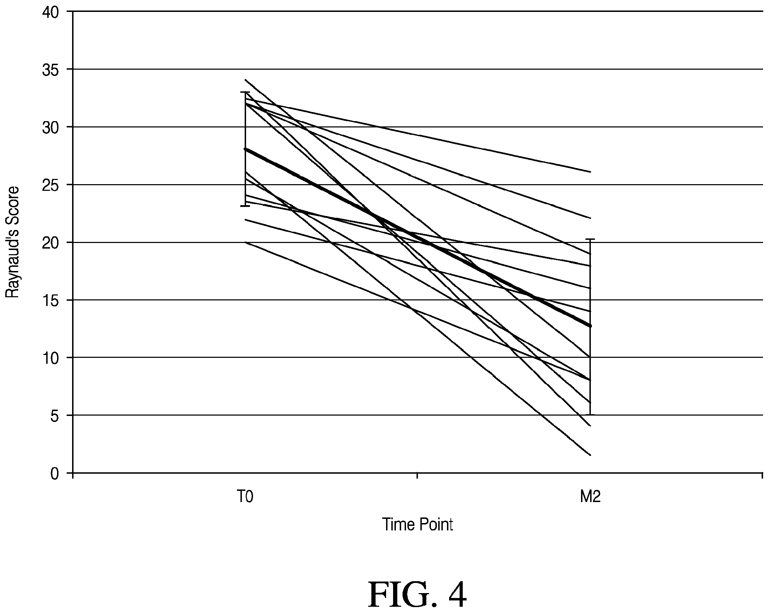

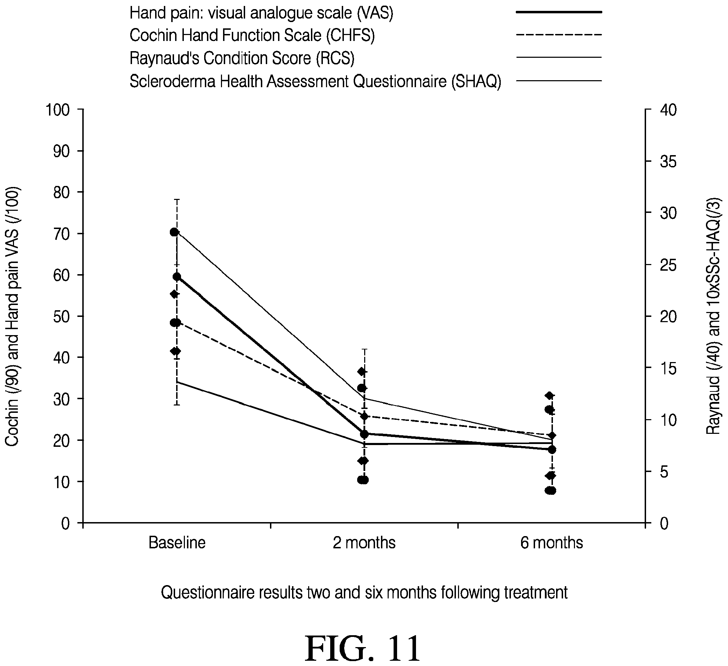

Average RS at baseline was 28±5 (mean ± standard deviation; range 20-34). Two months after treatment the RS had improved to an average of 12±8 (range 1.5-26). This change was statistically significant (p<0.001 by paired T test). Every patient exhibited an improvement in RS over the two month timeframe. A graph showing the data for each patient along with the mean and standard deviation (bold line) at two months is shown in **Figure 4**. A graph showing the data for the combined group is shown in **Figure 11****,**

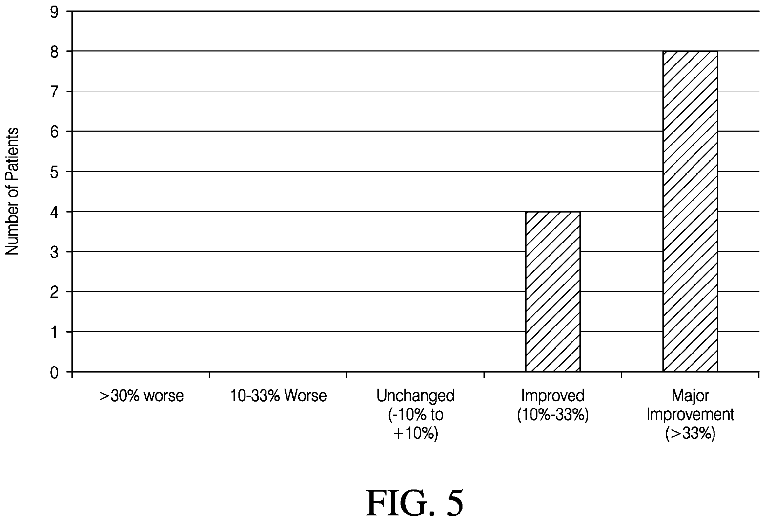

The data further show that all patients exhibited a minimum of a 20% improvement in their Raynaud's Condition Score ("RCS"), as shown in **Figure 5****.** Across all patients, a 53.7% reduction in the RCS was observed from baseline to month 2 (p<.0001), and reached a 67.5% reduction at month 6 (p<.0001). *See,* **Figure 11****.** These data, as well as data regarding hand pain (VAS) and nailfold capillaroscopy discussed below demonstrate that the adipose-derived regenerative cells disclosed herein are useful in improving vascular manifestations of Scleroderma.

### 2. Pain

Pain is a frequent issue for patients with scleroderma. This arises from Raynaud's Syndrome, the presence of digital ulcers, and other factors such as the stiffness and limited range of motion of the hand.

Pain was assessed using a standard Visual Analog Scale in which patients report their sense of their current pain on a scale of one to 100 by, for example, placing a mark on a 100mm line. This is a very widely used, well-validated tool for assessing pain in a wide array of clinical conditions including scleroderma.

Average Pain at baseline was 59.4±17.2 (mean ± standard deviation; range 20-80). Two months after treatment the Pain score had improved to an average of 21.6±17.5 (range 0-40). This change was statistically significant (p<0.001 by paired T test). The collective data for the 12 patients is provided in **Figure 11****.**

A study of the relevance of changes in pain assessed using the VAS tool showed that a score of 0-4mm may be assessed as "no pain", 5-44mm as "mild pain", 45-75 as "moderate pain", and 75-100 as "severe pain". By these criteria there were two patients in severe pain prior to treatment; none after treatment-indeed, both patients in severe pain prior to treatment exhibited no pain at two months after treatment. By the same criteria there were nine patients exhibiting moderate pain prior to treatment but only one at two months. Eleven of 12 patients (92%) evaluated at two months had only mild or no pain compared to only one patient (8%) at baseline. The same study of the VAS tool indicated that a change in pain of 33% is clinically significant to the patient. Nine of 12 patients (75%) treated exhibited a decrease in pain of at least 33%.

### 3. Health Assessment Questionnaire

Disease states such as scleroderma take a significant toll on the general health and quality of life of patients. The Scleroderma Health Assessment Questionnaire (SHAQ) is a well-established and validated tool for evaluating health in patients with scleroderma.

Health was assessed using a standard SHAQ. The average score at baseline was 1.28±0.31 (mean ± standard deviation; range 0.8-1.9). Two months after treatment the average SHAQ score had improved to an average of 0.66±0.56 (range 0-1.5). This change was statistically significant (p<0.001 by paired T test). A graph showing the data for each patient along with the mean and standard deviation (bold line) is shown below.

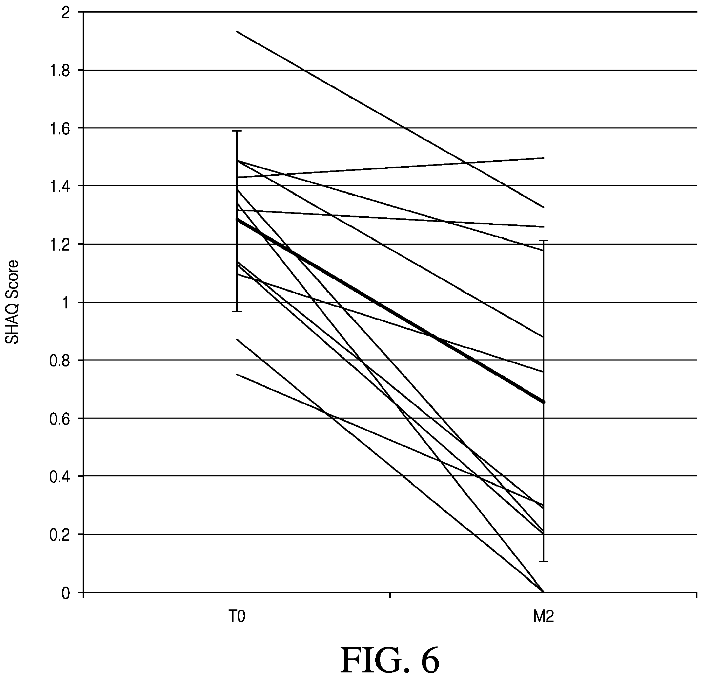

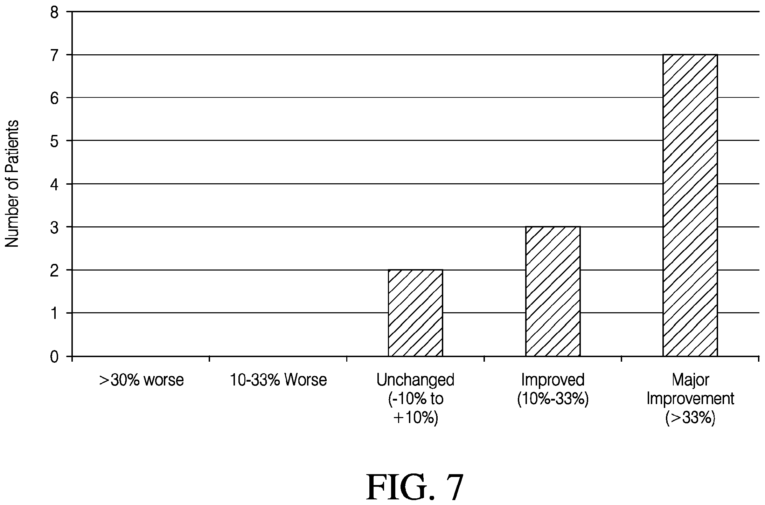

Studies have shown that a change in score of this type of ≥0.2 is symptomatically significant to the patient. The average improvement observed in this study was 0.6. Further, 10 of 12 patients exhibited an improvement in SHAQ of greater than 0.2. The average improvement in SHAQ in this group was 0.75±0.35 (range 0.3 to 1.3). The data at two months is presented in **Figures 6** **and** **7****.** By way of comparison, a 281 patient clinical trial of iloprost, an intravenous drug approved in the European Union for treatment of scleroderma, the mean improvement in overall SHAQ was 0.23±0.64. A 45.2% improvement in patient's perceived health status was observed at 2 months (p=0.001), and a 42.4% improvement in patient's perceived health status was observed at 6 months, as assessed by A graph showing the 6 month data is provided in **Figure 11****.**

As shown in **Figure 11**, the majority of patients exhibited major improvements in the SHAQ score. SHAQ can also be regarded as a useful marker of change in SSc status in clinical practice because an improvement in HAQ is associated with an improvement in physician global assessment. This global aspect indicates the ability of the intervention to apply benefit to patients at regions distal to the treatment site (hands) and is consistent with patient reports of improvement in scleroderma symptoms in the face and other areas. These data demonstrate that the adipose-derived regenerative cells disclosed herein are useful for sustained improvement (e.g., longer than 6 months) in overall and general health status of subjects with Scleroderma.

### 4. ADRCs Improve Hand Disability: Cochin Scale, Grip Strength, Pinch Strength, Kapandji Index, Corners Distance, and Finger Mobility

The Cochin Hand Functional Scale ("CHFS") is a widely used, validated patient-reported measure of hand function. This tool assesses the ability of the patient to perform everyday tasks with their hands such as doing up buttons and zippers and preparing food.

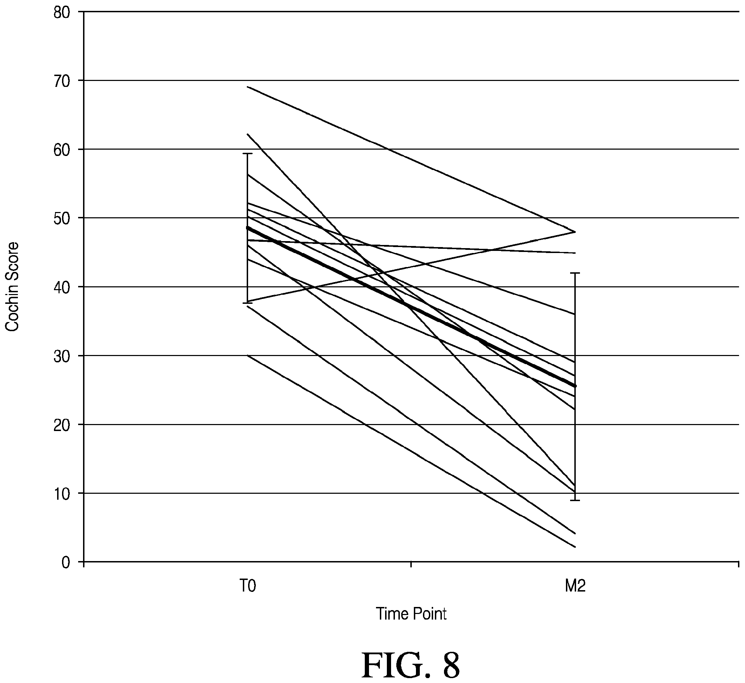

Hand function was assessed using the Cochin tool. The average score at baseline was 48.5±11.0 (mean ± standard deviation; range 30-69). Two months after treatment the average Cochin score had improved to an average of 25.5±16.5 (range 2-48). This change was statistically significant (p<0.001 by paired T test). A graph showing the two month data for each patient along with the mean and standard deviation (bold line) is shown in **Figure 8****.** A 47.4% (p <0.001) and 56.0% (p <0.001) decrease in CHFS compared to baseline was observed at 2 and 6 months post-treatment, respectively. Data are presented in **Table 1,** below. The 2 and 6 month CHFS data are graphically represented in **Figure 11****.**

Improvement of hand function as assessed by CHFS was also associated with a significant increase in hand strength and first cornder distance. Grip strength was evaluated by the art-accepted Jamar score. *See*, Schmidt, et al. (1970), Arch Phys Med Rehabil. 51:321-7. Grip strength increased at month 6, compared to baseline, with a mean of + 4.8 (± 6.4) kgs for the dominant hand (p=0.033) and + 4.0 (± 3.5) kgs for the non-dominant hand (p=0.033). Furthermore, a significant increase of the pinch strength score was observed at month 6. Specifically, pinch strength improved with a mean of +1.0 (±1.1) kg for the dominant hand (p = 0.009), and + 0.8 (±1.2) kg for the non-dominant hand (p= 0.050).

Subjects exhibited improved hand function as assessed by measurement of the first corner distance, as well as the sum of the 2^{nd} 3^{rd} and 4^{th} corner distance, in millimeters. Subjects also showed a significant improvement of finger flexion, as measured by the distance of the finger pad to the distal palmar line. The data are presented in **Table 1,** below.

**TABLE 1: ASSESMENT OF HAND FUNCTION**

| | **Baseline** | **2 months** | **P value*** | **6 months** | **P value**** |
|---|---|---|---|---|---|
| **CHFS total** | 48.5 (± 10.8) | 25.8 (± 17.0) | **p = 0.001** | 21.2 (± 15.4) | **p <.001** |
| Mean (SD) | | | | | |
| **Jamar score (kg) Non-dominant hand** | | | **p = 0.006** | | **(W) p = 0.002** |
| Mean (SD) | 14.9 (± 6.1) | 17.5 (± 7.8) | | 17.6 (±8.0) | |
| Median (range) | 14.0 [11.0 - 19.0] | 18.5 [14.0 - 21.5] | | 20.0 [11.0 - 23.0] | |
| **Jamar score (kg) Dominant hand** | | | **p** = **0.072 (NS)** | | **p = 0.033** |
| Mean (SD) | 16.0 (± 5.8) | 17.9 (± 7.3) | | 19.4 (± 7.4) | |
| Median (range) | 15.0 [12.0 - 18.0] | 18.5 [14.0 - 23.0] | | 20.0 [14.0 - 25.5] | |
| **Pinch score (kg) Non-dominant hand** | 1.3 (± 0.9) | 2.2 (± 0.7) | **p = 0.071 (NS)** | 2.1 (± 1.0) | **p = 0.050** |
| Mean (SD) | | | | | |
| **Pinch score (kg) Dominant hand** | 1.3 (± 1.1) | 2.0 (± 0.8) | **p = 0.199 (NS)** | 2.3 (± 1.3) | **p = 0.009** |
| Mean (SD) | | | | | |
| **Kapandji score /10 Non-dominant hand** | | | **(W) p = 1.000 (NS)** | | **(W) p = 0.187 (NS)** |
| Mean (SD) | 8.5 (± 1.2) | 8.4 (± 1.6) | | 8.8 (± 1.3) | |
| Median (range) | 9.0 [8.0 - 9.5] | 9.0 [8.0 - 9.5] | | 9.0 [8.0 - 9.8] | |
| **Kapandji score/10 Dominant hand** | | | **p = 0.380 (NS)** | | **p = 0.111 (NS)** |
| Mean (SD) | 8.0 (± 1.4) | 8.3 (± 1.6) | | 8.4 (± 1.7) | |
| Median (range) | 8.0 [7.0 - 9.0] | 9.0 [7.5 - 9.5] | | 9.3 [7.3 - 9.5] | |
| **1st corner distance (mm) Non-dominant hand** | 115.8 (± 24.5) | 122.3 (± 20.9) | **p = 0.042** | 132.8 (± 28.0) | **p = 0.002** |
| Mean (SD) | | | | | |
| **1st corner distance (mm) Dominant hand** | 105.6 (±24.7) | 112.9 (± 29.2) | **p = 0.010** | 118.9(± 31.2) | **p = 0.002** |
| Mean (SD) | | | | | |
| **Sum of corners distances (mm) Non-dominant hand** | 132.1 (± 24.6) | 133.7 (± 29.4) | **p = 0.052 (NS)** | 137.9 (± 27.0) | **p <.001** |
| Mean (SD) | | | | | |
| **Sum of corners distances (mm) Dominant hand** | 133.9 (± 18.5) | 131.2 (± 20.7) | **p = 0.822** | 140.2 (± 26.6) | **p = 0.073 (NS)** |
| Mean (SD) | | | | | |
| **Sum of Pad/DPL distance (mm) Non-dominant hand** | 48.1 (± 54.5) | 46.8 (± 52.0) | **p = 0.830 (NS)** | 37.8 (± 43.0) | **p = 0.109 (NS)** |
| Mean (SD) | | | | | |
| **Sum of Pad/DPL distance (mm) Dominant hand** | 52.0 (± 46.5) | 47.3 (± 43.8) | **p = 0.395 (NS)** | 43.9 (± 42.7) | **p = 0.111 (NS)** |
| Mean (SD) | | | | | |

| | | | | | |
|---|---|---|---|---|---|
| *P value from baseline to M2, **P value from baseline to M6 CHFS= Cochin Hand Functional Scale DPL= Distal Palmar Line Median given when p was calculated with Wilcoxon test | | | | | |

### 5. ADRCs Improve Vascular Manifestations of Scleroderma

Digital ulcers are relatively common in patients with scleroderma. Five of the twelve patients in this study had digital ulcers at baseline. Healing of digital ulcers was noted at the 2 and 6 month follow-up visits. The data are presented in **Table 2,** below.

**TABLE 2: DIGITAL ULCER OUTCOME**

| | Baseline No. DU's | 2 Mo's No. of DU's | 6 Mo's No. DU's |
|---|---|---|---|
| Patient 1 | | | |
| **Non-Dominant Hand** | 1 | 1 | 0 |
| **Dominant Hand** | 0 | 0 | 0 |

| Patient 3 | | | |
|---|---|---|---|
| **Non-Dominant Hand** | 1 | 0 | 0 |
| **Dominant Hand** | 4 | 0 | 1 |

| Patient 4 | | | |
|---|---|---|---|
| **Non-Dominant Hand** | 0 | 0 | 1 |
| **Dominant Hand** | 5 | 7 | 3 |

| Patient 7 | | | |
|---|---|---|---|
| **Non-Dominant Hand** | 1 | 0 | 1 |
| **Dominant Hand** | 1 | 0 | 1 |

| Patient 10 | | | |
|---|---|---|---|
| **Non-Dominant Hand** | 1 | 1 | 1 |
| **Dominant Hand** | 1 | 1 | 0 |

Vascular manifestations of scleroderma can also be assessed using the Nailfold Capillary Microscopy test, *e.g.,* as described in Lee, et al. (1983) J. Rheumatol. 10:930-938.In this test, the following parameters are measured: (1) the number of capillary loops/finger; (2) the presence or absence of sludge; (3) the number of enlarged capillary loops (over 4x the normal afferent, transition, and efferent limb width); (4) number of giant capillary loops/finger (10x or greater the normal width of capillary limbs; and (5) avascular score. For the avascular score, each finger was rated from 0 to 3, with 0= no avascular area; 1= 1-2 discrete avascular areas; 2 = > 2 discrete avascular areas; 3= extensive and confluent avascular areas. The 2 and 6 month data are presented in Table 3, below.

**TABLE 3: EFFECT OF AUTOLOGOUS ADIPOSE-DERIVED REGENERATIVE CELL INJECTION ON VASCULAR MANIFESTATIONS**

| | **Baseline** | **2 months** | ***P value** | **6 months** | ****P value** |
|---|---|---|---|---|---|
| **Raynaud's Condition Score (RCS)** Mean (SD) | 7.1 (± 1.2) | 3.2 (±1.7) | **p <.001** | 2.2 (±1.5) | **p <.001** |
| **Hand VAS /100** | 59.4 (± 17.2) | 21.6 (± 17.5) | **p = 0.001** | 17.8 (± 15.3) | **p <.001** |
| Mean (SD) | | | | | |

| **Capillaroscopy data** | | | | | |
|---|---|---|---|---|---|
| **Number of capillary loops** | | | | | |
| Non-dominant hand | | | | | |
| Mean (±SD) | 32.2 (± 20.9) | 28.4 (± 17.4) | | 30.5 (± 18.9) | |
| Median (range) | 30.0 [15.0 - 48.0] | 25.0 [15.0 - 41.0] | **(W) p = 0.031** | 30.0 [16.0 - 43.5] | **(W) p = 0.413** |
| Dominant hand | | | | | |
| Mean (±SD) | 27.3 (± 18.6) | 24.8 (± 16.8) | | 27.7 (± 17.1) | |
| Median (range) | 25.5 [11.5 - 39.0] | 20.5 [13.0 - 32.0] | **(W) p = 0.077** | 25.0 [15.0 - 37.5] | **(W) p = 0.762** |
| **Number of giant capillary** | | | | | |
| Non-dominant hand | | | | | |
| Mean (±SD) | 4.3 (± 4.8) | 3.6 (± 5.0) | | 3.6 (± 5.2) | |
| Median (range) | 3.0 [0.0 - 6.0] | 2.0 [0.0 - 5.0] | **(W) p = 0.006** | 2.0 [0.0 - 4.0] | **(W) p = 0.068** |
| | | | | | |
| Dominant hand | | | | | |
| Mean (±SD) | 4.1 (± 5.3) | 3.6 (± 4.4) | | 3.1 (± 4.3) | |
| Median (range) | 2.0 [0.0 - 7.0] | 2.0 [1.0 - 5.0] | **(W) p = 0.325** | 1.0 [0.0 - 4.0] | **(W) p = 0.017** |
| **Number of dystrophic capillaries** | | | | | |
| Non-dominant hand | | | | | |
| Mean (±SD) | 4.4 (± 5.8) | 2.2 (± 2.7) | | 1.9 (± 2.5) | |
| Median (range) | 2.0 [0.0 - 7.0] | 1.0 [0.0 - 3.0] | **(W) p = 0.003** | 1.0 [0.0 - 3.0] | **(W) p = 0.003** |
| Dominant hand | | | | | |
| Mean (±SD) | 4.7 (± 6.7) | 2.6 (± 3.6) | | 2.2 (± 2.8) | |
| Median (range) | 2.0 [0.0 - 6.51 | 1.0 [0.0 - 4.01 | **(W) p = 0.002** | 1.0 [0.0- 3.51 | **(W) p = 0.002** |
| **Vascular suppression score** | | | | | |
| Non-dominant hand | | | | | |
| Mean (±SD) | 1.6 (± 0.7) | 1.5 (± 0.7) | **p = 0.031** | 1.3 (± 0.7) | **p = 0.003** |
| | | | | | |
| Dominant hand | | | | | |
| Mean (±SD) | 1.7 (± 0.8) | 1.6 (± 0.6) | **p = 0.656** | 1.5 (± 0.7) | **p = 0.010** |

| | | | | | |
|---|---|---|---|---|---|
| *P value from baseline to M2, **P value from baseline to M6 Median given when p was calculated with Wilcoxon test | | | | | |

The data above indicate that ADRCs improve vascular manifestations of Scleroderma.

### 6. ADRCs Improve Sclerodactaly and Fibrosis

Significant improvement in sclerodactaly and fibrosis was also observed.

The Modified Rodnan Skin Score (MRSS) is the most widely used test to assess dermal skin thickness, and is an art-recognized surrogate measure of disease severity and mortality risk in patients with diffuse cutaneous systemic sclerosis: an increase in skin thickening is associated with involvement of internal organs and increased mortality rates. In addition, an improvement in the skin thickness score is associated with a more favorable outcome. *See*, Czirjak, et al. (2008) Rheumatolology 47(suppl. 5): 44-45. The total skin surface area is arbitrarily divided into 17 different areas: fingers, hands, forearms, arms, feet, legs, and thighs (in pairs), and face, chest, and abdomen. In each of these areas, the skin score is evaluated by manual palpation. The skin score is 0 for uninvolved skin, 1 for mild thickening, 2 for moderate thickening, and 3 for severe thickening (hidebound skin). The total skin score is the sum of the skin scores of the individual areas, the maximum possible score being 51. The skin score tends to correlate with the extent of dermal fibrosis, which in turn correlates with the extent of fibrosis and dysfunction of the internal organs, such as pulmonary fibrosis, scleroderma heart disease, renal disease, and gastrointestinal involvement.

No significant change of the MRSS focused on hands was observed from baseline to month 2 or month 6. However, an at least 25% reduction of the MRSS was observed in 4 patients, with skin softening noted on the dorsal part of the hand. Global MRSS significantly decreased from baseline (mean ±SD 13.9± 9.8) to month 2 (11.7 ±9.8; p = 0.010), and to month 6 (11.5±10.1; p=0.013). 5 patients exhibited an at least 25% reduction in Global MRSS at month 6. The data are presented in **Table 4,** below.

Diameter of fingers, measured as ring size, is also an indicator of soft tissue changes of the digits in Scleroderma. *See*, Serup, J. (1985) Dermatology 171:41-44. A significant improvement in diameter of the figures, measured as ring size, was observed in pa tents at month 2 and 6, compared to baseline. The data are presented in **Table 4.** The data suggest that treatment with adipose-derived regenerative cells as disclosed herein can lead to improvement in skin edema.

**TABLE 3: EFFECT OF ADRC INJECTION ON SCLERODACTYLY AND HAND FIBROSIS**

| | **Baseline** | **2 months** | ***P value** | **6 months** | ****P value** |
|---|---|---|---|---|---|
| **Mean diameter of F1-F5 (ring-size) Non-dominant hand** | | | | | |
| Mean (SD) | 60.7 (± 2.3) | 59.3 (± 1.7) | **p = 0.010** | 58.1 (± 2.2) | **p <.001** |
| **Mean diameter of F1-F5 (ring-size) Dominant hand** | | | | | |
| Mean (SD) | 61.9 (± 2.2) | 60.7 (± 2.3) | **p = 0.013** | 59.8 (± 2.4) | **p <.001** |
| **MRSS applied to hand /18** | | | | | |
| Median (range) | 10.9 (± 4.9) | 10.0 (± 5.3) | **p = 0.067 (NS)** | 9.9 (± 6.0) | **p = 0.246 (NS)** |
| **Global MRSS /51** | | | | | |
| Mean (SD) | 13.9 (± 9.8) | 11.7 (± 9.8) | **p = 0.010** | 11.5 (± 10.1) | **p = 0.013** |

| | | | | | |
|---|---|---|---|---|---|
| *P value from baseline to month 2 **P value from baseline to month 6 MRSS = Modified Rodnan Skin Score | | | | | |

Patients also reported improvement in systemic symptoms of their disease, that is, improvement in symptoms in anatomic locations distal to the site of treatment (the fingers).

### Patient A: Quotes

Patient: After the treatment, as I told you, I had a feeling that I got 10 new fingers. I had no longer the same feelings than before. I feel much more flexibility; I have no more pain, no more this electric type pain in my hands, in my forearms and on my shoulders. My fingers are less blue compared to when I had my Raynaud's syndrome. My fingers are still a bit white but I am much more active than before. I can do everything naturally (without realizing) with no pain. I am very happy. However, in my right hand, I still feel some "heaviness" and my fingers are still a bit swollen compared to my left hand.
*Doctor: So, what are the changes in your everyday life?*
Patient: Everything changed. Before, I was not able to straighten or dry my hair; I was not able to type a letter, I was not to able to open a canned box, to peel vegetables, to cut meat, to clean the dishes, to make up.... There were a lot of things that I was not able to do such as painting, sewing... but now I can do everything naturally and without realizing.

### Patient B: Quotes

Patient: I was not able to use hand. I used to cut meat like that. I was not able to cut meat.
Doctor: And now?
Patient: Now, I can do everything, I can use the keyboard, I have no longer this electric feeling in my fingertips. Before, I had pain and this "electric" feeling in my fingertips.
Doctor: What kind of pain did you have on your fingertips?
Patient: it was like an electric type pain, I used constantly feel this electric pain everywhere.

### Other Quotes:

My fingers returned to their normal color for the first time in 5 years
I don't feel any more pain, at all
I am not cold anymore, I stopped wearing gloves at all time
I can cut the meat of my kids and brush my hair, I am back to normal life
I am back to work [secretary]

These data demonstrate that the adipose-derived cells disclosed herein are useful for treating pain and fibrosis, e.g., in subjects with Scleroderma. In particular, the data demonstrate that the adipose-derived regenerative cells disclosed herein are useful for improving vascular function and fibrosis in subjects with Scleroderma.

### EXAMPLE 2

### Treatment of Neuropathic Pain Using Adipose Derived Cells

Chronic pain, e.g., neuropathic pain, a condition that afflicts at least 30% of Americans, is caused, e.g., by disorders of the nervous system, also known as neuropathy, and can be accompanied by, or caused by, tissue damage, including nerve fibers that are damaged, dysfunction or injured. Neuropathic pain may also be caused by, e.g. pathologic lesions, neurodegeneration processes, or prolonged dysfunction of parts of the peripheral or central nervous system. However, neuropathic pain can also be present when no discernible tissue damage is evident.

Neuropathy is a collection of disorders that occurs when nerves of the peripheral nervous system (the part of the nervous system outside of the brain and spinal cord) are damaged. The condition is generally referred to as peripheral neuropathy, and it is most commonly due to damage to nerve axons. Neuropathy usually causes pain and numbness in the hands and feet. It can result from traumatic injuries, infections, metabolic disorders, and exposure to toxins. One of the most common causes of neuropathy is diabetes. Neuropathy can affect nerves that control muscle movement (motor nerves) and those that detect sensations such as coldness or pain (sensory nerves). In some cases - autonomic neuropathy - it can affect internal organs, such as the heart, blood vessels, bladder, or intestines.

Thus, in another example, provided herein is a method of treating an individual having chronic pain comprising administering to the individual a therapeutically-effective amount of a composition comprising ADRCs. In a specific embodiment, the chronic pain, e.g., neuropathic pain, is, or is caused by, neuritis (e.g., polyneuritis, brachial neuritis, optic neuritis, vestibular neuritis, cranial neuritis, or arsenic neuritis), diabetes mellitus (e.g., diabetic neuropathy), peripheral neuropathy, reflex sympathetic dystrophy syndrome, phantom limb pain, post-amputation pain, postherpetic neuralgia, shingles, central pain syndrome (pain caused, e.g., by damage to the brain, spinal cord and/or brainstem), Guillain-Barre Syndrome, degenerative disc disease, cancer, multiple sclerosis, kidney disorders, alcoholism, human immunodeficiency virus-related neuropathy, Wartenberg's Migratory Sensory Neuropathy, fibromyalgia syndrome, causalgia, spinal cord injury, or exposure to a chemical agent, e.g., trichloroethylene or dapsone (diaminyl-diphenyl sulfone). In specific embodiments, the peripheral neuropathy is mononeuropathy (damage to a single peripheral nerve); polyneuropathy (damage to more than one peripheral nerve, frequently sited in different parts of the body), mononeuritis multiplex (simultaneous or sequential damage to noncontiguous nerve trunks), or autonomic neuropathy. Peripheral neuropathy, e.g., mononeuritis multiplex, may be caused by, e.g., diabetes mellitus, vasculitis (e.g., polyarteritis nodosa, Wegener granulomatosis, or Churg-Strauss syndrome), rheumatoid arthritis, lupus erythematosus (SLE), sarcoidosis, an amyloidosis, or cryoglobulinemia.

Levels of neuropathic pain may be assessed, e.g., by the Visual Analog Scale (VAS). Numeric Pain Intensity Scale, Graphic Rating Scale, Verbal Rating Scale, Pain Faces Scale (Faces Pain Scale), Numeric Pain Intensity & Pain Distress Scales, Brief Pain Inventory (BPI), Memorial Pain Assessment, Alder Hey Triage Pain Score, Dallas Pain Questionnaire, Dolorimeter Pain Index (DPI), Face Legs Activity Cry Consolability Scale, Lequesne Scale, McGill Pain Questionnaire (MPQ), Descriptor differential scale (DDS), Neck Pain and disability Scale (NPAD), Numerical 11-Point Box (BS-11), Roland-Morris Back Pain Questionnaire, or the Wong-Baker FACES Pain Rating Scale. An improvement after administration of the composition to the individual in one or more of these assessments of pain is considered therapeutically effective.

The composition can be, for example, administered locally, distally from a site of neuropathic pain, to a nerve or set of nerves that serve a damaged area of the body of an individual, e.g., an area of the body in which the individual is experiencing the neuropathic pain. For example, the composition can be administered along the spine at any point at which nerve trunks emerge from the spinal column, e.g., any of the cervical nerves, thoracic nerves, or lumbar nerves. In specific embodiments, the composition can be administered adjacent to the spinal cord at which point nerves emerging at C1, C2, C3, C4, C5, C6, or C7, or T1, T2, T3, T4, T5, T6, T7, T8, T9, T10, T11 or T12, or L1, L2, L3, L4 or L5, or at the sacrum.

### In Vivo Model for Treating Neuropathic Pain with Compositions Comprising Adipose-Derived Cells

Below is a rat model for chronic, painful peripheral mononeuropathy.

Peripheral mononeuropathy is surgically induced in rats as follows. Rats are anesthetized with sodium pentobarbital (40 mg/kg. i.p.). The common sciatic nerve is exposed at the level of the middle of the thigh by blunt dissection through biceps femoris. Proximal to the sciatic nerve's trifurcation, about 7 mm of nerve is freed of adhering tissue and 4 ligatures (4.0 chromic gut) are tied loosely around it with about 1 mm spacing. The length of nerve thus affected is 45 mm long. The ligatures are tied such that the diameter of the nerve is seen to be just barely constricted when viewed with 40x magnification. The desired degree of constriction retards, but does not arrest, circulation through the superficial epineurial vasculature. The incision is closed in layers. In each animal, an identical dissection is performed on the opposite side, except that the sciatic nerve is not ligated. Groups of control rats are used, wherein some rats are not operated upon and others receive bilateral sham procedures (sciatic exposure without ligation).

Each group is subsequently treated with ADRCs or placebo. The animals are inspected every 1 or 2 days during the first 14 postoperative days and at about weekly intervals thereafter. During these inspections, each rat is placed upon a table and carefully observed for 1-2 minutes. Notes are made of the animal's gait, the posture of the affected hind paw, the condition of the hind paw skin, and the extent, if present, of autotomy. Particular attention is given to the condition of the claws because autotomy involving frank tissue damage can be indicated by gnawed claw tips. Postoperative, post-administration behavior of the rats is observed, including appetite and hyperalgesic responses to noxious radiant heat and chemogenic pain.

### Assessment of Response to Noxious Heat

The rats are placed beneath an inverted, clear plastic cage (18x28x13 cm) upon an elevated floor of window glass. A radiant heat source beneath the glass floor is aimed at the plantar hind paw. Stimulus onset activates a timer controlled by a photocell positioned to receive light reflected from the hind paw. The hind paw withdrawal reflex interrupts the photocell's light and automatically stopped the timer. Latencies are measured to the nearest 0.1 sec. The hind paws are tested alternately with 5 min intervals between consecutive tests. Five latency measurements are taken for each hind paw in each test session. The 5 latencies per side are averaged and a difference score is computed by subtracting the average latency of the control side from the average latency of the ligated side. Difference scores are compared for each treatment group.

### EXAMPLE 3

### Treatment of Non-localized Fibrosis Using Adipose Derived Cells Comprising Regenerative Cells

Chemotherapy is often associated with widespread fibrosis. This clinical observation has been extended to an animal model in which agents such as bleomycin are delivered locally or systemically to induce fibrosis as evidenced by significant increase in skin thickness (which can be measured by histologic evaluation). In one such model bleomycin is injected into the skin of mice. Local or systemic (eg: intravascular) delivery of ADRCs to the bleomycin-treated mice leads to decrease in skin thickness and improvement in vascularity at the treated area indicating reduced fibrosis.

Specifically, bleomycin (0.1mL; 300 µg/ml) is delivered to mice by subcutaneous injection in the inter scapular region and both flanks using a 26-gauge needle, for 7 days per week for 4 consecutive weeks. One week after completion of bleomycin injury mice are treated with ADRCs. Skin fibrosis is evaluated by histological analyses 5 and 13 weeks post-BLM injection. Results show that treatment with ADRCs correct the dermal lesion induced by bleomycin such that dermal thickness was indistinguishable from that of normal, un-injured skin. Similarly, treatment with ADRCs is associated with a significant increase in vascularity.

Similarly, administration of bleomycin systemically or by inhalation is a model of pulmonary fibrosis. Treatment of animals injured in this fashion with ADRCs can lead to reduced fibrosis and consequent improvement in lung function.

Additional disclosure is also provided by the following numbered examples:
1. A method of modulating pain in a subject in need thereof, comprising:
   administering a therapeutically effective amount of adipose derived regenerative cells to said subject.
2. The method of example 1, further comprising selecting, identifying, or classifying said subject as an individual experiencing pain.
3. The method of anyone of examples 1 or 2, wherein said selection, identification, or classification is made by a physician or clinical or diagnostic evaluation.
4. The method of anyone of examples 1-3, further comprising determining or measuring pain in said subject before, after, or during administration of said adipose derived regenerative cells.
5. A method of modulating fibrosis in a subject in need thereof, comprising:
   administering a therapeutically effective amount of adipose derived cells comprising regenerative cells to said subject.
6. The method of example 5, further comprising selecting, identifying, or classifying said subject as an individual experiencing fibrosis or having a fibrotic disease.
7. The method of anyone of examples 5 or 6, wherein said selection, identification, or classification is made by a physician or clinical or diagnostic evaluation.
8. The method of anyone of examples 5-7, further comprising determining or measuring fibrosis or a marker thereof, such as the amount of or level of TNF-alpha, in said subject or in a biological sample obtained from said subject before, after, or during administration of said adipose derived regenerative cells.
9. A method of improving range of motion in a joint in a subject in need thereof, comprising:
   administering a therapeutically effective amount of adipose derived cells comprising regenerative cells to said subject.
10. The method of example 9, further comprising selecting, identifying, or classifying said subject as an individual experiencing a loss of joint mobility or a reduction in the range of motion of a joint.
11. The method of any one of examples 9 or 10, wherein said selection, identification, or classification is made by a physician or clinical or diagnostic evaluation.
12. The method of any one of examples 9-11, further comprising determining or measuring the mobility or range of motion of a joint of said subject before, after, or during administration of said adipose derived regenerative cells.
13. The method of any one of examples 1-12, wherein said adipose-derived regenerative cells comprise stem cells.
14. The method of any one of examples 1-13, wherein said adipose-derived regenerative cells comprise precursor cells.
15. The method of any one of examples 1-14, wherein said adipose-derived regenerative cells comprise progenitor cells.
16. The method of any one of examples 1-15, wherein said subject has scleroderma.

## Claims

1. A therapeutically effective amount of adipose-derived stem and regenerative cells for use in the treatment of Raynaud's phenomenon in a subject in need thereof.

2. The therapeutically effective amount of adipose-derived stem and regenerative cells for the use according to claim 1, wherein the Raynaud's phenomenon is secondary Raynaud's.

3. The therapeutically effective amount of adipose-derived stem and regenerative cells for the use according to claim 1, wherein the Raynaud's phenomenon comprises primary Raynaud's.

4. The therapeutically effective amount of adipose-derived stem and regenerative cells for the use according to claim 1, wherein the Raynaud's arises from one or more of scleroderma, systemic lupus erythematosus, rheumatoid arthritis, Sjögren's syndrome, dermatomyositis, polymyositis, mixed connective tissue disease, cold agglutinin disease, Ehlers-Danlos syndrome, atherosclerosis, Bueger's disease, Takayasu's arteritis, subclavian aneurisms, and thoracic outlet syndrome.

5. The therapeutically effective amount of adipose-derived stem and regenerative cells for the use according to any of the preceding claims, wherein the adipose-derived stem and regenerative cells are cryopreserved.

6. The therapeutically effective amount of adipose-derived stem and regenerative cells for the use according to any of the preceding claims, wherein the adipose-derived stem and regenerative cells are cultured.

7. The therapeutically effective amount of adipose-derived stem and regenerative cells for the use according to any of claims 1-5, wherein the adipose-derived stem and regenerative cells are not cultured.

8. The therapeutically effective amount of adipose-derived stem and regenerative cells for the use according to claim 1, wherein the adipose-derived stem and regenerative cells are extracted from adipose tissue from the subject to whom the cells are to be implanted.

9. The therapeutically effective amount of adipose-derived stem and regenerative cells for the use according to claim 1, wherein the adipose-derived stem and regenerative cells are for allogeneic transplantation.

10. The therapeutically effective amount of adipose-derived stem and regenerative cells for the use according to claim 1, wherein the treatment comprises reduction of pain.

11. The therapeutically effective amount of adipose-derived stem and regenerative cells for the use according to claim 1, wherein the treatment comprises reducing the extent of vasoconstriction when the subject is exposed to stress or cold.

12. The therapeutically effective amount of adipose-derived stem and regenerative cells for the use according to claim 1, wherein the treatment comprises reducing the Raynaud's severity score.

13. The therapeutically effective amount of adipose-derived stem and regenerative cells for the use according to claim 1, wherein the cells are formulated for administration to the subject's fingers.

14. The therapeutically effective amount of adipose-derived stem and regenerative cells for the use according to claim 1, wherein the adipose-derived stem and regenerative cells are comprised in a composition, wherein said composition comprises an additive.

## Patentansprüche

1. Therapeutisch wirksame Menge von aus Fett abgeleiteten Stammzellen und regenerativen Zellen zur Verwendung bei der Behandlung des Raynaud-Phänomens bei einem Subjekt, das dessen bedarf.

2. Therapeutisch wirksame Menge von aus Fett abgeleiteten Stammzellen und regenerativen Zellen zur Verwendung nach Anspruch 1, wobei das Raynaud-Phänomen sekundäres Raynaud ist.

3. Therapeutisch wirksame Menge von aus Fett abgeleiteten Stammzellen und regenerativen Zellen zur Verwendung nach Anspruch 1, wobei das Raynaud-Phänomen primäres Raynaud umfasst.

4. Therapeutisch wirksame Menge von aus Fett abgeleiteten Stammzellen und regenerativen Zellen zur Verwendung nach Anspruch 1, wobei das Raynaud zurückzuführen ist auf eines oder mehreren von Sklerodermie, systemischer Lupus erythematodes, rheumatoide Arthritis, Sjögren-Syndrom, Dermatomyositis, Polymyositis, gemischte Bindegewebskrankheit, Erkältungs-Agglutinin-Erkrankung, Ehlers-Danlos-Syndrom, Atherosklerose, Bueger-Erkrankung, Takayasu-Arteriitis, Subclavia-Aneurismen und Thorax-Outlet-Syndrom.

5. Therapeutisch wirksame Menge von aus Fett abgeleiteten Stammzellen und regenerativen Zellen zur Verwendung nach einem der vorhergehenden Ansprüche, wobei die von aus Fett abgeleiteten Stammzellen und die regenerativen Zellen kryokonserviert sind.

6. Therapeutisch wirksame Menge von aus Fett abgeleiteten Stammzellen und regenerativen Zellen zur Verwendung nach einem der vorhergehenden Ansprüche, wobei die von aus Fett abgeleiteten Stammzellen und die regenerativen Zellen kultiviert sind.

7. Therapeutisch wirksame Menge von aus Fett abgeleiteten Stammzellen und regenerativen Zellen zur Verwendung nach einem der Ansprüche 1-5, wobei die von aus Fett abgeleiteten Stammzellen und die regenerativen Zellen nicht kultiviert sind.

8. Therapeutisch wirksame Menge von aus Fett abgeleiteten Stammzellen und regenerativen Zellen zur Verwendung nach Anspruch 1, wobei die von aus Fett abgeleiteten Stammzellen und die regenerativen Zellen aus dem Fettgewebe des Subjekts extrahiert werden, in das sie implantiert werden sollen.

9. Therapeutisch wirksame Menge von aus Fett abgeleiteten Stammzellen und regenerativen Zellen zur Verwendung nach Anspruch 1, wobei die von aus Fett abgeleiteten Stammzellen und die regenerativen Zellen zur allogenetischen Transplantation bestimmt sind.

10. Therapeutisch wirksame Menge von aus Fett abgeleiteten Stammzellen und regenerativen Zellen zur Verwendung nach Anspruch 1, wobei die Behandlung das Verringern von Schmerzen umfasst.

11. Therapeutisch wirksame Menge von aus Fett abgeleiteten Stammzellen und regenerativen Zellen zur Verwendung nach Anspruch 1, wobei die Behandlung das Verringern des Ausmaßes der Vasokonstriktion umfasst, wenn das Subjekt Stress oder Kälte ausgesetzt ist.

12. Therapeutisch wirksame Menge von aus Fett abgeleiteten Stammzellen und regenerativen Zellen zur Verwendung nach Anspruch 1, wobei die Behandlung das Verringern des Raynaud-Schweregrad-Scores umfasst.

13. Therapeutisch wirksame Menge von aus Fett abgeleiteten Stammzellen und regenerativen Zellen zur Verwendung nach Anspruch 1, wobei die Zellen für die Verabreichung an die Finger eines Subjekts formuliert sind.

14. Therapeutisch wirksame Menge von aus Fett abgeleiteten Stammzellen und regenerativen Zellen zur Verwendung nach Anspruch 1, wobei die von aus Fett abgeleiteten Stammzellen und die regenerativen Zellen in einer Zusammensetzung enthalten sind, wobei die genannte Zusammensetzung ein Additiv umfasst.

## Revendications

1. Quantité thérapeutiquement efficace de cellules régénératives et souches dérivées de tissu adipeux, pour utilisation dans le traitement du phénomène de Raynaud chez un sujet en ayant besoin.

2. Quantité thérapeutiquement efficace de cellules régénératives et souches dérivées de tissu adipeux pour utilisation selon la revendication 1, dans laquelle le phénomène de Raynaud est le phénomène de Raynaud secondaire.

3. Quantité thérapeutiquement efficace de cellules régénératives et souches dérivées de tissu adipeux pour utilisation selon la revendication 1, dans laquelle le phénomène de Raynaud comprend le phénomène de Raynaud primaire.

4. Quantité thérapeutiquement efficace de cellules régénératives et souches dérivées de tissu adipeux pour utilisation selon la revendication 1, dans laquelle le phénomène de Raynaud résulte d'un ou plusieurs de la sclérodermie, du lupus érythémateux disséminé, de la polyarthrite rhumatoïde, du syndrome de Sjögren, de la dermatomyosite, de la polymyosite, de la connectivite mixte, de la maladie des agglutinines froides, du syndrome d'Ehlers-Danlos, de l'athérosclérose, de la maladie de Buerger, de l'artérite de Takayasu, des anévrysmes sous-claviers et du syndrome de la traversée thoracobrachiale.

5. Quantité thérapeutiquement efficace de cellules régénératives et souches dérivées de tissu adipeux pour utilisation selon l'une des revendications précédentes, dans laquelle les cellules régénératives et souches dérivées de tissu adipeux sont cryoconservées.

6. Quantité thérapeutiquement efficace de cellules régénératives et souches dérivées de tissu adipeux pour utilisation selon l'une des revendications précédentes, dans laquelle les cellules régénératives et souches dérivées de tissu adipeux sont mises en culture.

7. Quantité thérapeutiquement efficace de cellules régénératives et souches dérivées de tissu adipeux pour utilisation selon l'une des revendications 1 à 5, dans laquelle les cellules régénératives et souches dérivées de tissue adipeux ne sont pas mises en culture.

8. Quantité thérapeutiquement efficace de cellules régénératives et souches dérivées de tissu adipeux pour utilisation selon la revendication 1, dans laquelle les cellules régénératives et souches dérivées de tissu adipeux sont extraites du tissu adipeux provenant du sujet auquel les cellules doivent être implantées.

9. Quantité thérapeutiquement efficace de cellules régénératives et souches dérivées de tissu adipeux pour utilisation selon la revendication 1, dans laquelle les cellules régénératives et souches dérivées de tissu adipeux sont destinées à la transplantation allogénique.

10. Quantité thérapeutiquement efficace de cellules régénératives et souches dérivées de tissu adipeux pour utilisation selon la revendication 1, où le traitement comprend une réduction de la douleur.

11. Quantité thérapeutiquement efficace de cellules régénératives et souches dérivées de tissu adipeux pour utilisation selon la revendication 1, où le traitement comprend la réduction de l'étendue de la vasoconstriction lorsque le sujet est exposé au stress ou au froid.

12. Quantité thérapeutiquement efficace de cellules régénératives et souches dérivées de tissu adipeux pour utilisation selon la revendication 1, où le traitement comprend la réduction du score de gravité du phénomène de Raynaud.

13. Quantité thérapeutiquement efficace de cellules régénératives et souches dérivées de tissu adipeux pour utilisation selon la revendication 1, dans laquelle les cellules sont formulées pour être administrées aux doigts du sujet.

14. Quantité thérapeutiquement efficace de cellules régénératives et souches dérivées de tissu adipeux pour utilisation selon la revendication 1, dans laquelle les cellules régénératives et souches dérivées du tissu adipeux sont contenues dans une composition, où ladite composition comprend un additif.
